# EUROPEAN PATENT SPECIFICATION

(11) **EP 3 018 209 B1**
(45) Date of publication and mention of the grant of the patent: **16.10.2019**
(21) Application number: 14820458.9
(22) Date of filing: 04.07.2014
(51) Int. Cl.: C12N 15/113, C07H 21/02, A61K 48/00, A61P 11/00

(54) **RESPIRATORY DISEASE-RELATED GENE SPECIFIC SIRNA, DOUBLE-HELICAL OLIGO RNA STRUCTURE CONTAINING SIRNA, COMPOSITON CONTAINING SAME FOR PREVENTING OR TREATING RESPIRATORY DISEASE**
ATEMWEGSERKRANKUNGSBEZOGENE GENSPEZIFISCHE SIRNA, DOPPELHELIX-OLIGO-RNA-STRUKTUR MIT DER SIRNA SOWIE ZUSAMMENSETZUNG DAMIT ZUR PRÄVENTION ODER BEHANDLUNG VON ATEMWEGSERKRANKUNGEN
ARNSI SPÉCIFIQUE DU GÈNE ASSOCIÉ À UNE MALADIE RESPIRATOIRE, STRUCTURE OLIGO-ARN DOUBLE HÉLICE CONTENANT L'ARNSI, COMPOSITION CONTENANT CETTE STRUCTURE POUR PRÉVENIR OU TRAITER UNE MALADIE RESPIRATOIRE

(30) Priority: 05.07.2013 KR 20130079311
(43) Date of publication of application: 11.05.2016
(60) Divisional application: 19180054.9
(73) Proprietor: Bioneer Corporation, Daejeon 306-220 (KR); Yuhan Corporation, Seoul 06927 (KR)
(72) Inventor: CHAE, Jeiwook, Daejeon 305-761 (KR); PARK, Han Oh, Daejeon 305-761 (KR); YOON, Pyoung Oh, Daejeon 305-751 (KR); HAN, Boram, Bucheon-si Gyeonggi-do 422-753 (KR); KIM, Mi Na, Daejeon 305-770 (KR)
(74) Representative: dompatent von Kreisler Selting Werner - Partnerschaft von Patent- und Rechtsanwälten mbB
(86) International application number: PCT/KR2014/006033
(87) International publication number: WO 2015/002513

(56) References cited:
- WO-A1-2011/119887
- KR-A- 20100 123 214
- US-A1- 2008 015 114
- US-A1- 2008 108 583
- US-A1- 2013 096 288

<110> BIONEER CORPORATION
<120> Respiratory disease related genes-specific siRNA, double-stranded oligo RNA molecules comprising the siRNA, and composition for the prevention or treatment of respiratory diseases comprising the same
<130> PP-B1388
<150> KR 10-2013-0079311
   <151> 2013-07-05
<160> 604
<170> KopatentIn 2.0
<210> 1
   <211> 23
   <212> RNA
   <213> Artificial Sequence
<220>
   <223> hCTGF
<400> 1
   atgtgcattc tccagccatc aag 23
<210> 2
   <211> 23
   <212> RNA
   <213> Artificial Sequence
<220>
   <223> hCTGF
<400> 2
   ttctgaacac cataggtaga atg 23
<210> 3
   <211> 23
   <212> RNA
   <213> Artificial Sequence
<220>
   <223> hCTGF
<400> 3
   gtctgatcgt tcaaagcatg aaa 23
<210> 4
   <211> 23
   <212> RNA
   <213> Artificial Sequence
<220>
   <223> hCTGF
<400> 4
   tatgactgtt tttcggacag ttt 23
<210> 5
   <211> 23
   <212> RNA
   <213> Artificial Sequence
<220>
   <223> hCTGF
<400> 5
   gggaaaagat tcccacccaa ttc 23
<210> 6
   <211> 23
   <212> RNA
   <213> Artificial Sequence
<220>
   <223> hCTGF
<400> 6
   gagacatggc atgaagccag aga 23
<210> 7
   <211> 23
   <212> RNA
   <213> Artificial Sequence
<220>
   <223> hCTGF
<400> 7
   ttcacatctc atttttccgt aaa 23
<210> 8
   <211> 23
   <212> RNA
   <213> Artificial Sequence
<220>
   <223> hCTGF
<400> 8
   tacaactgtc ccggagacaa tga 23
<210> 9
   <211> 23
   <212> RNA
   <213> Artificial Sequence
<220>
   <223> hCTGF
<400> 9
   ttgtctgatc gttcaaagca tga 23
<210> 10
   <211> 23
   <212> RNA
   <213> Artificial Sequence
<220>
   <223> hCTGF
<400> 10
   atgatttcag tagcacaagt tat 23
<210> 11
   <211> 23
   <212> RNA
   <213> Artificial Sequence
<220>
   <223> hCTGF
<400> 11
   taccagcaga aaggttagta tca 23
<210> 12
   <211> 23
   <212> RNA
   <213> Artificial Sequence
<220>
   <223> hCTGF
<400> 12
   cggagacatg gcatgaagcc aga 23
<210> 13
   <211> 23
   <212> RNA
   <213> Artificial Sequence
<220>
   <223> hCTGF
<400> 13
   ttgatatgac tgtttttcgg aca 23
<210> 14
   <211> 23
   <212> RNA
   <213> Artificial Sequence
<220>
   <223> hCTGF
<400> 14
   gagagacatt aactcattag act 23
<210> 15
   <211> 23
   <212> RNA
   <213> Artificial Sequence
<220>
   <223> hCTGF
<400> 15
   gagtgagaga cattaactca tta 23
<210> 16
   <211> 23
   <212> RNA
   <213> Artificial Sequence
<220>
   <223> hCTGF
<400> 16
   gtcgattaga ctggacagct tgt 23
<210> 17
   <211> 23
   <212> RNA
   <213> Artificial Sequence
<220>
   <223> hCTGF
<400> 17
   aagttacatg tttgcacctt tct 23
<210> 18
   <211> 23
   <212> RNA
   <213> Artificial Sequence
<220>
   <223> hCTGF
<400> 18
   aaaacattgt gccatgtcaa aca 23
<210> 19
   <211> 23
   <212> RNA
   <213> Artificial Sequence
<220>
   <223> hCTGF
<400> 19
   taagacttga cagtggaact aca 23
<210> 20
   <211> 23
   <212> RNA
   <213> Artificial Sequence
<220>
   <223> hCTGF
<400> 20
   gaacaccata ggtagaatgt aaa 23
<210> 21
   <211> 23
   <212> RNA
   <213> Artificial Sequence
<220>
   <223> hCTGF
<400> 21
   ggacagcttg tggcaagtga att 23
<210> 22
   <211> 23
   <212> RNA
   <213> Artificial Sequence
<220>
   <223> hCTGF
<400> 22
   ccctgccggt ggagttcaag tgc 23
<210> 23
   <211> 23
   <212> RNA
   <213> Artificial Sequence
<220>
   <223> hCTGF
<400> 23
   gcccagaccc aactatgatt aga 23
<210> 24
   <211> 23
   <212> RNA
   <213> Artificial Sequence
<220>
   <223> hCTGF
<400> 24
   aacaccatag gtagaatgta aag 23
<210> 25
   <211> 23
   <212> RNA
   <213> Artificial Sequence
<220>
   <223> hCTGF
<400> 25
   gtccttggca ggctgatttc tag 23
<210> 26
   <211> 23
   <212> RNA
   <213> Artificial Sequence
<220>
   <223> hCTGF
<400> 26
   catcttcggt ggtacggtgt acc 23
<210> 27
   <211> 23
   <212> RNA
   <213> Artificial Sequence
<220>
   <223> hCTGF
<400> 27
   agacttgaca gtggaactac att 23
<210> 28
   <211> 23
   <212> RNA
   <213> Artificial Sequence
<220>
   <223> hCTGF
<400> 28
   tttctgaaca ccataggtag aat 23
<210> 29
   <211> 23
   <212> RNA
   <213> Artificial Sequence
<220>
   <223> hCTGF
<400> 29
   cagcaccaga atgtatatta agg 23
<210> 30
   <211> 23
   <212> RNA
   <213> Artificial Sequence
<220>
   <223> hCTGF
<400> 30
   tagtatcatc agatagcatc tta 23
<210> 31
   <211> 23
   <212> RNA
   <213> Artificial Sequence
<220>
   <223> hCTGF
<400> 31
   ctctgacatt ctgattcgaa tga 23
<210> 32
   <211> 23
   <212> RNA
   <213> Artificial Sequence
<220>
   <223> hCTGF
<400> 32
   tggcaggctg atttctaggt agg 23
<210> 33
   <211> 23
   <212> RNA
   <213> Artificial Sequence
<220>
   <223> hCTGF
<400> 33
   cacagcacca gaatgtatat taa 23
<210> 34
   <211> 23
   <212> RNA
   <213> Artificial Sequence
<220>
   <223> hCTGF
<400> 34
   gttcaggaat cggaatcctg tcg 23
<210> 35
   <211> 23
   <212> RNA
   <213> Artificial Sequence
<220>
   <223> hCTGF
<400> 35
   catctttgaa tcgctgtact aca 23
<210> 36
   <211> 23
   <212> RNA
   <213> Artificial Sequence
<220>
   <223> hCTGF
<400> 36
   ctgatttcta ggtaggaaat gtg 23
<210> 37
   <211> 23
   <212> RNA
   <213> Artificial Sequence
<220>
   <223> hCTGF
<400> 37
   agccagagag tgagagacat taa 23
<210> 38
   <211> 23
   <212> RNA
   <213> Artificial Sequence
<220>
   <223> hCTGF
<400> 38
   gacagcttgt ggcaagtgaa ttt 23
<210> 39
   <211> 23
   <212> RNA
   <213> Artificial Sequence
<220>
   <223> hCTGF
<400> 39
   ttgaagaatg ttaagacttg aca 23
<210> 40
   <211> 23
   <212> RNA
   <213> Artificial Sequence
<220>
   <223> hCTGF
<400> 40
   gacagctagg atgtgcattc tcc 23
<210> 41
   <211> 23
   <212> RNA
   <213> Artificial Sequence
<220>
   <223> hCTGF
<400> 41
   gagtgtgacc aaaagttaca tgt 23
<210> 42
   <211> 23
   <212> RNA
   <213> Artificial Sequence
<220>
   <223> hCTGF
<400> 42
   gcctatcaag tttgagcttt ctg 23
<210> 43
   <211> 23
   <212> RNA
   <213> Artificial Sequence
<220>
   <223> hCTGF
<400> 43
   taccgactgg aagacacgtt tgg 23
<210> 44
   <211> 23
   <212> RNA
   <213> Artificial Sequence
<220>
   <223> hCTGF
<400> 44
   ccccagtgac agctaggatg tgc 23
<210> 45
   <211> 23
   <212> RNA
   <213> Artificial Sequence
<220>
   <223> hCTGF
<400> 45
   ctccagccat caagagactg agt 23
<210> 46
   <211> 23
   <212> RNA
   <213> Artificial Sequence
<220>
   <223> hCTGF
<400> 46
   aggctgattt ctaggtagga aat 23
<210> 47
   <211> 23
   <212> RNA
   <213> Artificial Sequence
<220>
   <223> hCTGF
<400> 47
   ccagagagtg agagacatta act 23
<210> 48
   <211> 23
   <212> RNA
   <213> Artificial Sequence
<220>
   <223> hCTGF
<400> 48
   cagtggaact acattagtac aca 23
<210> 49
   <211> 23
   <212> RNA
   <213> Artificial Sequence
<220>
   <223> hCTGF
<400> 49
   tctgaacacc ataggtagaa tgt 23
<210> 50
   <211> 23
   <212> RNA
   <213> Artificial Sequence
<220>
   <223> hCTGF
<400> 50
   agtgtccttg gcaggctgat ttc 23
<210> 51
   <211> 23
   <212> RNA
   <213> Artificial Sequence
<220>
   <223> hCTGF
<400> 51
   catgtttgca cctttctagt tga 23
<210> 52
   <211> 23
   <212> RNA
   <213> Artificial Sequence
<220>
   <223> hCTGF
<400> 52
   tgctcactga cctgcctgta gcc 23
<210> 53
   <211> 23
   <212> RNA
   <213> Artificial Sequence
<220>
   <223> hCTGF
<400> 53
   ctccctgcat cttcggtggt acg 23
<210> 54
   <211> 23
   <212> RNA
   <213> Artificial Sequence
<220>
   <223> hCTGF
<400> 54
   agcctcaatt tctgaacacc ata 23
<210> 55
   <211> 23
   <212> RNA
   <213> Artificial Sequence
<220>
   <223> hCTGF
<400> 55
   tgccattaca actgtcccgg aga 23
<210> 56
   <211> 23
   <212> RNA
   <213> Artificial Sequence
<220>
   <223> hCTGF
<400> 56
   acctgtgcct gccattacaa ctg 23
<210> 57
   <211> 23
   <212> RNA
   <213> Artificial Sequence
<220>
   <223> hCTGF
<400> 57
   agcagaaagg ttagtatcat cag 23
<210> 58
   <211> 23
   <212> RNA
   <213> Artificial Sequence
<220>
   <223> hCTGF
<400> 58
   gtgcagcatg gacgttcgtc tgc 23
<210> 59
   <211> 23
   <212> RNA
   <213> Artificial Sequence
<220>
   <223> hCTGF
<400> 59
   ctgcaccagc atgaagacat acc 23
<210> 60
   <211> 23
   <212> RNA
   <213> Artificial Sequence
<220>
   <223> hCTGF
<400> 60
   gacctgtgcc tgccattaca act 23
<210> 61
   <211> 23
   <212> RNA
   <213> Artificial Sequence
<220>
   <223> hCTGF
<400> 61
   aagagactga gtcaagttgt tcc 23
<210> 62
   <211> 23
   <212> RNA
   <213> Artificial Sequence
<220>
   <223> hCTGF
<400> 62
   gtgtccttgg caggctgatt tct 23
<210> 63
   <211> 23
   <212> RNA
   <213> Artificial Sequence
<220>
   <223> hCTGF
<400> 63
   ttgagagtgt gaccaaaagt tac 23
<210> 64
   <211> 23
   <212> RNA
   <213> Artificial Sequence
<220>
   <223> hCTGF
<400> 64
   gtgtgaccaa aagttacatg ttt 23
<210> 65
   <211> 23
   <212> RNA
   <213> Artificial Sequence
<220>
   <223> hCTGF
<400> 65
   ggcaaaaagt gcatccgtac tcc 23
<210> 66
   <211> 23
   <212> RNA
   <213> Artificial Sequence
<220>
   <223> hCTGF
<400> 66
   tcctgtcgat tagactggac agc 23
<210> 67
   <211> 23
   <212> RNA
   <213> Artificial Sequence
<220>
   <223> hCTGF
<400> 67
   aggaaatgtg gtagcctcac ttt 23
<210> 68
   <211> 23
   <212> RNA
   <213> Artificial Sequence
<220>
   <223> hCTGF
<400> 68
   ctgcatcttc ggtggtacgg tgt 23
<210> 69
   <211> 23
   <212> RNA
   <213> Artificial Sequence
<220>
   <223> hCTGF
<400> 69
   gcccaaggac caaaccgtgg ttg 23
<210> 70
   <211> 23
   <212> RNA
   <213> Artificial Sequence
<220>
   <223> hCTGF
<400> 70
   taactcatta gactggaact tga 23
<210> 71
   <211> 23
   <212> RNA
   <213> Artificial Sequence
<220>
   <223> hCTGF
<400> 71
   tgtgcattct ccagccatca aga 23
<210> 72
   <211> 23
   <212> RNA
   <213> Artificial Sequence
<220>
   <223> hCTGF 72
<400> 72
   tcgttcaaag catgaaatgg ata 23
<210> 73
   <211> 23
   <212> RNA
   <213> Artificial Sequence
<220>
   <223> hCTGF 73
<400> 73
   cagcatgaag acataccgag cta 23
<210> 74
   <211> 23
   <212> RNA
   <213> Artificial Sequence
<220>
   <223> hCTGF
<400> 74
   agattcccac ccaattcaaa aca 23
<210> 75
   <211> 23
   <212> RNA
   <213> Artificial Sequence
<220>
   <223> hCTGF
<400> 75
   cattgtgcca tgtcaaacaa ata 23
<210> 76
   <211> 23
   <212> RNA
   <213> Artificial Sequence
<220>
   <223> hCTGF
<400> 76
   cagtgtcctt ggcaggctga ttt 23
<210> 77
   <211> 23
   <212> RNA
   <213> Artificial Sequence
<220>
   <223> hCTGF
<400> 77
   ccctgcatct tcggtggtac ggt 23
<210> 78
   <211> 23
   <212> RNA
   <213> Artificial Sequence
<220>
   <223> hCTGF
<400> 78
   gagctaaatt ctgtggagta tgt 23
<210> 79
   <211> 23
   <212> RNA
   <213> Artificial Sequence
<220>
   <223> hCTGF
<400> 79
   aagattccca cccaattcaa aac 23
<210> 80
   <211> 23
   <212> RNA
   <213> Artificial Sequence
<220>
   <223> hCTGF
<400> 80
   tcccacccaa ttcaaaacat tgt 23
<210> 81
   <211> 23
   <212> RNA
   <213> Artificial Sequence
<220>
   <223> hCTGF
<400> 81
   cagcagaaag gttagtatca tca 23
<210> 82
   <211> 23
   <212> RNA
   <213> Artificial Sequence
<220>
   <223> hCTGF
<400> 82
   acgagtaata tgcctgctat ttg 23
<210> 83
   <211> 23
   <212> RNA
   <213> Artificial Sequence
<220>
   <223> hCTGF
<400> 83
   atcaagagac tgagtcaagt tgt 23
<210> 84
   <211> 23
   <212> RNA
   <213> Artificial Sequence
<220>
   <223> hCTGF
<400> 84
   atcggaatcc tgtcgattag act 23
<210> 85
   <211> 23
   <212> RNA
   <213> Artificial Sequence
<220>
   <223> hCTGF
<400> 85
   tggtagcctc acttttaatg aac 23
<210> 86
   <211> 23
   <212> RNA
   <213> Artificial Sequence
<220>
   <223> hCTGF
<400> 86
   gactgttttt cggacagttt att 23
<210> 87
   <211> 23
   <212> RNA
   <213> Artificial Sequence
<220>
   <223> hCTGF
<400> 87
   tacatgtttg cacctttcta gtt 23
<210> 88
   <211> 23
   <212> RNA
   <213> Artificial Sequence
<220>
   <223> hCTGF
<400> 88
   aaagttacat gtttgcacct ttc 23
<210> 89
   <211> 23
   <212> RNA
   <213> Artificial Sequence
<220>
   <223> hCTGF
<400> 89
   ttctactttg atatgactgt ttt 23
<210> 90
   <211> 23
   <212> RNA
   <213> Artificial Sequence
<220>
   <223> hCTGF
<400> 90
   cacccgggtt accaatgaca acg 23
<210> 91
   <211> 23
   <212> RNA
   <213> Artificial Sequence
<220>
   <223> hCTGF
<400> 91
   aagtgcatcc gtactcccaa aat 23
<210> 92
   <211> 23
   <212> RNA
   <213> Artificial Sequence
<220>
   <223> hCTGF
<400> 92
   aagcctatca agtttgagct ttc 23
<210> 93
   <211> 23
   <212> RNA
   <213> Artificial Sequence
<220>
   <223> hCTGF
<400> 93
   agcctatcaa gtttgagctt tct 23
<210> 94
   <211> 23
   <212> RNA
   <213> Artificial Sequence
<220>
   <223> hCTGF
<400> 94
   ctgtcccgga gacaatgaca tct 23
<210> 95
   <211> 23
   <212> RNA
   <213> Artificial Sequence
<220>
   <223> hCTGF
<400> 95
   gtggaactac attagtacac agc 23
<210> 96
   <211> 23
   <212> RNA
   <213> Artificial Sequence
<220>
   <223> hCTGF
<400> 96
   agccatcaag agactgagtc aag 23
<210> 97
   <211> 23
   <212> RNA
   <213> Artificial Sequence
<220>
   <223> hCTGF
<400> 97
   ttctgattcg aatgacactg ttc 23
<210> 98
   <211> 23
   <212> RNA
   <213> Artificial Sequence
<220>
   <223> hCTGF
<400> 98
   ctgttcagga atcggaatcc tgt 23
<210> 99
   <211> 23
   <212> RNA
   <213> Artificial Sequence
<220>
   <223> hCTGF
<400> 99
   tggacagctt gtggcaagtg aat 23
<210> 100
   <211> 23
   <212> RNA
   <213> Artificial Sequence
<220>
   <223> hCTGF
<400> 100
   tggcaagtga atttgcctgt aac 23
<210> 101
   <211> 23
   <212> RNA
   <213> Artificial Sequence
<220>
   <223> hCyr61
<400> 101
   gccagtgtac agcagcctga aaa 23
<210> 102
   <211> 23
   <212> RNA
   <213> Artificial Sequence
<220>
   <223> hCyr61
<400> 102
   ctcaacgagg actgcagcaa aac 23
<210> 103
   <211> 23
   <212> RNA
   <213> Artificial Sequence
<220>
   <223> hCyr61
<400> 103
   gggacactcc atgagtgtct gtg 23
<210> 104
   <211> 23
   <212> RNA
   <213> Artificial Sequence
<220>
   <223> hCyr61
<400> 104
   gggcagaccc tgtgaatata act 23
<210> 105
   <211> 23
   <212> RNA
   <213> Artificial Sequence
<220>
   <223> hCyr61
<400> 105
   gggaaagttt ccagcccaac tgt 23
<210> 106
   <211> 23
   <212> RNA
   <213> Artificial Sequence
<220>
   <223> hCyr61
<400> 106
   ttgttcaaac aacttcatgg tcc 23
<210> 107
   <211> 23
   <212> RNA
   <213> Artificial Sequence
<220>
   <223> hCyr61
<400> 107
   aagcagcgtt tcccttctac agg 23
<210> 108
   <211> 23
   <212> RNA
   <213> Artificial Sequence
<220>
   <223> hCyr61
<400> 108
   ttgtagaaag gaagccttgc tca 23
<210> 109
   <211> 23
   <212> RNA
   <213> Artificial Sequence
<220>
   <223> hCyr61
<400> 109
   ttggagcaca tgttactgct tca 23
<210> 110
   <211> 23
   <212> RNA
   <213> Artificial Sequence
<220>
   <223> hCyr61
<400> 110
   tggagcttgt ggagttgatg act 23
<210> 111
   <211> 23
   <212> RNA
   <213> Artificial Sequence
<220>
   <223> hCyr61
<400> 111
   tcccagtgct caaagacctg tgg 23
<210> 112
   <211> 23
   <212> RNA
   <213> Artificial Sequence
<220>
   <223> hCyr61
<400> 112
   aactggtatc tccacacgag tta 23
<210> 113
   <211> 23
   <212> RNA
   <213> Artificial Sequence
<220>
   <223> hCyr61
<400> 113
   tggacactaa tgcagccacg att 23
<210> 114
   <211> 23
   <212> RNA
   <213> Artificial Sequence
<220>
   <223> hCyr61
<400> 114
   gtgcggatgg acactaatgc agc 23
<210> 115
   <211> 23
   <212> RNA
   <213> Artificial Sequence
<220>
   <223> hCyr61
<400> 115
   tagtcgtcac ccttctccac ttg 23
<210> 116
   <211> 23
   <212> RNA
   <213> Artificial Sequence
<220>
   <223> hCyr61
<400> 116
   tggtcaaagt taccgggcag tgc 23
<210> 117
   <211> 23
   <212> RNA
   <213> Artificial Sequence
<220>
   <223> hCyr61
<400> 117
   agccttgctc attcttgagg agc 23
<210> 118
   <211> 23
   <212> RNA
   <213> Artificial Sequence
<220>
   <223> hCyr61
<400> 118
   ttcggtattt ttagaggtgc tcc 23
<210> 119
   <211> 23
   <212> RNA
   <213> Artificial Sequence
<220>
   <223> hCyr61
<400> 119
   cacgacattg tatgaagcac aat 23
<210> 120
   <211> 23
   <212> RNA
   <213> Artificial Sequence
<220>
   <223> hCyr61
<400> 120
   ctcattcttg aggagcatta agg 23
<210> 121
   <211> 23
   <212> RNA
   <213> Artificial Sequence
<220>
   <223> hCyr61
<400> 121
   gtgaaagaaa cccggatttg tga 23
<210> 122
   <211> 23
   <212> RNA
   <213> Artificial Sequence
<220>
   <223> hCyr61
<400> 122
   cccgaaccag tcaggtttac tta 23
<210> 123
   <211> 23
   <212> RNA
   <213> Artificial Sequence
<220>
   <223> hCyr61
<400> 123
   atgcagccac gattggagaa tac 23
<210> 124
   <211> 23
   <212> RNA
   <213> Artificial Sequence
<220>
   <223> hCyr61
<400> 124
   ttcgtccttt gacaaaagta aat 23
<210> 125
   <211> 23
   <212> RNA
   <213> Artificial Sequence
<220>
   <223> hCyr61
<400> 125
   gctcaacgag gactgcagca aaa 23
<210> 126
   <211> 23
   <212> RNA
   <213> Artificial Sequence
<220>
   <223> hCyr61
<400> 126
   aaaagtaaat gggagggcat tcc 23
<210> 127
   <211> 23
   <212> RNA
   <213> Artificial Sequence
<220>
   <223> hCyr61
<400> 127
   gtgtatgcca ttcggtattt tta 23
<210> 128
   <211> 23
   <212> RNA
   <213> Artificial Sequence
<220>
   <223> hCyr61
<400> 128
   ctgagtgtat gccattcggt att 23
<210> 129
   <211> 23
   <212> RNA
   <213> Artificial Sequence
<220>
   <223> hCyr61
<400> 129
   accctcggct ggtcaaagtt acc 23
<210> 130
   <211> 23
   <212> RNA
   <213> Artificial Sequence
<220>
   <223> hCyr61
<400> 130
   cccccgaacc agtcaggttt act 23
<210> 131
   <211> 23
   <212> RNA
   <213> Artificial Sequence
<220>
   <223> hCyr61
<400> 131
   ttccaagaac gtcatgatga tcc 23
<210> 132
   <211> 23
   <212> RNA
   <213> Artificial Sequence
<220>
   <223> hCyr61
<400> 132
   gtgatgggac tcattgtaga aag 23
<210> 133
   <211> 23
   <212> RNA
   <213> Artificial Sequence
<220>
   <223> hCyr61
<400> 133
   gccacgattg gagaatactt tgc 23
<210> 134
   <211> 23
   <212> RNA
   <213> Artificial Sequence
<220>
   <223> hCyr61
<400> 134
   aagcttttat tcgtcctttg aca 23
<210> 135
   <211> 23
   <212> RNA
   <213> Artificial Sequence
<220>
   <223> hCyr61
<400> 135
   ggacactaat gcagccacga ttg 23
<210> 136
   <211> 23
   <212> RNA
   <213> Artificial Sequence
<220>
   <223> hCyr61
<400> 136
   ttcatggtcc cagtgctcaa aga 23
<210> 137
   <211> 23
   <212> RNA
   <213> Artificial Sequence
<220>
   <223> hCyr61
<400> 137
   cgccttagtc gtcacccttc tcc 23
<210> 138
   <211> 23
   <212> RNA
   <213> Artificial Sequence
<220>
   <223> hCyr61
<400> 138
   ggggacactc catgagtgtc tgt 23
<210> 139
   <211> 23
   <212> RNA
   <213> Artificial Sequence
<220>
   <223> hCyr61
<400> 139
   cttgctcatt cttgaggagc att 23
<210> 140
   <211> 23
   <212> RNA
   <213> Artificial Sequence
<220>
   <223> hCyr61
<400> 140
   ggccagaaat gtattgttca aac 23
<210> 141
   <211> 23
   <212> RNA
   <213> Artificial Sequence
<220>
   <223> hCyr61
<400> 141
   ccagtgtaca gcagcctgaa aaa 23
<210> 142
   <211> 23
   <212> RNA
   <213> Artificial Sequence
<220>
   <223> hCyr61
<400> 142
   cccaaccctc ggctggtcaa agt 23
<210> 143
   <211> 23
   <212> RNA
   <213> Artificial Sequence
<220>
   <223> hCyr61
<400> 143
   cggatttgtg aggtgcggcc ttg 23
<210> 144
   <211> 23
   <212> RNA
   <213> Artificial Sequence
<220>
   <223> hCyr61
<400> 144
   gccttgtgaa agaaacccgg att 23
<210> 145
   <211> 23
   <212> RNA
   <213> Artificial Sequence
<220>
   <223> hCyr61
<400> 145
   ccctgagtgc cgccttgtga aag 23
<210> 146
   <211> 23
   <212> RNA
   <213> Artificial Sequence
<220>
   <223> hCyr61
<400> 146
   gggtctgtga cgaggatagt atc 23
<210> 147
   <211> 23
   <212> RNA
   <213> Artificial Sequence
<220>
   <223> hCyr61
<400> 147
   agcacatgtt actgcttcat ttt 23
<210> 148
   <211> 23
   <212> RNA
   <213> Artificial Sequence
<220>
   <223> hCyr61
<400> 148
   accctgtgaa tataactcca gaa 23
<210> 149
   <211> 23
   <212> RNA
   <213> Artificial Sequence
<220>
   <223> hCyr61
<400> 149
   gactcattgt agaaaggaag cct 23
<210> 150
   <211> 23
   <212> RNA
   <213> Artificial Sequence
<220>
   <223> hCyr61
<400> 150
   caggacttat tgggatacag cag 23
<210> 151
   <211> 23
   <212> RNA
   <213> Artificial Sequence
<220>
   <223> hCyr61
<400> 151
   cgagttacca atgacaaccc tga 23
<210> 152
   <211> 23
   <212> RNA
   <213> Artificial Sequence
<220>
   <223> hCyr61
<400> 152
   ggtttactta cgctggatgt ttg 23
<210> 153
   <211> 23
   <212> RNA
   <213> Artificial Sequence
<220>
   <223> hCyr61
<400> 153
   aagattagtt ggacagttta aag 23
<210> 154
   <211> 23
   <212> RNA
   <213> Artificial Sequence
<220>
   <223> hCyr61
<400> 154
   cgaaccagtc aggtttactt acg 23
<210> 155
   <211> 23
   <212> RNA
   <213> Artificial Sequence
<220>
   <223> hCyr61
<400> 155
   tgatgggact cattgtagaa agg 23
<210> 156
   <211> 23
   <212> RNA
   <213> Artificial Sequence
<220>
   <223> hCyr61
<400> 156
   gatccagtcc tgcaaatgca act 23
<210> 157
   <211> 23
   <212> RNA
   <213> Artificial Sequence
<220>
   <223> hCyr61
<400> 157
   ggagcacatg ttactgcttc att 23
<210> 158
   <211> 23
   <212> RNA
   <213> Artificial Sequence
<220>
   <223> hCyr61
<400> 158
   ttgtatgaag cacaataaag att 23
<210> 159
   <211> 23
   <212> RNA
   <213> Artificial Sequence
<220>
   <223> hCyr61
<400> 159
   cacacctaga caaacaaggg aga 23
<210> 160
   <211> 23
   <212> RNA
   <213> Artificial Sequence
<220>
   <223> hCyr61
<400> 160
   tgtatgccat tcggtatttt tag 23
<210> 161
   <211> 23
   <212> RNA
   <213> Artificial Sequence
<220>
   <223> hCyr61
<400> 161
   aaccctttac aaggccagaa atg 23
<210> 162
   <211> 23
   <212> RNA
   <213> Artificial Sequence
<220>
   <223> hCyr61
<400> 162
   accctttaca aggccagaaa tgt 23
<210> 163
   <211> 23
   <212> RNA
   <213> Artificial Sequence
<220>
   <223> hCyr61
<400> 163
   ctggtatctc cacacgagtt acc 23
<210> 164
   <211> 23
   <212> RNA
   <213> Artificial Sequence
<220>
   <223> hCyr61
<400> 164
   ctccacacga gttaccaatg aca 23
<210> 165
   <211> 23
   <212> RNA
   <213> Artificial Sequence
<220>
   <223> hCyr61
<400> 165
   ttgtgaaaga aacccggatt tgt 23
<210> 166
   <211> 23
   <212> RNA
   <213> Artificial Sequence
<220>
   <223> hCyr61
<400> 166
   tccagggcac acctagacaa aca 23
<210> 167
   <211> 23
   <212> RNA
   <213> Artificial Sequence
<220>
   <223> hCyr61
<400> 167
   tgggtgatgg gactcattgt aga 23
<210> 168
   <211> 23
   <212> RNA
   <213> Artificial Sequence
<220>
   <223> hCyr61
<400> 168
   tgcagccacg attggagaat act 23
<210> 169
   <211> 23
   <212> RNA
   <213> Artificial Sequence
<220>
   <223> hCyr61
<400> 169
   cagccacgat tggagaatac ttt 23
<210> 170
   <211> 23
   <212> RNA
   <213> Artificial Sequence
<220>
   <223> hCyr61
<400> 170
   gagtgtatgc cattcggtat ttt 23
<210> 171
   <211> 23
   <212> RNA
   <213> Artificial Sequence
<220>
   <223> hCyr61
<400> 171
   atgccattcg gtatttttag agg 23
<210> 172
   <211> 23
   <212> RNA
   <213> Artificial Sequence
<220>
   <223> hCyr61
<400> 172
   gggcacacct agacaaacaa ggg 23
<210> 173
   <211> 23
   <212> RNA
   <213> Artificial Sequence
<220>
   <223> hCyr61
<400> 173
   ggtgatggga ctcattgtag aaa 23
<210> 174
   <211> 23
   <212> RNA
   <213> Artificial Sequence
<220>
   <223> hCyr61
<400> 174
   gaacgtcatg atgatccagt cct 23
<210> 175
   <211> 23
   <212> RNA
   <213> Artificial Sequence
<220>
   <223> hCyr61
<400> 175
   ttggacagtt taaagctttt att 23
<210> 176
   <211> 23
   <212> RNA
   <213> Artificial Sequence
<220>
   <223> hCyr61
<400> 176
   agcccaactg taaacatcag tgc 23
<210> 177
   <211> 23
   <212> RNA
   <213> Artificial Sequence
<220>
   <223> hCyr61
<400> 177
   cacacgagtt accaatgaca acc 23
<210> 178
   <211> 23
   <212> RNA
   <213> Artificial Sequence
<220>
   <223> hCyr61
<400> 178
   atgaagcagc gtttcccttc tac 23
<210> 179
   <211> 23
   <212> RNA
   <213> Artificial Sequence
<220>
   <223> hCyr61
<400> 179
   gacactaatg cagccacgat tgg 23
<210> 180
   <211> 23
   <212> RNA
   <213> Artificial Sequence
<220>
   <223> hCyr61
<400> 180
   aacatgtatt gaacacgaca ttg 23
<210> 181
   <211> 23
   <212> RNA
   <213> Artificial Sequence
<220>
   <223> hCyr61
<400> 181
   acgacattgt atgaagcaca ata 23
<210> 182
   <211> 23
   <212> RNA
   <213> Artificial Sequence
<220>
   <223> hCyr61
<400> 182
   gacattgtat gaagcacaat aaa 23
<210> 183
   <211> 23
   <212> RNA
   <213> Artificial Sequence
<220>
   <223> hCyr61
<400> 183
   agttggacag tttaaagctt tta 23
<210> 184
   <211> 23
   <212> RNA
   <213> Artificial Sequence
<220>
   <223> hCyr61
<400> 184
   gaccctgtga atataactcc aga 23
<210> 185
   <211> 23
   <212> RNA
   <213> Artificial Sequence
<220>
   <223> hCyr61
<400> 185
   cacctagaca aacaagggag aag 23
<210> 186
   <211> 23
   <212> RNA
   <213> Artificial Sequence
<220>
   <223> hCyr61
<400> 186
   ctcattgtag aaaggaagcc ttg 23
<210> 187
   <211> 23
   <212> RNA
   <213> Artificial Sequence
<220>
   <223> hCyr61
<400> 187
   aagcatattt tctctaggct ttt 23
<210> 188
   <211> 23
   <212> RNA
   <213> Artificial Sequence
<220>
   <223> hCyr61
<400> 188
   gggcattcca tcccttcctg aag 23
<210> 189
   <211> 23
   <212> RNA
   <213> Artificial Sequence
<220>
   <223> hCyr61
<400> 189
   ccctttacaa ggccagaaat gta 23
<210> 190
   <211> 23
   <212> RNA
   <213> Artificial Sequence
<220>
   <223> hCyr61
<400> 190
   ttgagtgtga agaaataccg gcc 23
<210> 191
   <211> 23
   <212> RNA
   <213> Artificial Sequence
<220>
   <223> hCyr61
<400> 191
   gtggacagcc agtgtacagc agc 23
<210> 192
   <211> 23
   <212> RNA
   <213> Artificial Sequence
<220>
   <223> hCyr61
<400> 192
   ttacgctgga tgtttgagtg tga 23
<210> 193
   <211> 23
   <212> RNA
   <213> Artificial Sequence
<220>
   <223> hCyr61
<400> 193
   tttccagccc aactgtaaac atc 23
<210> 194
   <211> 23
   <212> RNA
   <213> Artificial Sequence
<220>
   <223> hCyr61
<400> 194
   aaacatcagt gcacatgtat tga 23
<210> 195
   <211> 23
   <212> RNA
   <213> Artificial Sequence
<220>
   <223> hCyr61
<400> 195
   ggtctgtgac gaggatagta tca 23
<210> 196
   <211> 23
   <212> RNA
   <213> Artificial Sequence
<220>
   <223> hCyr61
<400> 196
   agcattaagg tatttcgaaa ctg 23
<210> 197
   <211> 23
   <212> RNA
   <213> Artificial Sequence
<220>
   <223> hCyr61
<400> 197
   tgggtttcca gggcacacct aga 23
<210> 198
   <211> 23
   <212> RNA
   <213> Artificial Sequence
<220>
   <223> hCyr61
<400> 198
   ttgctaagca tattttctct agg 23
<210> 199
   <211> 23
   <212> RNA
   <213> Artificial Sequence
<220>
   <223> hCyr61
<400> 199
   cgaggagtgg gtctgtgacg agg 23
<210> 200
   <211> 23
   <212> RNA
   <213> Artificial Sequence
<220>
   <223> hCyr61
<400> 200
   cacatgtatt gatggcgccg tgg 23
<210> 201
   <211> 23
   <212> RNA
   <213> Artificial Sequence
<220>
   <223> hPlekho1
<400> 201
   ctgaccttgg acttgatcca aga 23
<210> 202
   <211> 23
   <212> RNA
   <213> Artificial Sequence
<220>
   <223> hPlekho1
<400> 202
   ttgagtgaaa ccggagctac aaa 23
<210> 203
   <211> 23
   <212> RNA
   <213> Artificial Sequence
<220>
   <223> hPlekho1
<400> 203
   agccaagaac cgtatcttgg atg 23
<210> 204
   <211> 23
   <212> RNA
   <213> Artificial Sequence
<220>
   <223> hPlekho1
<400> 204
   caagaaccgt atcttggatg agg 23
<210> 205
   <211> 23
   <212> RNA
   <213> Artificial Sequence
<220>
   <223> hPlekho1
<400> 205
   cccctgagga accaacctct tgt 23
<210> 206
   <211> 23
   <212> RNA
   <213> Artificial Sequence
<220>
   <223> hPlekho1
<400> 206
   aagttgacgc ccacagagaa agg 23
<210> 207
   <211> 23
   <212> RNA
   <213> Artificial Sequence
<220>
   <223> hPlekho1
<400> 207
   gagatttgga aaaaccgcta tgt 23
<210> 208
   <211> 23
   <212> RNA
   <213> Artificial Sequence
<220>
   <223> hPlekho1
<400> 208
   tgctgagagc tttcgggttg acc 23
<210> 209
   <211> 23
   <212> RNA
   <213> Artificial Sequence
<220>
   <223> hPlekho1
<400> 209
   gggtctggaa cttgtcgggt tgg 23
<210> 210
   <211> 23
   <212> RNA
   <213> Artificial Sequence
<220>
   <223> hPlekho1
<400> 210
   tgggagaggc atcatcgaat tgg 23
<210> 211
   <211> 23
   <212> RNA
   <213> Artificial Sequence
<220>
   <223> hPlekho1
<400> 211
   atcgtggatc aatgccctca act 23
<210> 212
   <211> 23
   <212> RNA
   <213> Artificial Sequence
<220>
   <223> hPlekho1
<400> 212
   acctcttgtg ctgagagctt tcg 23
<210> 213
   <211> 23
   <212> RNA
   <213> Artificial Sequence
<220>
   <223> hPlekho1
<400> 213
   cacggcaccc aacctgatct tcc 23
<210> 214
   <211> 23
   <212> RNA
   <213> Artificial Sequence
<220>
   <223> hPlekho1
<400> 214
   gaggacagct atcttgccca tcc 23
<210> 215
   <211> 23
   <212> RNA
   <213> Artificial Sequence
<220>
   <223> hPlekho1
<400> 215
   gacacctaat ggctgtggct tcc 23
<210> 216
   <211> 23
   <212> RNA
   <213> Artificial Sequence
<220>
   <223> hPlekho1
<400> 216
   gtcgggttgg acagactctt atc 23
<210> 217
   <211> 23
   <212> RNA
   <213> Artificial Sequence
<220>
   <223> hPlekho1
<400> 217
   tcagtaccgg aagagcctga tgt 23
<210> 218
   <211> 23
   <212> RNA
   <213> Artificial Sequence
<220>
   <223> hPlekho1
<400> 218
   ttatctccgt gttgctggat aaa 23
<210> 219
   <211> 23
   <212> RNA
   <213> Artificial Sequence
<220>
   <223> hPlekho1
<400> 219
   tccgccggat tctgagtcag agc 23
<210> 220
   <211> 23
   <212> RNA
   <213> Artificial Sequence
<220>
   <223> hPlekho1
<400> 220
   ctcgagacag ggcaaaaatc cag 23
<210> 221
   <211> 23
   <212> RNA
   <213> Artificial Sequence
<220>
   <223> hPlekho1
<400> 221
   aaccaacctc ttgtgctgag agc 23
<210> 222
   <211> 23
   <212> RNA
   <213> Artificial Sequence
<220>
   <223> hPlekho1
<400> 222
   caggacctgg tagcaaggaa act 23
<210> 223
   <211> 23
   <212> RNA
   <213> Artificial Sequence
<220>
   <223> hPlekho1
<400> 223
   ctgggagagg catcatcgaa ttg 23
<210> 224
   <211> 23
   <212> RNA
   <213> Artificial Sequence
<220>
   <223> hPlekho1
<400> 224
   gtccagaaga gaaggaatcg tgg 23
<210> 225
   <211> 23
   <212> RNA
   <213> Artificial Sequence
<220>
   <223> hPlekho1
<400> 225
   gccttgagtg aaaccggagc tac 23
<210> 226
   <211> 23
   <212> RNA
   <213> Artificial Sequence
<220>
   <223> hPlekho1
<400> 226
   ttggaaaaac cgctatgtgg tgc 23
<210> 227
   <211> 23
   <212> RNA
   <213> Artificial Sequence
<220>
   <223> hPlekho1
<400> 227
   gaagttgacg cccacagaga aag 23
<210> 228
   <211> 23
   <212> RNA
   <213> Artificial Sequence
<220>
   <223> hPlekho1
<400> 228
   gctgggagag gcatcatcga att 23
<210> 229
   <211> 23
   <212> RNA
   <213> Artificial Sequence
<220>
   <223> hPlekho1
<400> 229
   gaacttgtcg ggttggacag act 23
<210> 230
   <211> 23
   <212> RNA
   <213> Artificial Sequence
<220>
   <223> hPlekho1
<400> 230
   acagactctt atctccgtgt tgc 23
<210> 231
   <211> 23
   <212> RNA
   <213> Artificial Sequence
<220>
   <223> hPlekho1
<400> 231
   aggtcaccgt tgaggaggac agc 23
<210> 232
   <211> 23
   <212> RNA
   <213> Artificial Sequence
<220>
   <223> hPlekho1
<400> 232
   tccctggagg agatcctatc tca 23
<210> 233
   <211> 23
   <212> RNA
   <213> Artificial Sequence
<220>
   <223> hPlekho1
<400> 233
   gggagctgag agacctgtac aga 23
<210> 234
   <211> 23
   <212> RNA
   <213> Artificial Sequence
<220>
   <223> hPlekho1
<400> 234
   ctgagagacc tgtacagaca gat 23
<210> 235
   <211> 23
   <212> RNA
   <213> Artificial Sequence
<220>
   <223> hPlekho1
<400> 235
   acagggcaaa aatccagcac tcc 23
<210> 236
   <211> 23
   <212> RNA
   <213> Artificial Sequence
<220>
   <223> hPlekho1
<400> 236
   gagctttcgg gttgacctgg aca 23
<210> 237
   <211> 23
   <212> RNA
   <213> Artificial Sequence
<220>
   <223> hPlekho1
<400> 237
   cactcgagac agggcaaaaa tcc 23
<210> 238
   <211> 23
   <212> RNA
   <213> Artificial Sequence
<220>
   <223> hPlekho1
<400> 238
   gtccggaaat tctgcgggaa agg 23
<210> 239
   <211> 23
   <212> RNA
   <213> Artificial Sequence
<220>
   <223> hPlekho1
<400> 239
   tgggatgctg accttggact tga 23
<210> 240
   <211> 23
   <212> RNA
   <213> Artificial Sequence
<220>
   <223> hPlekho1
<400> 240
   gaccttggac ttgatccaag agg 23
<210> 241
   <211> 23
   <212> RNA
   <213> Artificial Sequence
<220>
   <223> hPlekho1
<400> 241
   aacctcttgt gctgagagct ttc 23
<210> 242
   <211> 23
   <212> RNA
   <213> Artificial Sequence
<220>
   <223> hPlekho1
<400> 242
   gaagaagaac aattccgcca agc 23
<210> 243
   <211> 23
   <212> RNA
   <213> Artificial Sequence
<220>
   <223> hPlekho1
<400> 243
   ttcaagaggt atttgacctg agt 23
<210> 244
   <211> 23
   <212> RNA
   <213> Artificial Sequence
<220>
   <223> hPlekho1
<400> 244
   aatcgtggat caatgccctc aac 23
<210> 245
   <211> 23
   <212> RNA
   <213> Artificial Sequence
<220>
   <223> hPlekho1
<400> 245
   cttggacttg atccaagagg aag 23
<210> 246
   <211> 23
   <212> RNA
   <213> Artificial Sequence
<220>
   <223> hPlekho1
<400> 246
   gacagactct tatctccgtg ttg 23
<210> 247
   <211> 23
   <212> RNA
   <213> Artificial Sequence
<220>
   <223> hPlekho1
<400> 247
   agagaaggaa tcgtggatca atg 23
<210> 248
   <211> 23
   <212> RNA
   <213> Artificial Sequence
<220>
   <223> hPlekho1
<400> 248
   ggggtctgga acttgtcggg ttg 23
<210> 249
   <211> 23
   <212> RNA
   <213> Artificial Sequence
<220>
   <223> hPlekho1
<400> 249
   cacccgagcc aagaaccgta tct 23
<210> 250
   <211> 23
   <212> RNA
   <213> Artificial Sequence
<220>
   <223> hPlekho1
<400> 250
   caccgttgag gaggacagct atc 23
<210> 251
   <211> 23
   <212> RNA
   <213> Artificial Sequence
<220>
   <223> hPlekho1
<400> 251
   gagaaggaat cgtggatcaa tgc 23
<210> 252
   <211> 23
   <212> RNA
   <213> Artificial Sequence
<220>
   <223> hPlekho1
<400> 252
   cgtgtgtgtg cgagtgcgaa tgc 23
<210> 253
   <211> 23
   <212> RNA
   <213> Artificial Sequence
<220>
   <223> hPlekho1
<400> 253
   cctggcagtg agtccagaag aga 23
<210> 254
   <211> 23
   <212> RNA
   <213> Artificial Sequence
<220>
   <223> hPlekho1
<400> 254
   cctgaggaac caacctcttg tgc 23
<210> 255
   <211> 23
   <212> RNA
   <213> Artificial Sequence
<220>
   <223> hPlekho1
<400> 255
   gctgagagac ctgtacagac aga 23
<210> 256
   <211> 23
   <212> RNA
   <213> Artificial Sequence
<220>
   <223> hPlekho1
<400> 256
   acctctacct cggatgggat gct 23
<210> 257
   <211> 23
   <212> RNA
   <213> Artificial Sequence
<220>
   <223> hPlekho1
<400> 257
   tggatcaatg ccctcaactc tgc 23
<210> 258
   <211> 23
   <212> RNA
   <213> Artificial Sequence
<220>
   <223> hPlekho1
<400> 258
   tcgagacagg gcaaaaatcc agc 23
<210> 259
   <211> 23
   <212> RNA
   <213> Artificial Sequence
<220>
   <223> hPlekho1
<400> 259
   tgagtgaaac cggagctaca aag 23
<210> 260
   <211> 23
   <212> RNA
   <213> Artificial Sequence
<220>
   <223> hPlekho1
<400> 260
   ctcaactctg ccatcacccg agc 23
<210> 261
   <211> 23
   <212> RNA
   <213> Artificial Sequence
<220>
   <223> hPlekho1
<400> 261
   aatcatagca agtttactct tgc 23
<210> 262
   <211> 23
   <212> RNA
   <213> Artificial Sequence
<220>
   <223> hPlekho1
<400> 262
   ttggacagac tcttatctcc gtg 23
<210> 263
   <211> 23
   <212> RNA
   <213> Artificial Sequence
<220>
   <223> hPlekho1
<400> 263
   tggacagact cttatctccg tgt 23
<210> 264
   <211> 23
   <212> RNA
   <213> Artificial Sequence
<220>
   <223> hPlekho1
<400> 264
   ccttgagtga aaccggagct aca 23
<210> 265
   <211> 23
   <212> RNA
   <213> Artificial Sequence
<220>
   <223> hPlekho1
<400> 265
   gattttcagg gagatttgga aaa 23
<210> 266
   <211> 23
   <212> RNA
   <213> Artificial Sequence
<220>
   <223> hPlekho1
<400> 266
   aaaccgctat gtggtgctga aag 23
<210> 267
   <211> 23
   <212> RNA
   <213> Artificial Sequence
<220>
   <223> hPlekho1
<400> 267
   agtaccggaa gagcctgatg tga 23
<210> 268
   <211> 23
   <212> RNA
   <213> Artificial Sequence
<220>
   <223> hPlekho1
<400> 268
   cttatctccg tgttgctgga taa 23
<210> 269
   <211> 23
   <212> RNA
   <213> Artificial Sequence
<220>
   <223> hPlekho1
<400> 269
   tcacccgagc caagaaccgt atc 23
<210> 270
   <211> 23
   <212> RNA
   <213> Artificial Sequence
<220>
   <223> hPlekho1
<400> 270
   ctacctcgga tgggatgctg acc 23
<210> 271
   <211> 23
   <212> RNA
   <213> Artificial Sequence
<220>
   <223> hPlekho1
<400> 271
   cccactcgag acagggcaaa aat 23
<210> 272
   <211> 23
   <212> RNA
   <213> Artificial Sequence
<220>
   <223> hPlekho1
<400> 272
   tcgggttgga cagactctta tct 23
<210> 273
   <211> 23
   <212> RNA
   <213> Artificial Sequence
<220>
   <223> hPlekho1
<400> 273
   agacctgtac agacagatgg acc 23
<210> 274
   <211> 23
   <212> RNA
   <213> Artificial Sequence
<220>
   <223> hPlekho1
<400> 274
   cttgagtgaa accggagcta caa 23
<210> 275
   <211> 23
   <212> RNA
   <213> Artificial Sequence
<220>
   <223> hPlekho1
<400> 275
   cggaaattct gcgggaaagg gat 23
<210> 276
   <211> 23
   <212> RNA
   <213> Artificial Sequence
<220>
   <223> hPlekho1
<400> 276
   caacctcttg tgctgagagc ttt 23
<210> 277
   <211> 23
   <212> RNA
   <213> Artificial Sequence
<220>
   <223> hPlekho1
<400> 277
   ctctcccgac cttgggaaaa aac 23
<210> 278
   <211> 23
   <212> RNA
   <213> Artificial Sequence
<220>
   <223> hPlekho1
<400> 278
   atctccgtgt tgctggataa agc 23
<210> 279
   <211> 23
   <212> RNA
   <213> Artificial Sequence
<220>
   <223> hPlekho1
<400> 279
   tggaacttgt cgggttggac aga 23
<210> 280
   <211> 23
   <212> RNA
   <213> Artificial Sequence
<220>
   <223> hPlekho1
<400> 280
   ggaatcgtgg atcaatgccc tca 23
<210> 281
   <211> 23
   <212> RNA
   <213> Artificial Sequence
<220>
   <223> hPlekho1
<400> 281
   ggaggacagc tatcttgccc atc 23
<210> 282
   <211> 23
   <212> RNA
   <213> Artificial Sequence
<220>
   <223> hPlekho1
<400> 282
   tcggctgggt ccggaaattc tgc 23
<210> 283
   <211> 23
   <212> RNA
   <213> Artificial Sequence
<220>
   <223> hPlekho1
<400> 283
   ggacctggta gcaaggaaac tgg 23
<210> 284
   <211> 23
   <212> RNA
   <213> Artificial Sequence
<220>
   <223> hPlekho1
<400> 284
   tcccactcga gacagggcaa aaa 23
<210> 285
   <211> 23
   <212> RNA
   <213> Artificial Sequence
<220>
   <223> hPlekho1
<400> 285
   ctctacctcg gatgggatgc tga 23
<210> 286
   <211> 23
   <212> RNA
   <213> Artificial Sequence
<220>
   <223> hPlekho1
<400> 286
   cggatccagg acctggtagc aag 23
<210> 287
   <211> 23
   <212> RNA
   <213> Artificial Sequence
<220>
   <223> hPlekho1
<400> 287
   cagggagctg agagacctgt aca 23
<210> 288
   <211> 23
   <212> RNA
   <213> Artificial Sequence
<220>
   <223> hPlekho1
<400> 288
   tgaggtcacc gttgaggagg aca 23
<210> 289
   <211> 23
   <212> RNA
   <213> Artificial Sequence
<220>
   <223> hPlekho1
<400> 289
   tcaccgttga ggaggacagc tat 23
<210> 290
   <211> 23
   <212> RNA
   <213> Artificial Sequence
<220>
   <223> hPlekho1
<400> 290
   ccaggcaaag agggtgctgc agg 23
<210> 291
   <211> 23
   <212> RNA
   <213> Artificial Sequence
<220>
   <223> hPlekho1
<400> 291
   gccaagaacc gtatcttgga tga 23
<210> 292
   <211> 23
   <212> RNA
   <213> Artificial Sequence
<220>
   <223> hPlekho1
<400> 292
   gagagacctg tacagacaga tgg 23
<210> 293
   <211> 23
   <212> RNA
   <213> Artificial Sequence
<220>
   <223> hPlekho1
<400> 293
   ggaccagctc tacatctctg aga 23
<210> 294
   <211> 23
   <212> RNA
   <213> Artificial Sequence
<220>
   <223> hPlekho1
<400> 294
   cctggaggag atcctatctc agc 23
<210> 295
   <211> 23
   <212> RNA
   <213> Artificial Sequence
<220>
   <223> hPlekho1
<400> 295
   ccaggacctg gtagcaagga aac 23
<210> 296
   <211> 23
   <212> RNA
   <213> Artificial Sequence
<220>
   <223> hPlekho1
<400> 296
   ccactcgaga cagggcaaaa atc 23
<210> 297
   <211> 23
   <212> RNA
   <213> Artificial Sequence
<220>
   <223> hPlekho1
<400> 297
   cctgtacaga cagatggacc tgc 23
<210> 298
   <211> 23
   <212> RNA
   <213> Artificial Sequence
<220>
   <223> hPlekho1
<400> 298
   cacctctacc tcggatggga tgc 23
<210> 299
   <211> 23
   <212> RNA
   <213> Artificial Sequence
<220>
   <223> hPlekho1
<400> 299
   tcccctgagg aaccaacctc ttg 23
<210> 300
   <211> 23
   <212> RNA
   <213> Artificial Sequence
<220>
   <223> hPlekho1
<400> 300
   gtcagtaccg gaagagcctg atg 23
<210> 301
   <211> 19
   <212> RNA
   <213> Artificial Sequence
<220>
   <223> mCTGF
<400> 301
   acacgaactc attagacta 19
<210> 302
   <211> 19
   <212> RNA
   <213> Artificial Sequence
<220>
   <223> mCTGF
<400> 302
   gaagtaaggg acacgaact 19
<210> 303
   <211> 19
   <212> RNA
   <213> Artificial Sequence
<220>
   <223> mCTGF
<400> 303
   gagcatgtgt cctccacta 19
<210> 304
   <211> 19
   <212> RNA
   <213> Artificial Sequence
<220>
   <223> mCTGF
<400> 304
   gtacggagac atggcgtaa 19
<210> 305
   <211> 19
   <212> RNA
   <213> Artificial Sequence
<220>
   <223> mCTGF
<400> 305
   agtcagattt ctagtagga 19
<210> 306
   <211> 19
   <212> RNA
   <213> Artificial Sequence
<220>
   <223> mCTGF
<400> 306
   gacactggtt tcgagacag 19
<210> 307
   <211> 19
   <212> RNA
   <213> Artificial Sequence
<220>
   <223> mCTGF
<400> 307
   gtgaacaaat ggcctttat 19
<210> 308
   <211> 19
   <212> RNA
   <213> Artificial Sequence
<220>
   <223> mCTGF
<400> 308
   cacgaactca ttagactat 19
<210> 309
   <211> 19
   <212> RNA
   <213> Artificial Sequence
<220>
   <223> mCTGF
<400> 309
   tcaacctcag acactggtt 19
<210> 310
   <211> 19
   <212> RNA
   <213> Artificial Sequence
<220>
   <223> mCTGF
<400> 310
   cctgctgtgc atcctccta 19
<210> 311
   <211> 19
   <212> RNA
   <213> Artificial Sequence
<220>
   <223> mCTGF
<400> 311
   ggccatacaa gtagtctgt 19
<210> 312
   <211> 19
   <212> RNA
   <213> Artificial Sequence
<220>
   <223> mCTGF
<400> 312
   gcctactttt tggagtgta 19
<210> 313
   <211> 19
   <212> RNA
   <213> Artificial Sequence
<220>
   <223> mCTGF
<400> 313
   ggtgagtcct tccaaagca 19
<210> 314
   <211> 19
   <212> RNA
   <213> Artificial Sequence
<220>
   <223> mCTGF
<400> 314
   cagtttacac ttgacagtt 19
<210> 315
   <211> 19
   <212> RNA
   <213> Artificial Sequence
<220>
   <223> mCTGF
<400> 315
   aaactccaaa caccatagg 19
<210> 316
   <211> 19
   <212> RNA
   <213> Artificial Sequence
<220>
   <223> mCTGF
<400> 316
   cttgtctgtt agactggac 19
<210> 317
   <211> 19
   <212> RNA
   <213> Artificial Sequence
<220>
   <223> mCTGF
<400> 317
   acacttgaca gttgttcat 19
<210> 318
   <211> 19
   <212> RNA
   <213> Artificial Sequence
<220>
   <223> mCTGF
<400> 318
   agacactggt ttcgagaca 19
<210> 319
   <211> 19
   <212> RNA
   <213> Artificial Sequence
<220>
   <223> mCTGF
<400> 319
   agctgcaaat accaatgca 19
<210> 320
   <211> 19
   <212> RNA
   <213> Artificial Sequence
<220>
   <223> mCTGF
<400> 320
   tcagacactg gtttcgaga 19
<210> 321
   <211> 19
   <212> RNA
   <213> Artificial Sequence
<220>
   <223> mCTGF
<400> 321
   caccaaagtg agaacgtta 19
<210> 322
   <211> 19
   <212> RNA
   <213> Artificial Sequence
<220>
   <223> mCTGF
<400> 322
   gcatccggac acctaaaat 19
<210> 323
   <211> 19
   <212> RNA
   <213> Artificial Sequence
<220>
   <223> mCTGF
<400> 323
   gtactagctg aggttattt 19
<210> 324
   <211> 19
   <212> RNA
   <213> Artificial Sequence
<220>
   <223> mCTGF
<400> 324
   gacacgaact cattagact 19
<210> 325
   <211> 19
   <212> RNA
   <213> Artificial Sequence
<220>
   <223> mCTGF
<400> 325
   ctgcaaatac caatgcact 19
<210> 326
   <211> 19
   <212> RNA
   <213> Artificial Sequence
<220>
   <223> mCTGF
<400> 326
   ggacacctaa aatcgccaa 19
<210> 327
   <211> 19
   <212> RNA
   <213> Artificial Sequence
<220>
   <223> mCTGF
<400> 327
   gtgaagacat acagggcta 19
<210> 328
   <211> 19
   <212> RNA
   <213> Artificial Sequence
<220>
   <223> mCTGF
<400> 328
   ccaaacacca taggtagga 19
<210> 329
   <211> 19
   <212> RNA
   <213> Artificial Sequence
<220>
   <223> mCTGF
<400> 329
   ctgtaacaag ccagatttt 19
<210> 330
   <211> 19
   <212> RNA
   <213> Artificial Sequence
<220>
   <223> mCTGF
<400> 330
   ggtgaacaaa tggccttta 19
<210> 331
   <211> 19
   <212> RNA
   <213> Artificial Sequence
<220>
   <223> mCTGF
<400> 331
   ggtgagtcct tccaaagca 19
<210> 332
   <211> 19
   <212> RNA
   <213> Artificial Sequence
<220>
   <223> mCTGF
<400> 332
   gcatccggac acctaaaat 19
<210> 333
   <211> 19
   <212> RNA
   <213> Artificial Sequence
<220>
   <223> mCTGF
<400> 333
   ctgcaaatac caatgcact 19
<210> 334
   <211> 19
   <212> RNA
   <213> Artificial Sequence
<220>
   <223> mCTGF
<400> 334
   caaaaagtgc atccggaca 19
<210> 335
   <211> 19
   <212> RNA
   <213> Artificial Sequence
<220>
   <223> mCTGF
<400> 335
   ggacacctaa aatcgccaa 19
<210> 336
   <211> 19
   <212> RNA
   <213> Artificial Sequence
<220>
   <223> mCTGF
<400> 336
   gtgaagacat acagggcta 19
<210> 337
   <211> 19
   <212> RNA
   <213> Artificial Sequence
<220>
   <223> mCTGF
<400> 337
   ccttcccgag aagggtcaa 19
<210> 338
   <211> 19
   <212> RNA
   <213> Artificial Sequence
<220>
   <223> mCTGF
<400> 338
   cgcctgttct aagacctgt 19
<210> 339
   <211> 19
   <212> RNA
   <213> Artificial Sequence
<220>
   <223> mCTGF
<400> 339
   ctgtgcctgc cattacaac 19
<210> 340
   <211> 19
   <212> RNA
   <213> Artificial Sequence
<220>
   <223> mCTGF
<400> 340
   agcggtgagt ccttccaaa 19
<210> 341
   <211> 19
   <212> RNA
   <213> Artificial Sequence
<220>
   <223> mCTGF
<400> 341
   gcaccagtgt gaagacata 19
<210> 342
   <211> 20
   <212> RNA
   <213> Artificial Sequence
<220>
   <223> mCTGF
<400> 342
   gacaatacct tctgcagact 20
<210> 343
   <211> 19
   <212> RNA
   <213> Artificial Sequence
<220>
   <223> mCTGF
<400> 343
   cgcaagatcg gagtgtgca 19
<210> 344
   <211> 19
   <212> RNA
   <213> Artificial Sequence
<220>
   <223> mCTGF
<400> 344
   gcaaaaagtg catccggac 19
<210> 345
   <211> 19
   <212> RNA
   <213> Artificial Sequence
<220>
   <223> mCTGF
<400> 345
   tcttcggtgg gtcggtgta 19
<210> 346
   <211> 19
   <212> RNA
   <213> Artificial Sequence
<220>
   <223> mCTGF
<400> 346
   tcccgagaag ggtcaagct 19
<210> 347
   <211> 19
   <212> RNA
   <213> Artificial Sequence
<220>
   <223> mCTGF
<400> 347
   gtgcatccgg acacctaaa 19
<210> 348
   <211> 19
   <212> RNA
   <213> Artificial Sequence
<220>
   <223> mCTGF
<400> 348
   cgatggcgag atcatgaaa 19
<210> 349
   <211> 19
   <212> RNA
   <213> Artificial Sequence
<220>
   <223> mCTGF
<400> 349
   gatggcgaga tcatgaaaa 19
<210> 350
   <211> 19
   <212> RNA
   <213> Artificial Sequence
<220>
   <223> mCTGF
<400> 350
   ctgtcaagtt tgagctttc 19
<210> 351
   <211> 19
   <212> RNA
   <213> Artificial Sequence
<220>
   <223> mCTGF
<400> 351
   ctcttctgcg atttcggct 19
<210> 352
   <211> 19
   <212> RNA
   <213> Artificial Sequence
<220>
   <223> mCTGF
<400> 352
   gttaccaatg acaatacct 19
<210> 353
   <211> 19
   <212> RNA
   <213> Artificial Sequence
<220>
   <223> mCTGF
<400> 353
   cctgtcaagt ttgagcttt 19
<210> 354
   <211> 19
   <212> RNA
   <213> Artificial Sequence
<220>
   <223> mCTGF
<400> 354
   ggaagatgta cggagacat 19
<210> 355
   <211> 19
   <212> RNA
   <213> Artificial Sequence
<220>
   <223> mCTGF
<400> 355
   catccggaca cctaaaatc 19
<210> 356
   <211> 19
   <212> RNA
   <213> Artificial Sequence
<220>
   <223> mCTGF
<400> 356
   tgtcaagttt gagctttct 19
<210> 357
   <211> 19
   <212> RNA
   <213> Artificial Sequence
<220>
   <223> mCTGF
<400> 357
   caaggaccgc acagcagtt 19
<210> 358
   <211> 19
   <212> RNA
   <213> Artificial Sequence
<220>
   <223> mCTGF
<400> 358
   ggaagacaca tttggccca 19
<210> 359
   <211> 19
   <212> RNA
   <213> Artificial Sequence
<220>
   <223> mCTGF
<400> 359
   ggcaaaaagt gcatccgga 19
<210> 360
   <211> 19
   <212> RNA
   <213> Artificial Sequence
<220>
   <223> mCTGF
<400> 360
   gactggaaga cacatttgg 19
<210> 361
   <211> 19
   <212> RNA
   <213> Artificial Sequence
<220>
   <223> mCTGF
<400> 361
   tgaagacata cagggctaa 19
<210> 362
   <211> 19
   <212> RNA
   <213> Artificial Sequence
<220>
   <223> mCTGF
<400> 362
   gggaaatgct gcgaggagt 19
<210> 363
   <211> 19
   <212> RNA
   <213> Artificial Sequence
<220>
   <223> mCTGF
<400> 363
   acaggaagat gtacggaga 19
<210> 364
   <211> 19
   <212> RNA
   <213> Artificial Sequence
<220>
   <223> mCTGF
<400> 364
   ttggcccaga cccaactat 19
<210> 365
   <211> 19
   <212> RNA
   <213> Artificial Sequence
<220>
   <223> mCTGF
<400> 365
   cctaaaatcg ccaagcctg 19
<210> 366
   <211> 19
   <212> RNA
   <213> Artificial Sequence
<220>
   <223> mCTGF
<400> 366
   gaagacatac agggctaag 19
<210> 367
   <211> 19
   <212> RNA
   <213> Artificial Sequence
<220>
   <223> mCTGF
<400> 367
   actatgatgc gagccaact 19
<210> 368
   <211> 19
   <212> RNA
   <213> Artificial Sequence
<220>
   <223> mCTGF
<400> 368
   agaagggcaa aaagtgcat 19
<210> 369
   <211> 19
   <212> RNA
   <213> Artificial Sequence
<220>
   <223> mCTGF
<400> 369
   atcgccaagc ctgtcaagt 19
<210> 370
   <211> 19
   <212> RNA
   <213> Artificial Sequence
<220>
   <223> mCTGF
<400> 370
   aggaagatgt acggagaca 19
<210> 371
   <211> 19
   <212> RNA
   <213> Artificial Sequence
<220>
   <223> mCTGF
<400> 371
   ccgactggaa gacacattt 19
<210> 372
   <211> 19
   <212> RNA
   <213> Artificial Sequence
<220>
   <223> mCTGF
<400> 372
   gcccagaccc aactatgat 19
<210> 373
   <211> 19
   <212> RNA
   <213> Artificial Sequence
<220>
   <223> mCTGF
<400> 373
   cgccaagcct gtcaagttt 19
<210> 374
   <211> 19
   <212> RNA
   <213> Artificial Sequence
<220>
   <223> mCTGF
<400> 374
   ccgatggcga gatcatgaa 19
<210> 375
   <211> 19
   <212> RNA
   <213> Artificial Sequence
<220>
   <223> mCTGF
<400> 375
   tccggacacc taaaatcgc 19
<210> 376
   <211> 18
   <212> RNA
   <213> Artificial Sequence
<220>
   <223> mCTGF
<400> 376
   tttgagcttt ctggctgc 18
<210> 377
   <211> 19
   <212> RNA
   <213> Artificial Sequence
<220>
   <223> mCTGF
<400> 377
   ggagaactgt gtacggagc 19
<210> 378
   <211> 19
   <212> RNA
   <213> Artificial Sequence
<220>
   <223> mCTGF
<400> 378
   ctgcaccagt gtgaagaca 19
<210> 379
   <211> 19
   <212> RNA
   <213> Artificial Sequence
<220>
   <223> mCTGF
<400> 379
   agcgcctgtt ctaagacct 19
<210> 380
   <211> 19
   <212> RNA
   <213> Artificial Sequence
<220>
   <223> mCTGF
<400> 380
   cgactggaag acacatttg 19
<210> 381
   <211> 19
   <212> RNA
   <213> Artificial Sequence
<220>
   <223> mCTGF
<400> 381
   gagaagggtc aagctgcct 19
<210> 382
   <211> 19
   <212> RNA
   <213> Artificial Sequence
<220>
   <223> mCTGF
<400> 382
   ctacaggaag atgtacgga 19
<210> 383
   <211> 19
   <212> RNA
   <213> Artificial Sequence
<220>
   <223> mCTGF
<400> 383
   gacctggagg aaaacatta 19
<210> 384
   <211> 19
   <212> RNA
   <213> Artificial Sequence
<220>
   <223> mCTGF
<400> 384
   tggagcgcct gttctaaga 19
<210> 385
   <211> 19
   <212> RNA
   <213> Artificial Sequence
<220>
   <223> mCTGF
<400> 385
   tgccattaca actgtcctg 19
<210> 386
   <211> 19
   <212> RNA
   <213> Artificial Sequence
<220>
   <223> mCTGF
<400> 386
   cggagtgtgc actgccaaa 19
<210> 387
   <211> 19
   <212> RNA
   <213> Artificial Sequence
<220>
   <223> mCTGF
<400> 387
   tcggagtgtg cactgccaa 19
<210> 388
   <211> 19
   <212> RNA
   <213> Artificial Sequence
<220>
   <223> mCTGF
<400> 388
   cggacaccta aaatcgcca 19
<210> 389
   <211> 19
   <212> RNA
   <213> Artificial Sequence
<220>
   <223> mCTGF
<400> 389
   gcctgttcta agacctgtg 19
<210> 390
   <211> 19
   <212> RNA
   <213> Artificial Sequence
<220>
   <223> mCTGF
<400> 390
   caggaagatg tacggagac 19
<210> 391
   <211> 19
   <212> RNA
   <213> Artificial Sequence
<220>
   <223> mCTGF
<400> 391
   tgcaccagtg tgaagacat 19
<210> 392
   <211> 19
   <212> RNA
   <213> Artificial Sequence
<220>
   <223> mCTGF
<400> 392
   tgcatccgga cacctaaaa 19
<210> 393
   <211> 19
   <212> RNA
   <213> Artificial Sequence
<220>
   <223> mCTGF
<400> 393
   atggcgagat catgaaaaa 19
<210> 394
   <211> 19
   <212> RNA
   <213> Artificial Sequence
<220>
   <223> mCTGF
<400> 394
   ccaaccgcaa gatcggagt 19
<210> 395
   <211> 19
   <212> RNA
   <213> Artificial Sequence
<220>
   <223> mCTGF
<400> 395
   aacattaaga agggcaaaa 19
<210> 396
   <211> 19
   <212> RNA
   <213> Artificial Sequence
<220>
   <223> mCTGF
<400> 396
   gccattacaa ctgtcctgg 19
<210> 397
   <211> 19
   <212> RNA
   <213> Artificial Sequence
<220>
   <223> mCTGF
<400> 397
   tcgccaagcc tgtcaagtt 19
<210> 398
   <211> 19
   <212> RNA
   <213> Artificial Sequence
<220>
   <223> mCTGF
<400> 398
   agctgggaga actgtgtac 19
<210> 399
   <211> 19
   <212> RNA
   <213> Artificial Sequence
<220>
   <223> mCTGF
<400> 399
   agctgcctac cgactggaa 19
<210> 400
   <211> 19
   <212> RNA
   <213> Artificial Sequence
<220>
   <223> mCTGF
<400> 400
   acagagtgga gcgcctgtt 19
<210> 401
   <211> 19
   <212> RNA
   <213> Artificial Sequence
<220>
   <223> mCyr61
<400> 401
   ctgtgaagtg cgtccttgt 19
<210> 402
   <211> 19
   <212> RNA
   <213> Artificial Sequence
<220>
   <223> mCyr61
<400> 402
   gtgaagatgc ggttccgat 19
<210> 403
   <211> 19
   <212> RNA
   <213> Artificial Sequence
<220>
   <223> mCyr61
<400> 403
   ctgcaaatgt aactacaac 19
<210> 404
   <211> 19
   <212> RNA
   <213> Artificial Sequence
<220>
   <223> mCyr61
<400> 404
   ggatgaatgg tgccttgct 19
<210> 405
   <211> 19
   <212> RNA
   <213> Artificial Sequence
<220>
   <223> mCyr61
<400> 405
   gcagaccctg tgaatataa 19
<210> 406
   <211> 19
   <212> RNA
   <213> Artificial Sequence
<220>
   <223> mCyr61
<400> 406
   gaagtaaatg aaagcgcct 19
<210> 407
   <211> 19
   <212> RNA
   <213> Artificial Sequence
<220>
   <223> mCyr61
<400> 407
   ggacaaccag tgtacagca 19
<210> 408
   <211> 19
   <212> RNA
   <213> Artificial Sequence
<220>
   <223> mCyr61
<400> 408
   gcattaagga ctccctgga 19
<210> 409
   <211> 19
   <212> RNA
   <213> Artificial Sequence
<220>
   <223> mCyr61
<400> 409
   tgtacagcct attcaatga 19
<210> 410
   <211> 19
   <212> RNA
   <213> Artificial Sequence
<220>
   <223> mCyr61
<400> 410
   cgactgtaca gcctattca 19
<210> 411
   <211> 19
   <212> RNA
   <213> Artificial Sequence
<220>
   <223> mCyr61
<400> 411
   tggagcgggc attattgct 19
<210> 412
   <211> 19
   <212> RNA
   <213> Artificial Sequence
<220>
   <223> mCyr61
<400> 412
   gacttcgctt catagtact 19
<210> 413
   <211> 19
   <212> RNA
   <213> Artificial Sequence
<220>
   <223> mCyr61
<400> 413
   gtactggagc gggcattat 19
<210> 414
   <211> 19
   <212> RNA
   <213> Artificial Sequence
<220>
   <223> mCyr61
<400> 414
   ggcattattg ctccatatt 19
<210> 415
   <211> 19
   <212> RNA
   <213> Artificial Sequence
<220>
   <223> mCyr61
<400> 415
   gagttctttt caaccctct 19
<210> 416
   <211> 19
   <212> RNA
   <213> Artificial Sequence
<220>
   <223> mCyr61
<400> 416
   ctgtgaatat aactccaga 19
<210> 417
   <211> 19
   <212> RNA
   <213> Artificial Sequence
<220>
   <223> mCyr61
<400> 417
   cgaagatgga gagatgttt 19
<210> 418
   <211> 19
   <212> RNA
   <213> Artificial Sequence
<220>
   <223> mCyr61
<400> 418
   ccaagaatgt catgatgat 19
<210> 419
   <211> 19
   <212> RNA
   <213> Artificial Sequence
<220>
   <223> mCyr61
<400> 419
   ggagttaacg agaaacaat 19
<210> 420
   <211> 19
   <212> RNA
   <213> Artificial Sequence
<220>
   <223> mCyr61
<400> 420
   cctgcactct aaaactgca 19
<210> 421
   <211> 19
   <212> RNA
   <213> Artificial Sequence
<220>
   <223> mCyr61
<400> 421
   ggcaagaaat gcagcaaga 19
<210> 422
   <211> 19
   <212> RNA
   <213> Artificial Sequence
<220>
   <223> mCyr61
<400> 422
   gccagaaatg catcgttca 19
<210> 423
   <211> 19
   <212> RNA
   <213> Artificial Sequence
<220>
   <223> mCyr61
<400> 423
   gtctgcgcta aacaactca 19
<210> 424
   <211> 19
   <212> RNA
   <213> Artificial Sequence
<220>
   <223> mCyr61
<400> 424
   gcaagcatca tggagacgt 19
<210> 425
   <211> 19
   <212> RNA
   <213> Artificial Sequence
<220>
   <223> mCyr61
<400> 425
   gcgagttctt ttcaaccct 19
<210> 426
   <211> 19
   <212> RNA
   <213> Artificial Sequence
<220>
   <223> mCyr61
<400> 426
   agtactggag cgggcatta 19
<210> 427
   <211> 19
   <212> RNA
   <213> Artificial Sequence
<220>
   <223> mCyr61
<400> 427
   gaatggtgcc ttgctcatt 19
<210> 428
   <211> 19
   <212> RNA
   <213> Artificial Sequence
<220>
   <223> mCyr61
<400> 428
   agaaataccg gcccaaata 19
<210> 429
   <211> 19
   <212> RNA
   <213> Artificial Sequence
<220>
   <223> mCyr61
<400> 429
   cagccgcagt tggagaaga 19
<210> 430
   <211> 19
   <212> RNA
   <213> Artificial Sequence
<220>
   <223> mCyr61
<400> 430
   gagagatgtt ttccaagaa 19
<210> 431
   <211> 19
   <212> RNA
   <213> Artificial Sequence
<220>
   <223> mCyr61
<400> 431
   ctgtgaagtg cgtccttgt 19
<210> 432
   <211> 19
   <212> RNA
   <213> Artificial Sequence
<220>
   <223> mCyr61
<400> 432
   gtgaagatgc ggttccgat 19
<210> 433
   <211> 19
   <212> RNA
   <213> Artificial Sequence
<220>
   <223> mCyr61
<400> 433
   ctgcaaatgt aactacaac 19
<210> 434
   <211> 19
   <212> RNA
   <213> Artificial Sequence
<220>
   <223> mCyr61
<400> 434
   gcagaccctg tgaatataa 19
<210> 435
   <211> 19
   <212> RNA
   <213> Artificial Sequence
<220>
   <223> mCyr61
<400> 435
   gtgtacagca gcctaaaaa 19
<210> 436
   <211> 19
   <212> RNA
   <213> Artificial Sequence
<220>
   <223> mCyr61
<400> 436
   ctgaagaggc ttcctgtct 19
<210> 437
   <211> 19
   <212> RNA
   <213> Artificial Sequence
<220>
   <223> mCyr61
<400> 437
   gtaactacaa ctgcccgca 19
<210> 438
   <211> 19
   <212> RNA
   <213> Artificial Sequence
<220>
   <223> mCyr61
<400> 438
   caagaaatac cggcccaaa 19
<210> 439
   <211> 19
   <212> RNA
   <213> Artificial Sequence
<220>
   <223> mCyr61
<400> 439
   gcattaagga ctccctgga 19
<210> 440
   <211> 19
   <212> RNA
   <213> Artificial Sequence
<220>
   <223> mCyr61
<400> 440
   ggacaaccag tgtacagca 19
<210> 441
   <211> 19
   <212> RNA
   <213> Artificial Sequence
<220>
   <223> mCyr61
<400> 441
   agaaccagtc agatttact 19
<210> 442
   <211> 19
   <212> RNA
   <213> Artificial Sequence
<220>
   <223> mCyr61
<400> 442
   tgtacagcct attcaatga 19
<210> 443
   <211> 19
   <212> RNA
   <213> Artificial Sequence
<220>
   <223> mCyr61
<400> 443
   ccagtgcaca tgtattgat 19
<210> 444
   <211> 19
   <212> RNA
   <213> Artificial Sequence
<220>
   <223> mCyr61
<400> 444
   cgactgtaca gcctattca 19
<210> 445
   <211> 19
   <212> RNA
   <213> Artificial Sequence
<220>
   <223> mCyr61
<400> 445
   ctgtgaatat aactccaga 19
<210> 446
   <211> 19
   <212> RNA
   <213> Artificial Sequence
<220>
   <223> mCyr61
<400> 446
   ggagttaacg agaaacaat 19
<210> 447
   <211> 19
   <212> RNA
   <213> Artificial Sequence
<220>
   <223> mCyr61
<400> 447
   ccaagaatgt catgatgat 19
<210> 448
   <211> 19
   <212> RNA
   <213> Artificial Sequence
<220>
   <223> mCyr61
<400> 448
   gaagaggctt cctgtcttt 19
<210> 449
   <211> 19
   <212> RNA
   <213> Artificial Sequence
<220>
   <223> mCyr61
<400> 449
   cccatggcca gaaatgcat 19
<210> 450
   <211> 19
   <212> RNA
   <213> Artificial Sequence
<220>
   <223> mCyr61
<400> 450
   gccagaaatg catcgttca 19
<210> 451
   <211> 19
   <212> RNA
   <213> Artificial Sequence
<220>
   <223> mCyr61
<400> 451
   ggcaagaaat gcagcaaga 19
<210> 452
   <211> 19
   <212> RNA
   <213> Artificial Sequence
<220>
   <223> mCyr61
<400> 452
   aggcagaccc tgtgaatat 19
<210> 453
   <211> 19
   <212> RNA
   <213> Artificial Sequence
<220>
   <223> mCyr61
<400> 453
   gtctgcgcta aacaactca 19
<210> 454
   <211> 19
   <212> RNA
   <213> Artificial Sequence
<220>
   <223> mCyr61
<400> 454
   gcgagttctt ttcaaccct 19
<210> 455
   <211> 19
   <212> RNA
   <213> Artificial Sequence
<220>
   <223> mCyr61
<400> 455
   gctctgaaag ggatctgca 19
<210> 456
   <211> 19
   <212> RNA
   <213> Artificial Sequence
<220>
   <223> mCyr61
<400> 456
   tgaagagtgg gtttgtgat 19
<210> 457
   <211> 19
   <212> RNA
   <213> Artificial Sequence
<220>
   <223> mCyr61
<400> 457
   agaaataccg gcccaaata 19
<210> 458
   <211> 19
   <212> RNA
   <213> Artificial Sequence
<220>
   <223> mCyr61
<400> 458
   gagagatgtt ttccaagaa 19
<210> 459
   <211> 19
   <212> RNA
   <213> Artificial Sequence
<220>
   <223> mCyr61
<400> 459
   gactgtacag cctattcaa 19
<210> 460
   <211> 19
   <212> RNA
   <213> Artificial Sequence
<220>
   <223> mCyr61
<400> 460
   agttaccaat gacaaccca 19
<210> 461
   <211> 19
   <212> RNA
   <213> Artificial Sequence
<220>
   <223> mCyr61
<400> 461
   ccggatctgt gaagtgcgt 19
<210> 462
   <211> 19
   <212> RNA
   <213> Artificial Sequence
<220>
   <223> mCyr61
<400> 462
   atgcagcaag accaagaaa 19
<210> 463
   <211> 19
   <212> RNA
   <213> Artificial Sequence
<220>
   <223> mCyr61
<400> 463
   ctaaacaact caacgagga 19
<210> 464
   <211> 19
   <212> RNA
   <213> Artificial Sequence
<220>
   <223> mCyr61
<400> 464
   gaaataccgg cccaaatac 19
<210> 465
   <211> 19
   <212> RNA
   <213> Artificial Sequence
<220>
   <223> mCyr61
<400> 465
   ctgtaaggtc tgcgctaaa 19
<210> 466
   <211> 19
   <212> RNA
   <213> Artificial Sequence
<220>
   <223> mCyr61
<400> 466
   gaaccgcgag ttcttttca 19
<210> 467
   <211> 19
   <212> RNA
   <213> Artificial Sequence
<220>
   <223> mCyr61
<400> 467
   gatgctccag tgtcaagaa 19
<210> 468
   <211> 19
   <212> RNA
   <213> Artificial Sequence
<220>
   <223> mCyr61
<400> 468
   gcgaagatgg agagatgtt 19
<210> 469
   <211> 19
   <212> RNA
   <213> Artificial Sequence
<220>
   <223> mCyr61
<400> 469
   gatccagtcc tgcaaatgt 19
<210> 470
   <211> 19
   <212> RNA
   <213> Artificial Sequence
<220>
   <223> mCyr61
<400> 470
   cctgcaaatg taactacaa 19
<210> 471
   <211> 19
   <212> RNA
   <213> Artificial Sequence
<220>
   <223> mCyr61
<400> 471
   ttccgactgt acagcctat 19
<210> 472
   <211> 19
   <212> RNA
   <213> Artificial Sequence
<220>
   <223> mCyr61
<400> 472
   aagtgcgtcc ttgtggaca 19
<210> 473
   <211> 19
   <212> RNA
   <213> Artificial Sequence
<220>
   <223> mCyr61
<400> 473
   acaactgccc gcatcccaa 19
<210> 474
   <211> 19
   <212> RNA
   <213> Artificial Sequence
<220>
   <223> mCyr61
<400> 474
   gctgtaaggt ctgcgctaa 19
<210> 475
   <211> 19
   <212> RNA
   <213> Artificial Sequence
<220>
   <223> mCyr61
<400> 475
   gagttaatcg caattggaa 19
<210> 476
   <211> 19
   <212> RNA
   <213> Artificial Sequence
<220>
   <223> mCyr61
<400> 476
   ccttgtggac aaccagtgt 19
<210> 477
   <211> 19
   <212> RNA
   <213> Artificial Sequence
<220>
   <223> mCyr61
<400> 477
   gttccgatgc gaagatgga 19
<210> 478
   <211> 19
   <212> RNA
   <213> Artificial Sequence
<220>
   <223> mCyr61
<400> 478
   gatggagaga tgttttcca 19
<210> 479
   <211> 19
   <212> RNA
   <213> Artificial Sequence
<220>
   <223> mCyr61
<400> 479
   ggagagatgt tttccaaga 19
<210> 480
   <211> 19
   <212> RNA
   <213> Artificial Sequence
<220>
   <223> mCyr61
<400> 480
   gcctattcaa tgacatcca 19
<210> 481
   <211> 19
   <212> RNA
   <213> Artificial Sequence
<220>
   <223> mCyr61
<400> 481
   ccatggccag aaatgcatc 19
<210> 482
   <211> 19
   <212> RNA
   <213> Artificial Sequence
<220>
   <223> mCyr61
<400> 482
   tcaagaaata ccggcccaa 19
<210> 483
   <211> 19
   <212> RNA
   <213> Artificial Sequence
<220>
   <223> mCyr61
<400> 483
   cccaactgta aacaccagt 19
<210> 484
   <211> 19
   <212> RNA
   <213> Artificial Sequence
<220>
   <223> mCyr61
<400> 484
   ctgtaaacac cagtgcaca 19
<210> 485
   <211> 19
   <212> RNA
   <213> Artificial Sequence
<220>
   <223> mCyr61
<400> 485
   ggaggtggag ttaacgaga 19
<210> 486
   <211> 19
   <212> RNA
   <213> Artificial Sequence
<220>
   <223> mCyr61
<400> 486
   tccagcccaa ctgtaaaca 19
<210> 487
   <211> 19
   <212> RNA
   <213> Artificial Sequence
<220>
   <223> mCyr61
<400> 487
   tgcagcaaga ccaagaaat 19
<210> 488
   <211> 19
   <212> RNA
   <213> Artificial Sequence
<220>
   <223> mCyr61
<400> 488
   aagaaatacc ggcccaaat 19
<210> 489
   <211> 19
   <212> RNA
   <213> Artificial Sequence
<220>
   <223> mCyr61
<400> 489
   catgtattga tggcgccgt 19
<210> 490
   <211> 19
   <212> RNA
   <213> Artificial Sequence
<220>
   <223> mCyr61
<400> 490
   tgaagaggct tcctgtctt 19
<210> 491
   <211> 19
   <212> RNA
   <213> Artificial Sequence
<220>
   <223> mCyr61
<400> 491
   tcctgcaaat gtaactaca 19
<210> 492
   <211> 19
   <212> RNA
   <213> Artificial Sequence
<220>
   <223> mCyr61
<400> 492
   ccaccgctct gaaagggat 19
<210> 493
   <211> 19
   <212> RNA
   <213> Artificial Sequence
<220>
   <223> mCyr61
<400> 493
   cccaaatact gcggctcct 19
<210> 494
   <211> 19
   <212> RNA
   <213> Artificial Sequence
<220>
   <223> mCyr61
<400> 494
   ctctgcagac cagaactgt 19
<210> 495
   <211> 19
   <212> RNA
   <213> Artificial Sequence
<220>
   <223> mCyr61
<400> 495
   ggttggaatg caatttcgg 19
<210> 496
   <211> 19
   <212> RNA
   <213> Artificial Sequence
<220>
   <223> mCyr61
<400> 496
   aacaccagtg cacatgtat 19
<210> 497
   <211> 19
   <212> RNA
   <213> Artificial Sequence
<220>
   <223> mCyr61
<400> 497
   agagtgggtt tgtgatgaa 19
<210> 498
   <211> 19
   <212> RNA
   <213> Artificial Sequence
<220>
   <223> mCyr61
<400> 498
   agcccaactg taaacacca 19
<210> 499
   <211> 19
   <212> RNA
   <213> Artificial Sequence
<220>
   <223> mCyr61
<400> 499
   cgcaattgga aaaggcagc 19
<210> 500
   <211> 19
   <212> RNA
   <213> Artificial Sequence
<220>
   <223> mCyr61
<400> 500
   gaatgcaatt tcggcgcca 19
<210> 501
   <211> 19
   <212> RNA
   <213> Artificial Sequence
<220>
   <223> mPlekho1
<400> 501
   gagaaggagt catggatca 19
<210> 502
   <211> 19
   <212> RNA
   <213> Artificial Sequence
<220>
   <223> mPlekho1
<400> 502
   gatttggaaa aaccgctat 19
<210> 503
   <211> 19
   <212> RNA
   <213> Artificial Sequence
<220>
   <223> mPlekho1
<400> 503
   ggatgctaac attagacct 19
<210> 504
   <211> 19
   <212> RNA
   <213> Artificial Sequence
<220>
   <223> mPlekho1
<400> 504
   cacaagcact gaagaagtt 19
<210> 505
   <211> 19
   <212> RNA
   <213> Artificial Sequence
<220>
   <223> mPlekho1
<400> 505
   tgtttgacct gagtgacta 19
<210> 506
   <211> 19
   <212> RNA
   <213> Artificial Sequence
<220>
   <223> mPlekho1
<400> 506
   cgccctgagt tctgccatt 19
<210> 507
   <211> 19
   <212> RNA
   <213> Artificial Sequence
<220>
   <223> mPlekho1
<400> 507
   cattagacct gatccaaga 19
<210> 508
   <211> 19
   <212> RNA
   <213> Artificial Sequence
<220>
   <223> mPlekho1
<400> 508
   ggacctggta gcaaggaaa 19
<210> 509
   <211> 19
   <212> RNA
   <213> Artificial Sequence
<220>
   <223> mPlekho1
<400> 509
   ggaaactgga gaagactca 19
<210> 510
   <211> 19
   <212> RNA
   <213> Artificial Sequence
<220>
   <223> mPlekho1
<400> 510
   tggagaagac tcaggagct 19
<210> 511
   <211> 19
   <212> RNA
   <213> Artificial Sequence
<220>
   <223> mPlekho1
<400> 511
   ctctacctca gatgggatg 19
<210> 512
   <211> 19
   <212> RNA
   <213> Artificial Sequence
<220>
   <223> mPlekho1
<400> 512
   cctatcccag agggacact 19
<210> 513
   <211> 19
   <212> RNA
   <213> Artificial Sequence
<220>
   <223> mPlekho1
<400> 513
   ggaaaaaccg ctatgtggt 19
<210> 514
   <211> 19
   <212> RNA
   <213> Artificial Sequence
<220>
   <223> mPlekho1
<400> 514
   gtttgacctg agtgactat 19
<210> 515
   <211> 19
   <212> RNA
   <213> Artificial Sequence
<220>
   <223> mPlekho1
<400> 515
   ctcagatggg atgctaaca 19
<210> 516
   <211> 19
   <212> RNA
   <213> Artificial Sequence
<220>
   <223> mPlekho1
<400> 516
   gagctgagag acctgtaca 19
<210> 517
   <211> 19
   <212> RNA
   <213> Artificial Sequence
<220>
   <223> mPlekho1
<400> 517
   ctcgagacag agcaaaaat 19
<210> 518
   <211> 19
   <212> RNA
   <213> Artificial Sequence
<220>
   <223> mPlekho1
<400> 518
   agtcatggat caacgccct 19
<210> 519
   <211> 19
   <212> RNA
   <213> Artificial Sequence
<220>
   <223> mPlekho1
<400> 519
   aaagagagtg ctgcaggaa 19
<210> 520
   <211> 19
   <212> RNA
   <213> Artificial Sequence
<220>
   <223> mPlekho1
<400> 520
   actatgagaa gtgcgaaga 19
<210> 521
   <211> 19
   <212> RNA
   <213> Artificial Sequence
<220>
   <223> mPlekho1
<400> 521
   gactcccacc tcagacaga 19
<210> 522
   <211> 19
   <212> RNA
   <213> Artificial Sequence
<220>
   <223> mPlekho1
<400> 522
   cgtgagtcct gaagagaag 19
<210> 523
   <211> 19
   <212> RNA
   <213> Artificial Sequence
<220>
   <223> mPlekho1
<400> 523
   tgaaaggcga ccagctcta 19
<210> 524
   <211> 19
   <212> RNA
   <213> Artificial Sequence
<220>
   <223> mPlekho1
<400> 524
   cctcttgtgc tgagagctt 19
<210> 525
   <211> 19
   <212> RNA
   <213> Artificial Sequence
<220>
   <223> mPlekho1
<400> 525
   ccacaagcac tgaagaagt 19
<210> 526
   <211> 19
   <212> RNA
   <213> Artificial Sequence
<220>
   <223> mPlekho1
<400> 526
   cacagtcaat accggaaga 19
<210> 527
   <211> 19
   <212> RNA
   <213> Artificial Sequence
<220>
   <223> mPlekho1
<400> 527
   cctactcgag acagagcaa 19
<210> 528
   <211> 19
   <212> RNA
   <213> Artificial Sequence
<220>
   <223> mPlekho1
<400> 528
   ccgtgagtcc tgaagagaa 19
<210> 529
   <211> 19
   <212> RNA
   <213> Artificial Sequence
<220>
   <223> mPlekho1
<400> 529
   ctaaaaaccg tatcttgga 19
<210> 530
   <211> 19
   <212> RNA
   <213> Artificial Sequence
<220>
   <223> mPlekho1
<400> 530
   gtgtttgacc tgagtgact 19
<210> 531
   <211> 19
   <212> RNA
   <213> Artificial Sequence
<220>
   <223> mPlekho1
<400> 531
   gagaaggagt catggatca 19
<210> 532
   <211> 19
   <212> RNA
   <213> Artificial Sequence
<220>
   <223> mPlekho1
<400> 532
   ccagagctaa aaaccgtat 19
<210> 533
   <211> 19
   <212> RNA
   <213> Artificial Sequence
<220>
   <223> mPlekho1
<400> 533
   ctatcttgcc caccctact 19
<210> 534
   <211> 19
   <212> RNA
   <213> Artificial Sequence
<220>
   <223> mPlekho1
<400> 534
   gatttggaaa aaccgctat 19
<210> 535
   <211> 19
   <212> RNA
   <213> Artificial Sequence
<220>
   <223> mPlekho1
<400> 535
   ctgagtgact atgagaagt 19
<210> 536
   <211> 19
   <212> RNA
   <213> Artificial Sequence
<220>
   <223> mPlekho1
<400> 536
   ggatgctaac attagacct 19
<210> 537
   <211> 19
   <212> RNA
   <213> Artificial Sequence
<220>
   <223> mPlekho1
<400> 537
   ctccggatgg aaaccatca 19
<210> 538
   <211> 19
   <212> RNA
   <213> Artificial Sequence
<220>
   <223> mPlekho1
<400> 538
   caccttcaag ctgaggaat 19
<210> 539
   <211> 19
   <212> RNA
   <213> Artificial Sequence
<220>
   <223> mPlekho1
<400> 539
   ccgctatgtg gtgctgaaa 19
<210> 540
   <211> 19
   <212> RNA
   <213> Artificial Sequence
<220>
   <223> mPlekho1
<400> 540
   ggaggtaaaa gatgagaaa 19
<210> 541
   <211> 19
   <212> RNA
   <213> Artificial Sequence
<220>
   <223> mPlekho1
<400> 541
   tgtttgacct gagtgacta 19
<210> 542
   <211> 19
   <212> RNA
   <213> Artificial Sequence
<220>
   <223> mPlekho1
<400> 542
   cgccctgagt tctgccatt 19
<210> 543
   <211> 19
   <212> RNA
   <213> Artificial Sequence
<220>
   <223> mPlekho1
<400> 543
   cattagacct gatccaaga 19
<210> 544
   <211> 19
   <212> RNA
   <213> Artificial Sequence
<220>
   <223> mPlekho1
<400> 544
   ggacctggta gcaaggaaa 19
<210> 545
   <211> 19
   <212> RNA
   <213> Artificial Sequence
<220>
   <223> mPlekho1
<400> 545
   ggaaactgga gaagactca 19
<210> 546
   <211> 19
   <212> RNA
   <213> Artificial Sequence
<220>
   <223> mPlekho1
<400> 546
   tggagaagac tcaggagct 19
<210> 547
   <211> 19
   <212> RNA
   <213> Artificial Sequence
<220>
   <223> mPlekho1
<400> 547
   ctctacctca gatgggatg 19
<210> 548
   <211> 19
   <212> RNA
   <213> Artificial Sequence
<220>
   <223> mPlekho1
<400> 548
   cgtatcttgg atgaggtca 19
<210> 549
   <211> 19
   <212> RNA
   <213> Artificial Sequence
<220>
   <223> mPlekho1
<400> 549
   ggaaaaaccg ctatgtggt 19
<210> 550
   <211> 19
   <212> RNA
   <213> Artificial Sequence
<220>
   <223> mPlekho1
<400> 550
   cctatcccag agggacact 19
<210> 551
   <211> 19
   <212> RNA
   <213> Artificial Sequence
<220>
   <223> mPlekho1
<400> 551
   gtcaccgttg aggaggaca 19
<210> 552
   <211> 19
   <212> RNA
   <213> Artificial Sequence
<220>
   <223> mPlekho1
<400> 552
   ctttctcggc cttgggaaa 19
<210> 553
   <211> 19
   <212> RNA
   <213> Artificial Sequence
<220>
   <223> mPlekho1
<400> 553
   gagctgagag acctgtaca 19
<210> 554
   <211> 19
   <212> RNA
   <213> Artificial Sequence
<220>
   <223> mPlekho1
<400> 554
   agaaggaggt aaaagatga 19
<210> 555
   <211> 19
   <212> RNA
   <213> Artificial Sequence
<220>
   <223> mPlekho1
<400> 555
   agtcatggat caacgccct 19
<210> 556
   <211> 19
   <212> RNA
   <213> Artificial Sequence
<220>
   <223> mPlekho1
<400> 556
   actatgagaa gtgcgaaga 19
<210> 557
   <211> 19
   <212> RNA
   <213> Artificial Sequence
<220>
   <223> mPlekho1
<400> 557
   aaagagagtg ctgcaggaa 19
<210> 558
   <211> 19
   <212> RNA
   <213> Artificial Sequence
<220>
   <223> mPlekho1
<400> 558
   cctcccagca cagtcaata 19
<210> 559
   <211> 19
   <212> RNA
   <213> Artificial Sequence
<220>
   <223> mPlekho1
<400> 559
   cgtgagtcct gaagagaag 19
<210> 560
   <211> 19
   <212> RNA
   <213> Artificial Sequence
<220>
   <223> mPlekho1
<400> 560
   tgaaaggcga ccagctcta 19
<210> 561
   <211> 19
   <212> RNA
   <213> Artificial Sequence
<220>
   <223> mPlekho1
<400> 561
   cgggtcgatc tggacaagt 19
<210> 562
   <211> 19
   <212> RNA
   <213> Artificial Sequence
<220>
   <223> mPlekho1
<400> 562
   ccggaaattc tgcgggaaa 19
<210> 563
   <211> 19
   <212> RNA
   <213> Artificial Sequence
<220>
   <223> mPlekho1
<400> 563
   gtgtttgacc tgagtgact 19
<210> 564
   <211> 19
   <212> RNA
   <213> Artificial Sequence
<220>
   <223> mPlekho1
<400> 564
   ccgtgagtcc tgaagagaa 19
<210> 565
   <211> 19
   <212> RNA
   <213> Artificial Sequence
<220>
   <223> mPlekho1
<400> 565
   ctaaaaaccg tatcttgga 19
<210> 566
   <211> 19
   <212> RNA
   <213> Artificial Sequence
<220>
   <223> mPlekho1
<400> 566
   cgttgaggag gacagctat 19
<210> 567
   <211> 19
   <212> RNA
   <213> Artificial Sequence
<220>
   <223> mPlekho1
<400> 567
   cctactcgag acagagcaa 19
<210> 568
   <211> 19
   <212> RNA
   <213> Artificial Sequence
<220>
   <223> mPlekho1
<400> 568
   cctcttgtgc tgagagctt 19
<210> 569
   <211> 19
   <212> RNA
   <213> Artificial Sequence
<220>
   <223> mPlekho1
<400> 569
   ccacaagcac tgaagaagt 19
<210> 570
   <211> 19
   <212> RNA
   <213> Artificial Sequence
<220>
   <223> mPlekho1
<400> 570
   cacagtcaat accggaaga 19
<210> 571
   <211> 19
   <212> RNA
   <213> Artificial Sequence
<220>
   <223> mPlekho1
<400> 571
   tgactatgag aagtgcgaa 19
<210> 572
   <211> 19
   <212> RNA
   <213> Artificial Sequence
<220>
   <223> mPlekho1
<400> 572
   caggaggtgt ttgacctga 19
<210> 573
   <211> 19
   <212> RNA
   <213> Artificial Sequence
<220>
   <223> mPlekho1
<400> 573
   cctctacctc agatgggat 19
<210> 574
   <211> 19
   <212> RNA
   <213> Artificial Sequence
<220>
   <223> mPlekho1
<400> 574
   ggccttggga aaaaccaga 19
<210> 575
   <211> 19
   <212> RNA
   <213> Artificial Sequence
<220>
   <223> mPlekho1
<400> 575
   gatgggatgc taacattag 19
<210> 576
   <211> 19
   <212> RNA
   <213> Artificial Sequence
<220>
   <223> mPlekho1
<400> 576
   agaaggagtc atggatcaa 19
<210> 577
   <211> 19
   <212> RNA
   <213> Artificial Sequence
<220>
   <223> mPlekho1
<400> 577
   acctcccagc acagtcaat 19
<210> 578
   <211> 19
   <212> RNA
   <213> Artificial Sequence
<220>
   <223> mPlekho1
<400> 578
   ccgttgagga ggacagcta 19
<210> 579
   <211> 19
   <212> RNA
   <213> Artificial Sequence
<220>
   <223> mPlekho1
<400> 579
   ccaccctact cgagacaga 19
<210> 580
   <211> 19
   <212> RNA
   <213> Artificial Sequence
<220>
   <223> mPlekho1
<400> 580
   gacaggatcc agccctctt 19
<210> 581
   <211> 19
   <212> RNA
   <213> Artificial Sequence
<220>
   <223> mPlekho1
<400> 581
   ctactcgaga cagagcaaa 19
<210> 582
   <211> 19
   <212> RNA
   <213> Artificial Sequence
<220>
   <223> mPlekho1
<400> 582
   tcaccttcaa gctgaggaa 19
<210> 583
   <211> 19
   <212> RNA
   <213> Artificial Sequence
<220>
   <223> mPlekho1
<400> 583
   cggactcaga caggatcca 19
<210> 584
   <211> 19
   <212> RNA
   <213> Artificial Sequence
<220>
   <223> mPlekho1
<400> 584
   ggcttcgacc tctacctca 19
<210> 585
   <211> 19
   <212> RNA
   <213> Artificial Sequence
<220>
   <223> mPlekho1
<400> 585
   gagctaaaaa ccgtatctt 19
<210> 586
   <211> 19
   <212> RNA
   <213> Artificial Sequence
<220>
   <223> mPlekho1
<400> 586
   ccgtatcttg gatgaggtc 19
<210> 587
   <211> 19
   <212> RNA
   <213> Artificial Sequence
<220>
   <223> mPlekho1
<400> 587
   tcaagctgag gaatcccta 19
<210> 588
   <211> 19
   <212> RNA
   <213> Artificial Sequence
<220>
   <223> mPlekho1
<400> 588
   tgagagacct gtacaggca 19
<210> 589
   <211> 19
   <212> RNA
   <213> Artificial Sequence
<220>
   <223> mPlekho1
<400> 589
   ggacagctat cttgcccac 19
<210> 590
   <211> 19
   <212> RNA
   <213> Artificial Sequence
<220>
   <223> mPlekho1
<400> 590
   ctgtggcttc gacctctac 19
<210> 591
   <211> 19
   <212> RNA
   <213> Artificial Sequence
<220>
   <223> mPlekho1
<400> 591
   ttttcaggga gatttggaa 19
<210> 592
   <211> 19
   <212> RNA
   <213> Artificial Sequence
<220>
   <223> mPlekho1
<400> 592
   accgctatgt ggtgctgaa 19
<210> 593
   <211> 19
   <212> RNA
   <213> Artificial Sequence
<220>
   <223> mPlekho1
<400> 593
   tggatcaacg ccctgagtt 19
<210> 594
   <211> 19
   <212> RNA
   <213> Artificial Sequence
<220>
   <223> mPlekho1
<400> 594
   aaaaccgtat cttggatga 19
<210> 595
   <211> 19
   <212> RNA
   <213> Artificial Sequence
<220>
   <223> mPlekho1
<400> 595
   agacctgatc caagaggaa 19
<210> 596
   <211> 19
   <212> RNA
   <213> Artificial Sequence
<220>
   <223> mPlekho1
<400> 596
   acctgatcca agaggaaga 19
<210> 597
   <211> 19
   <212> RNA
   <213> Artificial Sequence
<220>
   <223> mPlekho1
<400> 597
   gaggtaaaag atgagaaaa 19
<210> 598
   <211> 19
   <212> RNA
   <213> Artificial Sequence
<220>
   <223> mPlekho1
<400> 598
   acgtctccga gaaggaggt 19
<210> 599
   <211> 19
   <212> RNA
   <213> Artificial Sequence
<220>
   <223> mPlekho1
<400> 599
   agagaaggag tcatggatc 19
<210> 600
   <211> 19
   <212> RNA
   <213> Artificial Sequence
<220>
   <223> mPlekho1
<400> 600
   acctctacct cagatggga 19
<210> 601
   <211> 19
   <212> RNA
   <213> Artificial Sequence
<220>
   <223> SiCON
<400> 601
   cuuacgcuga guacuucga 19
<210> 602
   <211> 23
   <212> RNA
   <213> Artificial Sequence
<220>
   <223> hCTGF
<400> 602
   ttacaactgt cccggagaca atg 23
<210> 603
   <211> 23
   <212> RNA
   <213> Artificial Sequence
<220>
   <223> hCTGF
<400> 603
   aagggcaaaa agtgcatccg tac 23
<210> 604
   <211> 23
   <212> RNA
   <213> Artificial Sequence
<220>
   <223> hCTGF
<400> 604
   ttgaatcgct gtactacagg aag 23

## Description

### Technical Field

The present invention relates to a respiratory disease-related gene-specific siRNA and a high efficient structure comprising double helical-oligo RNA ('double helical-oligo RNA structure') containing the siRNA. The double helical-oligo RNA structure has a structure in which a hydrophilic material and a hydrophobic material are conjugated to both ends of double helical RNA (siRNA) by using a simple covalent bond or a linker-mediated covalent bond so as to be effectively delivered into cells, wherein the structure may be converted into a nanoparticle form by hydrophobic interactions of the double helical-oligo RNA structures in an aqueous solution. The siRNA included in the double helical-oligo RNA structure is preferably a siRNA specific to CTGF, Cyr61, or Plekhol (hereinafter, referred to as a CTGF, Cyr61 or Plekhol-specific siRNA), which is a gene related with respiratory diseases, particularly, idiopathic pulmonary fibrosis and chronic obstructive pulmonary disease (COPD).

Further, the present invention relates to a method for producing the double-helical oligo RNA structure and a pharmaceutical composition containing the double-helical oligo RNA structure for preventing or treating respiratory diseases, particularly, idiopathic pulmonary fibrosis and COPD.

### Background Art

Technologies for inhibiting gene expression are important tools in the development of a therapeutic agent and target validation for treating diseases. Among the technologies, since a role of an RNA interference (hereinafter, referred to as 'RNAi') had been found, it was found that the RNA interference acts on a sequence-specific mRNA in various kinds of mammalian cells (Silence of the transcripts: RNA interference in medicine. J. Mol. Med. (2005) 83: 764-773. When a long-chain double-stranded RNA is delivered into a cell, the delivered double-stranded RNA is converted into a small interfering RNA (hereinafter, referred to as 'siRNA') which is processed to 21 to 23 base pairs (bp) by Dicer endonuclease. siRNA is bonded to an RNA-induced silencing complex (RISC), whereby a guide (antisense) strand recognizes and decomposes a target mRNA to sequence-specifically inhibit expression of a target gene (NUCLEIC-ACID THERAPEUTICS: BASIC PRINCIPLES AND RECENT APPLICATIONS. Nature Reviews Drug Discovery. 2002. 1, 503-514).

Bertrand et al., found that as compared to an antisense oligonucleotide (ASO) on the same target gene, siRNA has an effect of significantly inhibiting mRNA expression in vitro and in vivo, and the corresponding effect is maintained for a long time (Comparison of antisense oligonucleotides and siRNAs in cell culture and in vivo. Biochem. Biophys. Res. Commun. 2002. 296: 1000-1004. In addition, since siRNA is complementarily coupled to a target mRNA to sequence-specifically regulate an expression of the target gene, a mechanism of the siRNA has an advantage in that a target to be applicable may be remarkably increased as compared to the existing antibody-based medical product or chemical pharmaceuticals (small molecular drug) (Progress Towards in Vivo Use of siRNAs. MOLECULAR THERAPY. 2006 13(4):664-670).

In order to develop the siRNA as a therapeutic agent even with excellent effect and variously usable range of the siRNA, the siRNA needs to be effectively delivered to a target cell with improved stability and a more efficient cell delivery of the siRNA (Harnessing In Vivo siRNA Delivery for Drug Discovery and Therapeutic Development. Drug Discov. Today. 2006 Jan; 11(1-2):67-73.

In order to solve the problems, research into modification of some nucleotides or backbone of siRNA to have a nucleic acid lyase resistance, or use of carriers such as viral vectors, liposomes or nanoparticles, etc. for improving stability in vivo, has been actively attempted.

Delivery systems using viral vectors such as adenovirus, retrovirus, etc, have high transfection efficacy, and also have high immunogenicity and oncogenicity. Meanwhile, a non-viral delivery system including nanoparticles has a low cell delivery efficiency as compared to the viral delivery system, but has high stability in vivo and is possible to be target-specifically delivered, has highly improved delivery effects such as uptake, internalization, etc., of RNAi oligonucleotide into cells or tissues, and rarely has cytotoxicity and immune stimulation, such that the non-viral delivery system is currently evaluated as a viable delivery method as compared to the viral delivery systems (Nonviral delivery of synthetic siRNA s in vivo. J Clin Invest. 2007 December 3; 117(12): 3623-3632).

In a method using a nanocarrier in the non-viral delivery systems, various polymers such as liposomes, cationic polymer composites, etc., are used to form nanoparticles, and a siRNA is supported on the nanoparticles, that is, nanocarrier, to be delivered into the cells. Among the methods using nanocarriers, a method using polymeric nanoparticle, polymer micelle, lipoplex, or the like, is mainly used, wherein the lipoplex consists of cationic lipid to interact with anionic lipid of endosome of a cell, thereby eliciting a destabilization effect of the endosome to deliver the siRNA into a cell (Proc. Natl. Acad. Sci. 15; 93(21):11493-8, 1996).

In addition, it is known that chemical materials, etc., are connected to end portions of a siRNA passenger (sense) strand to provide promoted pharmacokinetics characteristics, such that high efficacy may be induced in vivo (Nature 11; 432(7014):173-8, 2004). Here, stability of the siRNA may vary depending on properties of the chemical materials bonded to ends of the siRNA sense (passenger) or antisense (guide) strand. For example, a siRNA to which a polymer compound such as polyethylene glycol (PEG) is conjugated, interacts with an anionic phosphate group of siRNA in the presence of cationic materials to form a complex, thereby being a carrier having an improved siRNA stability (J. Control Release 129(2):107-16, 2008). In particular, micelle consisting of polymer complexes has an extremely small size, significantly uniform distribution, and is spontaneously form, thereby being easy to manage quality of formulation and secure reproducibility, as compared to other systems used as a drug delivery vehicle, such as microsphere, nanoparticle, etc.

Further, in order to improve an intracellular delivery efficiency of siRNA, technology of using a siRNA conjugate in which hydrophilic material which is a biocompatible polymer (for example, polyethylene glycol (PEG)) is conjugated to the siRNA by a simple covalent bond or a linker-mediated covalent bond, to thereby secure stability of siRNA and have effective cell membrane permeability was developed (see Korean Patent Publication No. 883471). However, the chemical modification of the siRNA and the conjugation with the polyethylene glycol (PEG) (PEGylation) still have disadvantages that stability in vivo is low and delivery into a target organ is not smooth. In order to solve the disadvantages, a double-helical oligo RNA structure in which a hydrophilic material and a hydrophobic material are bonded to oligonucleotides, in particular, double helical RNA such as siRNA, was developed, wherein the double-helical oligo RNA structure forms a self assembled nanoparticle named a self assembled micelle inhibitory RNA (SAMiRNA™) by a hydrophobic interaction of a hydrophobic material (see Korean Patent Publication No. 1224828), the SAMiRNA™ technology has an advantage in that homogenous nanoparticles having a significantly small size are capable of being obtained as compared to the existing delivery technologies.

As a specific example of the SAMiRNA™ technology, PEG (polyethylene glycol) is used as a hydrophilic material, wherein PEG is synthetic polymer, which is used for increasing solubility of pharmaceuticals, particularly, protein, and for controlling pharmacokinetics. PEG is a polydisperse material, a polymer in one batch consists of the sum of different number of monomers, a molecular weight is shown in the Gaussian curve, and polydispersity values (Mw/Mn) express the homogeneity degree of a material. That is, when PEG has a low molecular weight (3 to 5 kDa), the polydisperse value is about 1.01, and when PEG has a high molecular weight (20 kDa), the polydisperse value is about 1.2, which is high, such that as the molecular weight is higher, the homogeneity of the material is relatively low (F. M. Veronese. Peptide and protein PEGylation: a review of problems and solutions. Biomaterials (2001) 22:405-417). Accordingly, when the PEG is combined to the pharmaceuticals, polydispersed characteristic of PEG is reflected on the conjugate, such that it is not easy to perform verification of single material, and accordingly, production of materials having a low polydisperse value through synthesis of PEG and improvement of purification processes is on a rising trend, but has still problems due to polydispersed characteristic of the material, particularly, when PEG is combined to a material having a small molecular weight, there is difficult in confirming whether or not the combination is easily performed, etc. (Francesco M. Veronese and Gianfranco Pasut. PEGylation, successful approach to drug delivery. DRUG DISCOVERY TODAY (2005) 10(21):1451-1458).

Accordingly, recently, as an improved technology of SAMiRNA™ which is the existing self-assembled nanoparticles, a technology of a new form of carrier having a significantly small size as compared to the existing SAMiRNA™ and remarkably improved polydispersity has been developed by blocking the hydrophilic material of the double helical RNA structure configuring SAMiRNA™ as a base unit including 1 to 15 uniform monomers having a predetermined molecular weight and a linker as needed, and using the appropriate number of blocked hydrophilic materials as needed.

Meanwhile, since biopharmaceuticals specifically act to a target gene sequence or a protein structure, in order to evaluate the efficacy and safety in a non-clinical surrogate model, a material acting with the same mechanism as the corresponding biopharmaceuticals in human is additionally required even in species of the surrogate model. Therefore, in order to avoid difficulty in additionally finding the material including the same mechanism as human, a material acting with the same mechanism both in human (treatment target) in the mouse (the non-clinical surrogate model), is required to be developed.

Idiopathic Pulmonary Fibrosis (hereinafter, abbreviated as 'IPF') is a disease in which chronic inflammatory cells penetrate into a wall of an alveolar wall (lung alveolus) to make the lung become hard, causing severe structural change in lung tissue, such that lung function is gradually reduced to induce death. However, an effective treatment thereof does not exist yet, and Idiopathic Pulmonary Fibrosis is generally diagnosed when symptoms appear at last, and has extremely bad prognosis since a median survival time is only about three to five years. It is reported that the incidence frequency of the foreign countries is about 3-5 people per 100,000, and it is known that the incidence is generally high after the age of 50, and men have a two-times higher incidence than women.

The cause of IPF has not been clearly identified yet, and it is merely reported that high frequency is shown in smokers, antidepressants, chronic pulmonary inhalation due to gastroesophageal reflux, metal dust, wood dust, solvent inhalation, etc., are regarded as risk factors related with IPF occurrence. However, definitive causal factors cannot be found in the majority of patients.

It is known that IPF is continuously worsen with no treatment, and about 50% of patients die within 3-5 years, and once a lung is completely hardened by fibrosis as the disease progresses, there is no improvement despite any treatment, and accordingly, it is predicted that an early treatment increases efficacy. As the currently used therapeutic agent, a combination therapy method using steroid and azathioprine or cyclophosphamide, has been known, but it is difficult to say that there are special effects, and attempts of several fibrosis inhibitors in animal experiments and small group of patients failed in proving clear effects. In particular, there is no other effective treatment in patients with end-stage IPF, in addition to lung transplantation. Therefore, development of more effective therapeutic agent against IPF is desperately required.

Meanwhile, COPD, one of representative lung diseases together with asthma, is different from asthma in that it is accompanied by irreversible airway obstruction, and is a respiratory disease which is accompanied by abnormal inflammatory response of lung caused by repeated infection, harmful particles, gas inlet or smoking, and is not fully reversible, but shows increasingly progressed airflow limitation (Pauwels et al, Am J Respir Crit Care Med, 163:1256-1276, 2001). COPD is a disease caused by pathological changes of bronchioles and lung parenchyma by airway and lung parenchyma inflammation, and is characterized by obstructive bronchiolitis and emphysema (destruction of lung parenchyma). Types of COPD include chronic obstructive bronchitis, chronic bronchiolitis and emphysema. In COPD, the number of neutrophils is increased, and secretion of cytokines such as GM-CSF, TNF-α, IL-8, and MIP-2 is increased. Further, airway inflammation, a thickened muscle wall, and bronchial closure due to increased mucus secretion are also shown. When bronchus is closed, alveoli are expanded and damaged, such that an exchange ability of oxygen and carbon dioxide is damaged to increase respiratory failure.

The severity of COPD is emerging around the world since it is predicted that COPD ranked 6^{th} in 1990 among causes of death due to disease, but will rank in third place in 2020, and is the only disease of which the incidence is increased in 10 diseases. COPD has a high prevalence rate, causes a respiratory disorder, and requires a large amount of direct medical costs required for diagnosis and treatment of COPD, and a significant amount of indirect expenses such as losses due to dyspnea and leave of absence or loss due to premature death, which is a social and economic problem in the world (Chronic obstructive pulmonary disease (COPD) treatment guidelines 2005. Chronic obstructive airway disease Clinical Research Center, p.30-31).

Existing therapeutic drugs have not been confirmed as relieving reduction in long time lung function which is a characteristic of COPD. Accordingly, drug treatment in COPD has a main purpose of reducing symptoms or complications. Among the therapeutic drugs, a bronchodilator is a typical COPD allopathic drug, and an anti-inflammatory drug or corticosteroid is usually prescribed, but the effect is not significant, the application range is narrow, and there is great concern for side effects. As other drugs, it is known that only the influenza vaccine reduces serious symptoms and death by about 50% in patients with COPD (chronic obstructive pulmonary disease (COPD) treatment guidelines 2005. Chronic obstructive airway disease Clinical Research Center. p.52-58).

Meanwhile, many genetic factors are estimated as increasing (or decreasing) the risk of individual COPD. A genetic risk factor which has been demonstrated so far is genetic deficiency of α1-antitrypsin. Smoking significantly increases the risk of COPD, but occurrence of panlobular emphysema and reduction in lung function that rapidly progress at a young age are shown by both of smokers and non-smokers having significant genetic deficiency. Although other genes confirmed to be related with the COPD pathogenesis do not exist yet in addition to α1-antitrypsin, attempt for identifying biomarkers of the disease to be utilized for diagnosis through research into cellular, molecular, and genetic abnormal states that are basically shown in patients with COPD or attempt for finding a new treatment method is being progressed (P.J. Barnes and R.A. Stockely. COPD: current therapeutic interventions and future approaches. Eur Respir J. (2005) 25:1084-1106. In particular, research into diagnosis of COPD and selection of target for treatment through methods such as gene microarray, proteomics, etc., has been actively conducted, and analyses of genetic factors for obtaining COPD sensitivity (susceptibility) and causes of the worsen COPD symptoms induced by smoking have been mainly conducted (Peter J. Castaldi et al. The COPD genetic association compendium. Human Molecular Genetics, 2010, Vol. 19, No. 3 526-534).

CTGF (connective tissue growth factor; CCN2), which is one of matricellular proteins included in CCN family, is known to be a secretion cytokine involved in various biological processes such as cell adhesion, migration, proliferation, angiogenesis, wound repair, etc. Over-expression of CTGF is regarded as being main cause of symptoms such as scleroderma, fibrotic disease, and scar formation (Brigstock DR. Connective tissue growth factor (CCN2, CTGF) and organ fibrosis: lessons from transgenic animals. J Cell Commun Signal (2010) 4 (1): 1-4).In particular, regarding fibrosis, CTGF is known to cause sustained fibrosis together with TGF-β (Transforming growth factor-β) or to promote production of ECM (extracelluar matrix) under a condition in which fiber formation is caused, and recently, it is known to be capable of treating ocular disorders or muscular dystrophy caused by abnormal expression of CTGF by treating samples or materials that hinder expression of CTGF or inhibiting action thereof, but relevancy with a respiratory disease has not been suggested (U.S. Patent No. 7,622,454, and U.S Patent Application Publication No. 20120164151).

CYR61 (Cysteine-rich angiogenic inducer 61) is an extracelluar matrix (ECM)-related signaling protein included in CCN family, which is known to control various cellular activities such as cell adhesion, migration, proliferation, differentiation, apoptosis, etc. The purified CYR61 promotes attachment and spreading of endothelial cells in a similar manner to fibronectin, and does not have mitogenic activity, but acts to reinforce a mitogen effect of a fibroblast growth factor (MARIA L. KIREEVA et al. Cyr61, a Product of a Growth Factor-Inducible Immediate-Early Gene, Promotes Cell Proliferation, Migration, and Adhesion. MOLECULAR AND CELLULAR BIOLOGY, Apr. 1996, p. 1326-1334).

It is reported that Plekhol (Pleckstrin homology domain-containing family O member 1) is present in a plasma membrane or nucleus, acts as a non-enzymatic regulator of protein kinase CK2α1(Casein kinase 2, alpha 1), and is involved in apoptosis by inhibition of AP-1 action through C-terminal piece produced when being decomposed by Caspase 3 (Denis G. Bosc et al. Identification and Characterization of CKIP-1, a Novel Pleckstrin Homology Domain-containing Protein That Interacts with Protein Kinase CK2. The Journal of Biological Chemistry(2000) 275, 14295-14306; Lingqiang Zhang et al. Role for the pleckstrin homology domain containing protein CKIP-1 in AP-1 regulation and apoptosis. The EMBO Journal (2005) 24, 766-778).

As described above, prevalence of the respiratory diseases, particularly, idiopathic pulmonary fibrosis and COPD has increased, but therapeutic agents being capable of basically preventing and treating the idiopathic pulmonary fibrosis and COPD do not exist yet. Therefore, the demand for the therapeutic agent for the idiopathic pulmonary fibrosis and COPD showing high level of prevention and treatment effects without side effects is significantly largely present in the market.

WO 2011/119887 A1 discloses a double-stranded ribonucleic acid (dsRNA) comprising a sense strand and an antisense strand wherein the antisense strand is complementary to at least 12 contiguous nucleotides and wherein the dsRNA is an sd-rxRNA.

US 2008/0108583 A1 discloses inhibitors, in particular siRNAs, which down-regulate the expression of human pro-apoptotic genes.US 2013/0096288 A1 discloses a hybrid conjugate formed by covalently bonding siRNA and a polymeric compound for improving the vivo stability of siRNA.

### DISCLOSURE OF INVENTION

The embodiment of the present invention is reflected in claim 1.
Preferred embodiments are reflected in dependent claims 2 to 12.

An object of the present invention is to provide a novel siRNA specific to CTGF (hereinafter, referred to as a CTGF-specific siRNA), capable of inhibiting expression thereof at a significantly high efficiency, a double helical-oligo RNA structure.

Another object of the present invention is to provide a pharmaceutical composition including the CTGF-specific siRNA or the double-helical oligo RNA structure containing the siRNA as an effective component, for preventing or treating respiratory diseases, particularly, idiopathic pulmonary fibrosis and COPD.

Another object of the present invention is to provide a method for preventing or treating respiratory diseases, particularly, idiopathic pulmonary fibrosis and COPD, by using the CTGF-specific siRNA or the double-helical oligo RNA structure containing the siRNA.

### BRIEF Description of THE Drawings

FIG. 1 is a schematic diagram of a nanoparticle formed of a double-helical oligo polymer structure according to the present invention.
FIG. 2 is a graph showing an inhibition amount of target gene expression, confirmed after transforming a human fibroblast cell line with siRNA having sequence of SEQ ID NOs: 1 to 10, 101 to 110, and 201 to 210 as a sense strand according to the present invention.
   A : Graph showing CTGF expression amount according to treatment of 5 or 20 nM of siRNA having sequence of SEQ ID NOs: 1 to 10 as a sense strand.
   B : Graph showing Cyr61 expression amount according to treatment of 5 or 20 nM of siRNA having sequence of SEQ ID NOs: 101 to 110 as a sense strand.
   C : Graph showing Plekhol expression amount according to treatment of 5 or 20 nM of siRNA having sequence of SEQ ID NOs: 201 to 210 as a sense strand.

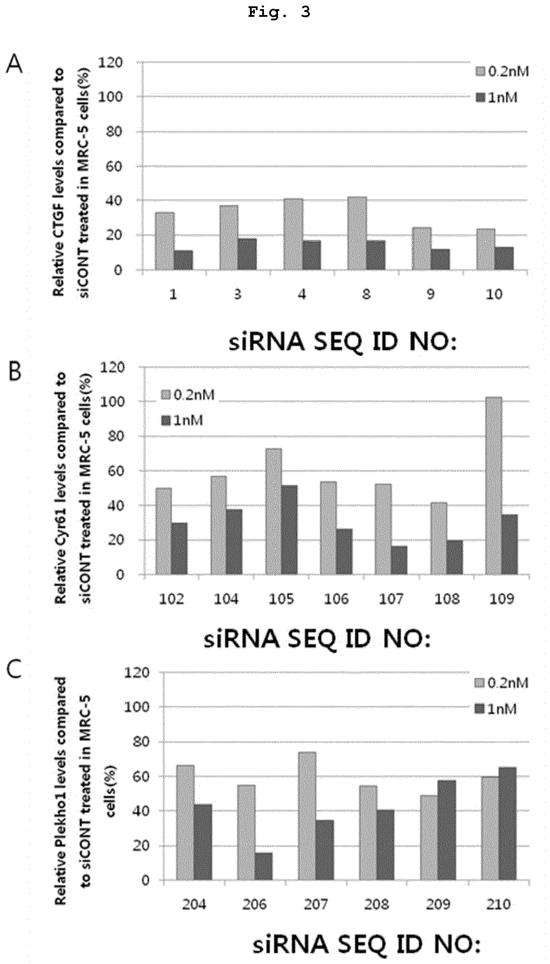

FIG. 3 is a graph showing an inhibition amount of target gene expression, confirmed after transforming a human fibroblast cell line with siRNA having sequence of SEQ ID NO: 1, 3, 4, 8, 9, 10, 102, 104, 105, 106, 107, 108, 109, 204, 206, 207, 208, 209 or 210 as a sense strand according to the present invention.
   A : Graph showing CTGF expression amount according to treatment of 0.2 or 1 nM of siRNA having sequence of SEQ ID NO: 1, 3, 4, 8, 9 or 10 as a sense strand.
   B : Graph showing Cyr61 expression amount according to treatment of 0.2 or 1 nM of siRNA having sequence of SEQ ID NO: 102, 104, 105, 106, 107, 108 or 109 as a sense strand.
   C : Graph showing Plekhol expression amount according to treatment of 0.2 or 1 nM of siRNA having sequence of SEQ ID NO: 204, 206, 207, 208, 209, or 210 as a sense strand.

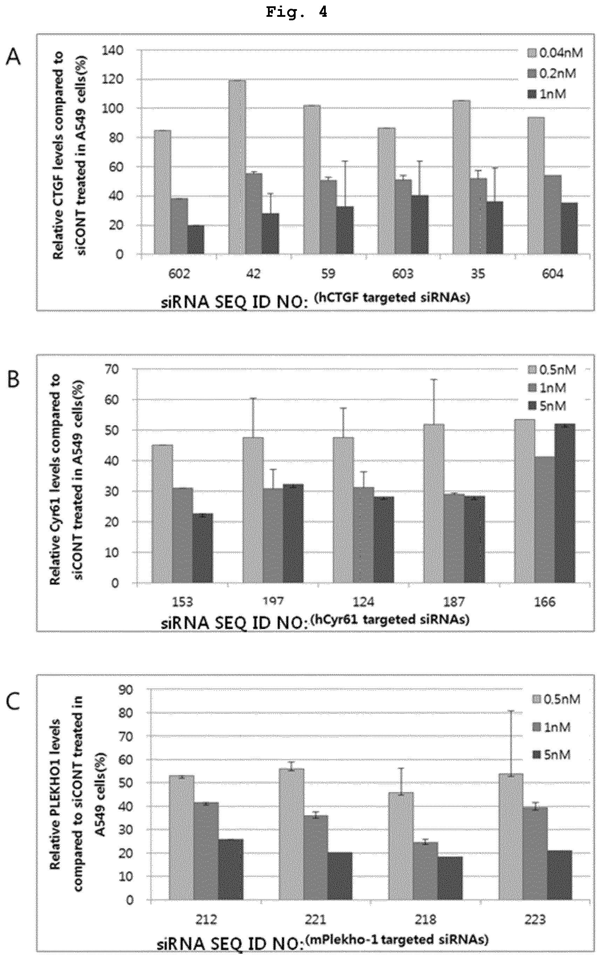

FIG. 4 is a graph showing an inhibition amount of target gene expression, confirmed after transforming a human lung cancer cell line with siRNA having sequence of SEQ ID NO: 35, 42, 59, 602, 603, 604, 124, 153, 166, 187, 197, 212, 218, 221 or 223 as a sense strand according to the present invention.
   A : Graph showing CTGF expression amount according to treatment of 0.04, 0.2 or 1 nM of siRNA having sequence of SEQ ID NO: 35, 42, 59, 602, 603, or 604 as a sense strand.
   B : Graph showing Cyr61 expression amount according to treatment of 0.5, 1 or 5 nM of siRNA having sequence of SEQ ID NO: 124, 153, 166, 187, or 197 as a sense strand.
   C : Graph showing Plekhol expression amount according to treatment of 0.5, 1 or 5 nM of siRNA having sequence of SEQ ID NO: 212, 218, 221 or 223 as a sense strand.

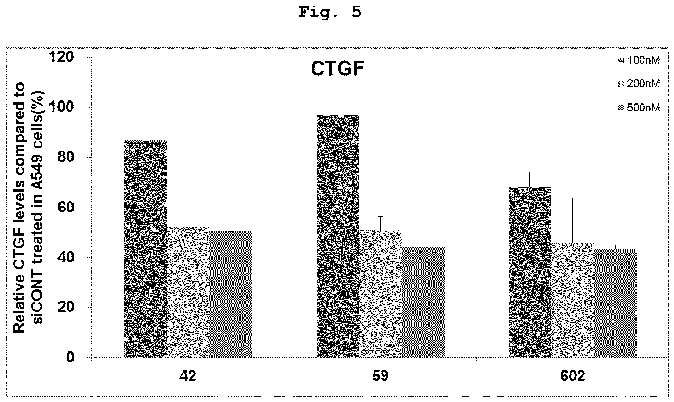

FIG. 5 is a graph showing an inhibition amount of target gene expression, confirmed after transforming a human lung cancer cell line with SAMiRNA having sequence of SEQ ID NO: 42, 59, or 602 as a sense strand according to the present invention.

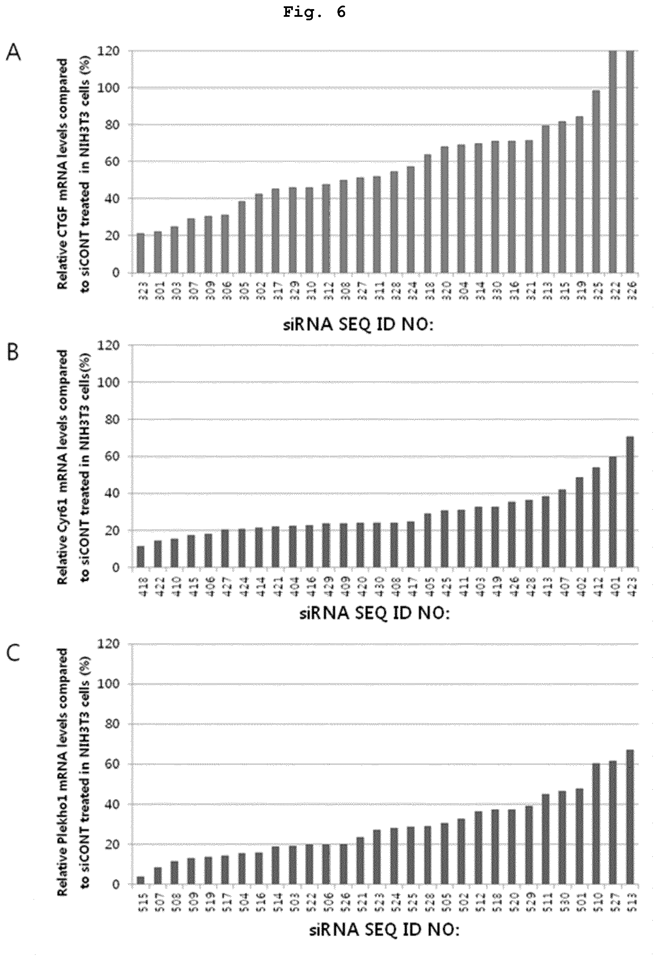

FIG. 6 is a graph showing an inhibition amount of target gene expression, confirmed after transforming a mouse fibroblast cell line with siRNA having sequence of SEQ ID NOs: 301 to 330, 401 to 430, 501 to 530 as a sense strand according to the present invention.
   A : Graph showing CTGF expression amount according to treatment of 20 nM of siRNA having sequence of SEQ ID NOs: 301 to 330 as a sense strand.
   B : Graph showing Cyr61 expression amount according to treatment of 20 nM of siRNA having sequence of SEQ ID NOs: 401 to 430 as a sense strand.
   C : Graph showing Plekhol expression amount according to treatment of 20 nM of siRNA having sequence of SEQ ID NOs: 501 to 530 as a sense strand.

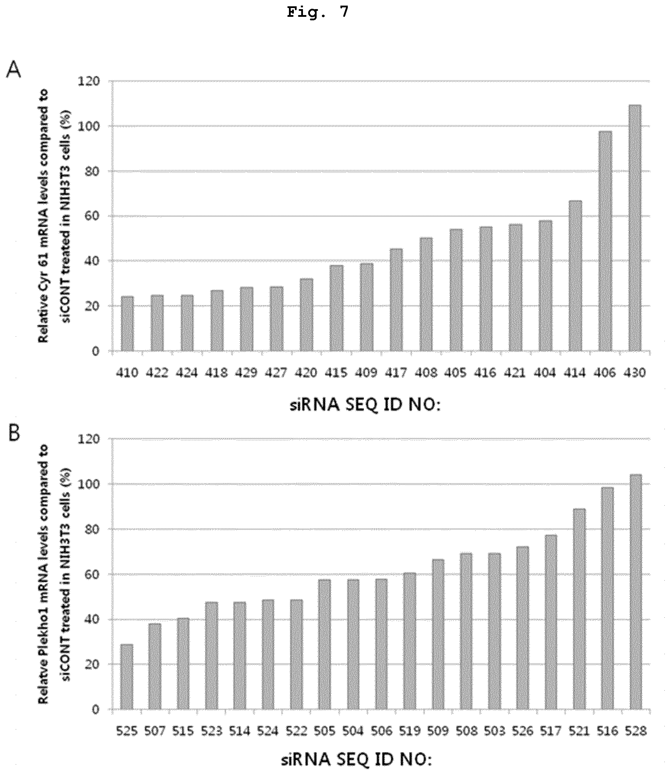

FIG. 7 is a graph showing an inhibition amount of target gene expression, confirmed after transforming a mouse fibroblast cell line with siRNA having sequence of SEQ ID NO: 404 to 406, 408 to 410, 414 to 418, 420 to 422, 424, 427, 429, 430, 503 to 509, 514 to 517, 519, 521 to 526 or 528 as a sense strand according to the present invention.
   A: Graph showing Cyr61 expression amount according to treatment of 5 nM of siRNA having sequence of SEQ ID NOs: 404 to 406, 408 to 410, 414 to 418, 420 to 422, 424, 427, 429, or 430 as a sense strand.
   B : Graph showing Plekhol expression amount according to treatment of 5 nM of siRNA having sequence of SEQ ID NO: 503 to 509, 514 to 517, 519, 521 to 526 or 528 as a sense strand.

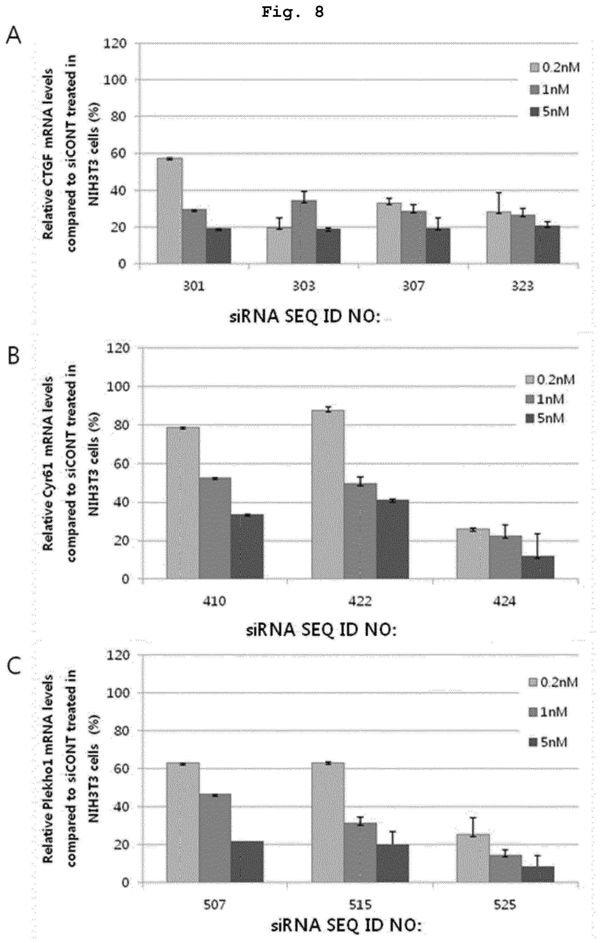

FIG. 8 is a graph showing an inhibition amount of target gene expression, confirmed after transforming a mouse fibroblast cell line with siRNA having sequence of SEQ ID NO: 301, 303, 307, 323, 410, 422, 424, 507, 515 or 525 as a sense strand according to the present invention.
   A : Graph showing CTGF expression amount according to treatment of 0.2, 1, or 5 nM of siRNA having sequence of SEQ ID NO: 301, 303, 307 or 323 as a sense strand.
   B : Graph showing Cyr61 expression amount according to treatment of 0.2, 1, or 5 nM of siRNA having sequence of SEQ ID NO: 410, 422 or 424 as a sense strand.
   C : Graph showing Plekhol expression amount according to treatment of 0.2, 1 or 5 nM of siRNA having sequence of SEQ ID NO: 507, 515 or 525 as a sense strand.

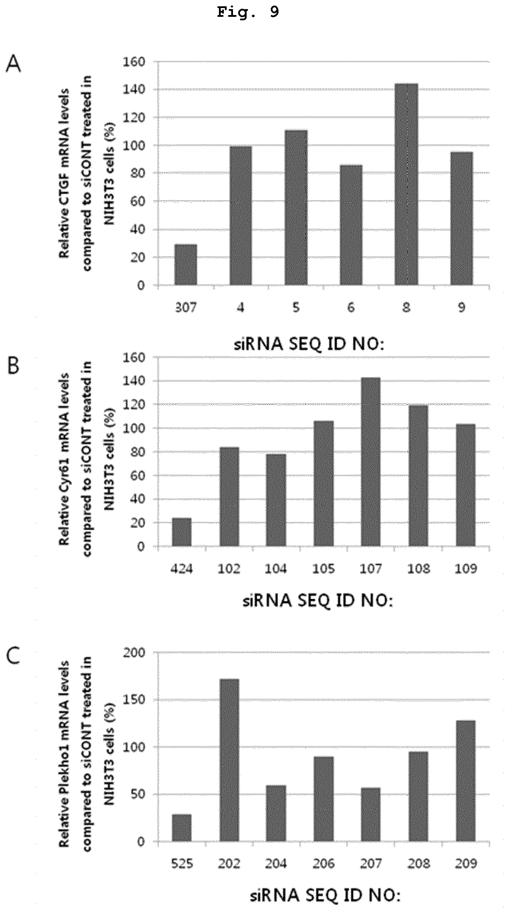

FIG. 9 is a graph showing an inhibition amount of target gene expression, confirmed after transforming a mouse fibroblast cell line with siRNA having a SEQ ID NO: 4, 5, 6, 8, 9, 102, 104, 105, 107, 108, 109, 202, 204, 206 to 209, 307, 424 or 525 as a sense strand according to the present invention.
   A : Graph showing CTGF expression amount according to treatment of 5 nM of siRNA having sequence of SEQ ID NO: 4, 5, 6, 8, 9 or 307 as a sense strand.
   B : Graph showing Cyr61 expression amount according to treatment of 5 nM of siRNA having sequence of SEQ ID NO: 102, 104, 105, 107, 108, 109 or 424 as a sense strand.
   C : Graph showing Plekhol expression amount according to treatment of 5 nM of siRNA having sequence of SEQ ID NO: 202, 204, 206 to 209 or 525 as a sense strand.

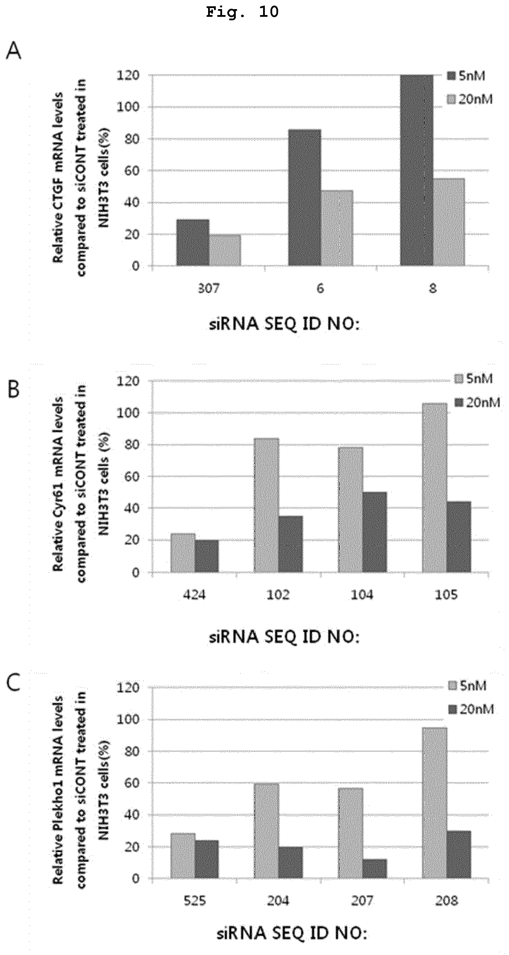

FIG. 10 is a graph showing an inhibition amount of target gene expression, confirmed after transforming a mouse fibroblast cell line with siRNA having sequence of SEQ ID NO: 6, 8, 102, 104, 105, 204, 207, 208, 307, 424 or 525 as a sense strand according to the present invention.
   A : Graph showing CTGF expression amount according to treatment of 5 or 20 nM of siRNA having sequence of SEQ ID NO: 6, 8 or 307 as a sense strand.
   B : Graph showing Cyr61 expression amount according to treatment of 5 or 20 nM of siRNA having sequence of SEQ ID NO: 102, 104, 105 or 424 as a sense strand.
   C : Graph showing Plekhol expression amount according to treatment of 5 or 20 nM of siRNA having sequence of SEQ ID NO: 204, 207, 208 or 525 as a sense strand.

### BEST MODE FOR CARRYING OUT THE INVENTION

In order to achieve the foregoing objects, the present invention provides a CTGF (respiratory diseases-related gene)-specific siRNA consisting of first oligonucleotide which is a sense strand including any one sequence selected from the group consisting of SEQ ID NOs: 1 to 600 and 602 to 604 and a second oligonucleotide which is an antisense strand including a complementary sequence thereto.

The siRNA in the present invention includes all materials having a general RNAi (RNA interference) action, and accordingly, it is obvious to a person skilled in the art that CTGF-specific siRNA includes CTGF-specific shRNA, etc.

SEQ ID NOs: 1 to 100, or 602 to 604 are sense strand sequences of CTGF (Homo sapiens)-specific siRNA.

The siRNA according to the present invention is preferably CTGF-specific siRNA including any one sequence selected from the group consisting of SEQ ID NOs: 1 to 10, 35, 42, 59, 602, 603, 604, 301 to 303, 305 to 307, 309, 317, 323 and 329, as a sense strand,
More preferably, the siRNA according to the present invention is CTGF-specific siRNA including any one sequence selected from the group consisting of SEQ ID NOs: 4, 5, 8, 9, 35, 42, 59, 601, 602, 604, 301, 303, 307 and 323, as a sense strand,
Most preferably, the siRNA according to the present invention is CTGF-specific siRNA including any one sequence selected from the group consisting of SEQ ID NO: 42, 59, 602 and 323, as a sense strand.

In particular, it was confirmed that some of human CTGF-specific siRNA according to the present invention is capable of simultaneously inhibiting expression of mouse CTGF.

The siRNA capable of simultaneously inhibiting the expression of human and mouse CTGF preferably includes a sense strand of CTGF-specific siRNA according to SEQ ID NO: 6 or 8.

Most preferably, the siRNA includes a sense strand of CTGF-specific siRNA according to SEQ ID NO: 6.

The sense strand or the antisense strand of the siRNA according to the present invention preferably has 19 to 31 nucleotides, and the siRNA includes a sense strand including any one sequence selected from SEQ ID NOs: 1 to 604 and an antisense strand complementary thereto.

Since the CTGF-specific siRNA according to the present invention has a base sequence designed to be capable of being complementarily bonded to mRNA encoding the corresponding gene, expression of the corresponding gene is capable of being effectively inhibited. Further, the CTGF-specific siRNA according to the present invention may include overhang which is a structure including one or two or more unpaired nucleotide(s) at 3' end of the siRNA, and
may include various modifications of the siRNA to provide nucleic acid lyase resistance, and to decrease non-specific immune response for improving stability in vivo. The modification of the first oligonucleotide or the second oligonucleotide configuring the siRNA may be one or more combinations selected from modification in which -OH group at 2' carbon in a sugar structure in one or more nucleotides is substituted with -CH₃(methyl), -OCH₃ (methoxy), -NH₂, -F(fluorine), -O-2-methoxyethyl, -O-propyl, -O-2-methylthioethyl, -O-3-aminopropyl, -O-3-dimethylaminopropyl, -O-N-methylacetamido or -O-dimethylamidooxyethyl; modification in which oxygen in a sugar structure in nucleotides is substituted with sulfur; and modification to phosphorothioate or boranophosphate, methyl phosphonate bindings from nucleotides bindings, or may be modification to peptide nucleic acid (PNA), modification to locked nucleic acid (LNA), or modification to unlocked nucleic acid (UNA) (Ann. Rev. Med. 55, 61-65 2004; US 5,660,985; US 5,958,691; US 6,531,584; US 5,808,023; US 6,326,358; US 6,175,001; Bioorg. Med. Chem. Lett. 14:1139-1143, 2003; RNA, 9:1034-1048, 2003; Nucleic Acid Res. 31:589-595, 2003; Nucleic Acids Research, 38(17) 5761-5773, 2010; Nucleic Acids Research, 39(5) 1823-1832, 2011).

The CTGF-specific siRNA according to the present invention may inhibit expression of the corresponding gene, and may remarkably inhibit expression of the corresponding protein.

The present invention also provides a conjugate in which a hydrophilic material and a hydrophobic material are conjugated to both ends of siRNA, for effective in vivo delivery and for improving stability, of the respiratory diseases-related gene-specific siRNA, particularly, the CTGF-specific siRNA.

In the siRNA conjugate in which a hydrophilic material and a hydrophobic material are bonded to the siRNA, a self-assembled nanoparticle is formed by a hydrophobic interaction of the hydrophobic material (see Korean Patent Publication No. 1224828), wherein the self-assembled nanoparticle has advantages in that internal delivery efficiency and stability in vivo are significantly excellent, and uniformity of a particle size is excellent, such that quality control (QC) is easily performed, whereby a process for producing drugs is easy.

In one preferable exemplary embodiment, a double-helical oligo RNA structure containing the CTGF-specific siRNA according to the present invention preferably has a structure represented by the following Structural Formula (1) :

Structural Formula 1 A-X-R-Y-B

In Structural Formula (1), A is a hydrophilic material, B is a hydrophobic material, X and Y are each independently a simple covalent bond or a linker-mediated covalent bond, and R is CTGF-specific siRNA.

More preferably, the double-helical oligo RNA structure containing the CTGF-specific siRNA according to the present invention has a structure represented by the following Structural Formula (2):

Structural Formula 2 A-X-S-Y-B AS

In Structural Formula (2), A, B, X and Y are the same as being defined in Structural Formula (1), S is a sense strand of the CTGF-specific siRNA, and AS is an antisense strand of the CTGF-specific siRNA.

More preferably, the double-helical oligo RNA structure containing the CTGF-specific siRNA according to the present invention has a structure represented by the following Structural Formula (3) or (4):

Structural Formula 3 A-X-5' S 3'-Y-B AS

Structural Formula 4 A-X-3' S S'-Y-B AS

In Structural Formulas (3) and (4), A, B, S, AS, X and Y are the same as being defined in Structural Formula (1), and 5' and 3' mean 5' end and 3' end of the sense strand of the CTGF-specific siRNA.

It is obvious to a person skilled in the art that in Structural Formulas (1) to (4), one to three phosphate group(s) may be bonded to 5' end of the antisense strand of the double-helical oligo RNA structure containing the CTGF-specific siRNA, and shRNA is capable of being used instead of using siRNA.

The hydrophilic material in Structural Formulas (1) to (4) is preferably a cationic or non-ionic polymer material having a molecular weight of 200 to 10,000, more preferably, a non-ionic polymer material having a molecular weight of 1,000 to 2,000. For example, non-ionic hydrophilic polymer compounds such as polyethylene glycol, polyvinyl pyrrolidone, polyoxazoline, etc., are preferably used as the hydrophilic polymer material, but the present invention is not necessarily limited thereto.

In particular, the hydrophilic material (A) in Structural Formulas (1) to (4) may be used in a hydrophilic material block form represented by the following Structural Formula (5) or (6), and by using an appropriate number (n in Structural Formula (5) or (6)) of hydrophilic material blocks as needed, problems caused by polydispersibility that may occur in a case of using a general synthetic polymer material, etc., may be largely improved.

Structural Formula 5 (A'ₘ-J)ₙ

Structural Formula 6 (J-A'ₘ)ₙ

In Structural Formula (5), A' is a hydrophilic material monomer, J is a linker for connecting hydrophilic material monomers (the sum is m) therebetween or a linker for connecting hydrophilic material monomers (the sum is m) to siRNA, m is an integer of 1 to 15, n is an integer of 1 to 10, and the repeating unit represented by (A'ₘ-J) or (J-A'ₘ) corresponds to a base unit of the hydrophilic material block.

When the hydrophilic material block is represented by Structural Formula (5) or (6), the double-helical oligo RNA structure containing the CTGF-specific siRNA according to the present invention may have a structure represented by the following Structural Formula (7) or (8):

Structural Formula 7 (A'ₘ-J)ₙ-X-R-Y-B

Structural Formula 8 (J-A'ₘ)ₙ-X-R-Y-B

In Structural Formulas (7) and (8), X, R, Y and B are the same as being defined in Structural Formula (1), and A', J, m and n are the same as being defined in Structural Formulas (5) and (6).

In Structural Formulas (5) and (6), the hydrophilic material monomer A' is usable without limitation as long as it meets the objects of the present invention among the monomers of the non-ionic hydrophilic polymer. The hydrophilic material monomer A' is preferably a monomer selected from the following compounds (1) to (3) shown in Table 1, more preferably, a monomer of the compound (1), and G in the compound (1) may be preferably selected from CH₂, O, S and NH.

In particular, among the hydrophilic material monomers, the monomer of Compound (1) may have various functional groups introduced thereinto and good affinity in vivo, and induce a little immune response, thereby having excellent bio-compatibility, and further, may increase stability in vivo of the oligonucleotide included in the structure of Structural Formula (7) or (8), and may increase delivery efficiency, which is significantly suitable for producing the structure according to the present invention.

**[Table 1] Monomer structure of hydrophilic material in the present invention**

| Compound (1) | Compound (2) | Compound (3) |
|---|---|---|
| 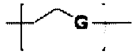 | 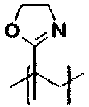 | 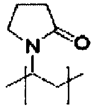 |
| G is O, S or NH | | |

In particular, the hydrophilic material in Structural Formulas (5) to (8) preferably has total molecular weight of 1,000 to 2,000. Therefore, for example, when hexaethylene glycol represented by Compound (1), that is, G is O, and m is 6, is used in Structural Formulas (7) and (8), a molecular weight of hexaethylene glycol spacer is 344, such that the repeating number (n) is preferred to be 3 to 5. In particular, the repeating unit of the hydrophilic group, that is, hydrophilic material block, represented by (A'ₘ-J) or (J-A'ₘ)ₙ in Structural Formulas (5) and (6), is capable of being used in an appropriate number represented by n, as needed. A which is the hydrophilic material monomer and J which is the linker included in each hydrophilic material block may be independently the same as each other or be different from each other. That is, when 3 hydrophilic material blocks are used (n = 3), the hydrophilic material monomer of the compound (1) is used in the first block, the hydrophilic material monomer of the compound (2) is used in the second block, the hydrophilic material monomer of the compound (3) is used in the third block, etc. That is, different hydrophilic material monomers for all hydrophilic material blocks may be used, and any one hydrophilic material monomer selected from the hydrophilic material monomers of compounds (1) to (3) may also be equally used in all hydrophilic material blocks. Similarly, linkers mediating the combination of the hydrophilic material monomer may also be same as each other or different from each other for all hydrophilic material blocks. Further, m which is the number of hydrophilic material monomers may be the same as each other or different from each other among all hydrophilic material blocks. That is, 3 hydrophilic material monomers (m=3) are connected in the first hydrophilic material block, 5 hydrophilic material monomers (m=5) are connected in the second hydrophilic material block, 4 hydrophilic material monomers (m=4) are connected in the third hydrophilic material block, etc. That is, hydrophilic material monomers each having different numbers or each having the same number may be used in all hydrophilic material blocks.

Further, in the present invention, the linker (J) is preferably selected from the group consisting of PO₃⁻, SO₃ and CO₂, but the present invention is not limited thereto. It is obvious to a person skilled in the art that any linker may be used depending on the used hydrophilic material monomer, etc., as long as it meets the objects of the present invention.

The hydrophobic material (B) in Structural Formulas (1) to (4), (7) and (8) serves to form a nanoparticle formed of the oligonucleotide structures according to Structural Formulas (1) to (4), (7) and (8) through hydrophobic interaction. The hydrophobic material preferably has a molecular weight of 250 to 1,000, and may include a steroid derivative, a glyceride derivative, glycerol ether, polypropylene glycol, C₁₂ to C₅₀ unsaturated or saturated hydrocarbon, diacylphosphatidylcholine, fatty acid, phospholipid, lipopolyamine, etc., but the present invention is not limited thereto. It is obvious to a person skilled in the art that any hydrophobic material may be used as long as it meets the objects of the present invention.

The steroid derivative may be selected from the group consisting of cholesterol, cholestanol, cholic acid, cholesteryl formate, cholestanyl formate, and cholesteryl amine, and the glyceride derivative may be selected from mono-, di- and tri-glyceride, etc., wherein the fatty acid of the glyceride is preferred to be C₁₂ to C₅₀ unsaturated or saturated fatty acid.

In particular, among the hydrophobic materials, saturated or unsaturated hydrocarbon or cholesterol is preferred since it is capable of easily being bonded in a synthetic step of the oligonucleotide structure according to the present invention, and C₂₄ hydrocarbon, particularly, tetradocosane including a disulfide bond is the most preferred.

The hydrophobic material is bonded to the distal end of the hydrophilic material, and it does not matter if the hydrophobic material is bonded to any position of the sense strand or the antisense strand of the siRNA.

The hydrophilic material or the hydrophobic material in Structural Formulas (1) to (4), (7) and (8) according to the present invention is bonded to the CTGF-specific siRNA by a simple covalent bond or a linker-mediated covalent bond (X or Y). In addition, the linker mediating the covalent bond is covalently bonded to the hydrophilic material or the hydrophobic material at the end of the CTGF-specific siRNA, and is not particularly limited as long as the bond that is possible to be decomposed in a specific environment is provided as needed. Therefore, any compound for the binding to activate the CTGF-specific siRNA and/or the hydrophilic material (or the hydrophobic material) in production of the double-helical oligo RNA. structure according to the present invention, may be used as the linker. The covalent bond may be any one of a non-degradable bond or a degradable bond. Here, examples of the non-degradable bond may include an amide bond or a phosphorylation bond, and examples of the degradable bond may include a disulfide bond, an acid degradable bond, an ester bond, an anhydride bond, a biodegradable bond or an enzymatically degradable bond, and the like, but the present invention is not limited thereto.

In addition, any CTGF-specific siRNA represented by R (or S and AS) in Structural Formulas (1) to (4), (7), and (8) is usable without limitation as long as it is siRNA capable of being specifically bonded to CTGF, Cyr61 or Plekhol. Preferably, the CTGF-specific siRNA consists of a sense strand including any one sequence selected from SEQ ID NOs: 1 to 600 and 602 to 604 and an antisense strand including a complementary sequence thereto.

In particular, the siRNA included in Structural Formulas (1) to (4), (7) and (8) according to the present invention is preferably CTGF-specific siRNA including any one sequence selected from the group consisting of SEQ ID NOs: 1 to 10, 35, 42, 59, 602 to 604 or 301 to 303, 305 to 307, 309, 317, 323 and 329, as a sense strand,

More preferably, the siRNA according to the present invention is CTGF-specific siRNA including any one sequence selected from the group consisting of SEQ ID NOs: 4, 5, 8, 9, 35, 42, 59, 602, 603, 604, 301, 303, 307 and 323, as a sense strand,
Most preferably, the siRNA according to the present invention is CTGF-specific siRNA including any one sequence of SEQ ID NO: 42, 59, 602 or 323, as a sense strand.

In addition, siRNA including human and mouse CTGF-specific siRNA sense strand according to SEQ ID NO: 6 or 8 are particularly preferred, which is because the siRNA having the sequence as the sense strand has an effect of simultaneously inhibiting expression of human and mouse CTGF, such that siRNA including human and mouse CTGF-specific siRNA sense strand according to SEQ ID NO: 6 is the most preferred.

In the double helical-oligo RNA structure containing the CTGF-specific siRNA according to the present invention, an amine group or a polyhistidine group may be additionally introduced into a portion of the distal end bonded with the siRNA of the hydrophilic material in the structure.

This makes it easy to perform intercellular introduction and escape from the endosome of the double helical-oligo RNA structure containing the CTGF-specific siRNA according to the present invention, and a possibility of introducing an amine group and using a polyhistidine group to easily perform the intercellular introduction and the escape from the endosome of the carriers such as Quantum dot, Dendrimer, liposome, etc., and the effect thereof, have been reported.

Specifically, it is known that the primary amine group expressed at the end or the outside of the carrier is protonated in vivo pH to form a conjugate with a negatively charged gene by an electrostatic interaction, and the escape from the endosome is easily performed due to internal tertiary amines having a buffer effect at a low pH of the endosome after being introduced into the cells, thereby being capable of protecting the carrier from decomposition of lysosome (gene delivery and expression inhibition using a polymer-based hybrid material. Polymer Sci. Technol., Vol. 23, No.3, pp254-259).

In addition, it is known that histidine which is one of non-essential amino acids, has an imidazole ring (pKa3 6.04) at a residue (-R) to increase a buffer capacity in the endosome and the lysosome, such that histidine expression may be used to increase an escape efficiency from the endosome in non-viral gene carriers including liposome (Novel histidine-conjugated galactosylated cationic liposomes for efficient hepatocyte selective gene transfer in human hepatoma HepG2 cells. J. Controlled Release 118, pp262-270).

The amine group or the polyhistidine group may be linked to the hydrophilic material or the hydrophilic material block through at least one linker.

When the amine group or the polyhistidine group is introduced into the hydrophilic material of the double-helical oligo RNA structure represented by Structural Formula (1), the double helical-oligo RNA structure may have a structure represented by Structural Formula (9) below:

Structural Formula 9 P-J₁-J₂-A-X-R-Y-B

In Structural Formula (9), A, B, R, X and Y are the same as being defined in Structural Formula (1),
P is an amine group or a polyhistidine group. J₁ and J₂ are linkers and may be each independently selected from a simple covalent bond, PO₃⁻, SO₃, CO₂, C₂₋₁₂ alkyl, alkenyl, and alkynyl, but the present invention is not limited thereto. It is obvious to a person skilled in the art that any linker is usable as J₁ and J₂ as long as it meets the objects of the present invention depending on the used hydrophilic material.

Preferably, when the amine group is introduced, J₂ is preferably a simple covalent bond or PO₃⁻, and J₁ is preferably C₆ alkyl, but the present invention is not limited thereto.

Further, when the polyhistidine group is introduced, in Structural Formula (9), J₂ is preferably a simple covalent bond or PO₃⁻, and J₁ is preferably the following Compound (4), but the present invention is not limited thereto.

### Compound 4

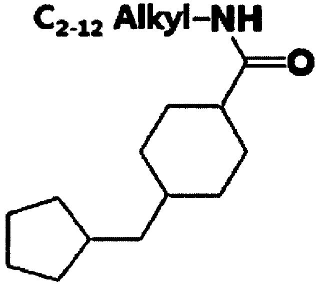

Further, when the hydrophilic material of the double-helical oligo RNA structure represented by Structural Formula (9) is a hydrophilic material block represented by Structural Formula (5) or (6), and the amine group or the polyhistidine group is introduced, the double-helical oligo RNA structure may be represented by Structural Formula (10) or (11):

Structural Formula 10 P-J₁-J₂-(A'ₘ-J)ₙ-X-R-Y-B

Structural Formula 11 P-J₁-J₂-(J-A'ₘ)ₙ-X-R-Y-B

In Structural Formulas (10) and (11), X, R, Y, B, A', J, m and n are the same as being defined in Structural Formula (5) or (6), and P, J1 and J2 are the same as being defined in Structural Formula (9).

In particular, in Structural Formulas (10) and (11), the hydrophilic material is preferably bonded to 3' end of the sense strand of the CTGF-specific siRNA, and in this case, Structural Formulas (9) to (11) may be represented by the following Structural Formulas (12) to (14):

Structural Formula 12 P-J₁-J₂-A-X-3' S 5'-Y-B AS

Structural Formula 13 P-J₁-J₂-(A'ₘ-J)ₙ-X-3' S 5'-Y-B AS

Structural Formula 14 P-J₁-J₂-(J-A'ₘ)ₙ-X-3' S 5'-Y-B AS

In Structural Formulas (12) to (14), X, R, Y, B, A, A' J, m, n, P, J₁ and J₂ are the same as being defined in Structural Formulas (9) to (11), and 5' and 3' mean 5' end and 3' end of the sense strand of the CTGF-specific siRNA.

The amine group that may be introduced in the present invention may be primary to tertiary amine groups, and the primary amine group is particularly preferred. The introduced amine group may be present as an amine salt. For example, the salt of the primary amine group may be present in a form of NH₃⁺.

Further, the polyhistidine group that may be introduced in the present invention preferably includes 3 to 10 histidines, more preferably, 5 to 8 histidines, and the most preferably, 6 histidines. In addition to histidine, at least one cystein may be additionally included.

Meanwhile, when a targeting moiety is provided in the double-helical oligo RNA structure containing the CTGF-specific siRNA according to the present invention and the nanoparticle formed therefrom, delivery to the target cell is effectively promoted to achieve delivery to the target cell even in a relatively low concentration of dosage, thereby showing a high control function for target gene expression, and thereby preventing non-specific delivery of the CTGF-specific siRNA into other organs and cells.

Accordingly, the present invention provides a double-helical oligo RNA structure in which a ligand (L), particularly, a ligand specifically bonded to a receptor that promotes target cell internalization through receptor-mediated endocytosis (RME), is additionally bonded to the structure according to Structural Formulas (1) to (4), (7) and (8). For example, the form in which the ligand is bonded to the double-helical oligo RNA structure according to Structural Formula (1), has a structure represented by the following Structural Formula (15):

Structural Formula 15 (Lᵢ-Z)-A-X-R-Y-B

In Structural Formula (15), A, B, X and Y are the same as being defined in Structural Formula (1), L is a ligand specifically bonded to a receptor that promotes target cell internalization through receptor-mediated endocytosis (RME), and i represents an integer from 1 to 5, preferably, an integer of 1 to 3.

The ligand in Structural Formula (15) may be preferably selected from the group consisting of a target receptor-specific antibody or aptamer, peptide that has properties of RME for promoting cell internalization in a target cell-specific manner; or folate (generally, folate and folic acid are intersectionally used, and the folate in the present invention means folate in a natural state or in an activated state in a human body), chemicals such as sugar, carbohydrate, etc., including hexoamines such as N-acetyl galactosamine (NAG), etc., glucose, mannose, but the present invention is not limited thereto.

Further, the hydrophilic material A in Structural Formula (15) may be used in the form of the hydrophilic material block represented by Structural Formulas (5) and (6) .

The present invention also provides a method for producing the double-helical oligo RNA structure containing the CTGF-specific siRNA.

The method for producing the double-helical oligo RNA structure containing the CTGF-specific siRNA according to the present invention may include the following steps:
(1) binding a hydrophilic material based on a solid support;
(2) synthesizing an RNA single strand based on the solid support containing the hydrophilic material bonded thereto;
(3) covalently binding a hydrophobic material to 5' end of the RNA single strand;
(4) synthesizing an RNA single strand having a complementary sequence to a sequence of the RNA single strand;
(5) separating and purifying an RNA-polymer structure and the RNA single strand from the solid support when the synthesizing of the RNA single strand is completed; and
(6) producing the double-helical oligo RNA structure by annealing the RNA-polymer structure and an RNA single strand having a complementary sequence thereto.

The solid support in the present invention is preferably a controlled pore glass (CPG), but the present invention is not limited thereto, but may be polystyrene, silica gel, cellulose paper, etc. In the CPG, a diameter is preferably 40 to 180 *µ*m, and a pore size is preferably 500 to 3000Å. After step (5) above, when the production is completed, it may be confirmed whether the purified RNA-polymer structure and the purified RNA single strand are produced as the desired RNA-polymer structure and the desired RNA single strand by measuring molecular weights by MALDI-TOF mass spectrometer. In the production method, Step (4) which is a step of synthesizing the RNA single strand having a complementary sequence to a sequence of the RNA single strand synthesized in Step (2) may be performed before Step (1) or may be performed during any one step of Steps (1) to (5).

In addition, the RNA single strand having a complementary sequence to the RNA single strand synthesized in Step (2) may contain a phosphate group bonded to 5' end thereof.

Meanwhile, the present invention provides a method for producing a double-helical oligo RNA structure in which a ligand is additionally bonded to the double-helical oligo RNA structure containing the CTGF-specific siRNA.

The method for producing the double-helical oligo RNA structure containing the ligand-bonded CTGF-specific siRNA according to the present invention may include the following steps:
(1) binding a hydrophilic material to a solid support containing a functional group bonded thereto;
(2) synthesizing an RNA single strand based on the solid support containing a functional group-hydrophilic material bonded thereto;
(3) covalently binding a hydrophilic material to 5' end of the RNA single strand;
(4) synthesizing an RNA single strand having a complementary sequence to a sequence of the RNA single strand;
(5) separating a functional group-RNA-polymer structure and the RNA single strand having the complementary sequence from the solid support when the synthesizing of the RNA single strand is completed;
(6) producing a ligand-RNA-polymer structure single strand by binding a ligand to an end of the hydrophilic material using the functional group; and
(7) producing a ligand-double-helical oligo RNA structure by annealing the ligand-RNA-polymer structure and an RNA single strand having a complementary sequence thereto.

After step (6) above, when the production is completed, whether the desired ligand-double-helical oligo RNA structure and the desired RNA single strand having a complementary sequence thereto are prepared may be confirmed by separating and purifying the ligand-RNA-polymer structure and the RNA single strand having a complementary sequence thereto, and measuring molecular weights by MALDI-TOF mass spectrometer. The ligand-double-helical oligo RNA structure may be produced by annealing the prepared ligand-RNA-polymer structure and the RNA single strand having a complementary sequence thereto. In the production method, Step (4) which is a step of synthesizing the RNA single strand having a complementary sequence to a sequence of the RNA single strand synthesized in Step (3), is an independent synthesis process, and may be performed before Step (1) or may be performed during any one step of Steps (1) to (6).

The present invention also provides a nanoparticle including the double-helical oligo RNA structure containing the CTGF-specific siRNA.

As described above, the double-helical oligo RNA structure containing the CTGF-specific siRNA is amphipathic structure containing both of hydrophobic materials and hydrophilic materials, wherein the hydrophilic materials have affinity through an interaction such as a hydrogen bond, etc., with water molecules present in the body to face toward the outside, and the hydrophobic materials face toward the inside through a hydrophobic interaction therebetween, thereby forming a thermodynamically stable nanoparticle. That is, the hydrophobic materials are positioned in the center of the nanoparticle, and the hydrophilic materials are positioned in the outside direction of the CTGF-specific siRNA to form nanoparticles protecting the CTGF-specific siRNA. These formed nanoparticles improve intracellular delivery of the CTGF, Cyr61, and/or Plekhol-specific siRNA and improve siRNA effect.

The nanoparticles according to the present invention may be formed of only the double-helical oligo RNA structure containing siRNAs each having the same sequence as each other, or may be formed of the double-helical oligo RNA structure containing siRNAs each having different sequence, wherein it is construed in the present invention that the siRNAs each having different sequence includes siRNA having different target genes, for example, CTGF-specific siRNA, or siRNA having the same target gene-specificity, but having different sequence.

Further, the double-helical oligo RNA structure containing other respiratory diseases-related gene-specific siRNA in addition to the CTGF-specific siRNA may also be included in the nanoparticle according to the present invention.

Further, the present invention provides a composition for preventing or treating respiratory diseases, particularly, idiopathic pulmonary fibrosis and COPD, including: the CTGF-specific siRNA, the double-helical oligo RNA structure containing the siRNA, and/or the nanoparticle formed of the double-helical oligo RNA structure.

The composition including the CTGF-specific siRNA according to the present invention, the double-helical oligo RNA structure containing the siRNA, and/or the nanoparticle formed of the double-helical oligo RNA structure as effective components, inhibits pulmonary artery remodeling and airway remodeling, such that the CTGF-specific siRNA or the composition containing the siRNA has an effect of preventing or treating the respiratory diseases.

In particular, the composition for preventing or treating respiratory diseases, including the double-helical oligo RNA structure according to the present invention may include:
a double-helical oligo RNA structure including a CTGF-specific siRNA that includes a sense strand including any one sequence selected from the group consisting of SEQ ID NOs: 1 to 100 or 602 to 604 and 301 to 400, preferably, any one sequence selected from the group consisting of SEQ ID NOs: 1 to 10, 35, 42, 59, 602 to 604, 301 to 303, 305 to 307, 309, 317, 323 and 329, more preferably, any one sequence selected from the group consisting of SEQ ID NOs: 4, 5, 8, 9, 35, 42, 59, 602 to 604, 301, 303, 307 and 323, the most preferably, any one sequence of SEQ ID NO: 42, 59, 602 or 323, and an antisense strand including a complementary sequence thereto;
In addition, the composition for preventing or treating respiratory diseases, including the double-helical oligo RNA structure according to the present invention may include: a double-helical oligo RNA structure including a CTGF-specific siRNA that includes a sense strand of human and mouse CTGF-specific siRNA according to sequence of SEQ ID NO: 6 or 8, preferably, SEQ ID NO: 6 and an antisense strand including a complementary sequence thereto.

In addition, a double-helical oligo RNA structure containing the CTGF-specific siRNA may be included in the composition, and additionally, siRNA specific to the other respiratory diseases-related gene in addition to CTGF or a double-helical oligo RNA structure containing the other respiratory diseases-related gene-specific siRNA may also be included in the composition according to the present invention.

At the time of using a composition for preventing or treating respiratory diseases additionally including the double-helical oligo RNA structure containing the other respiratory diseases-related gene-specific siRNA together with the CTGF-specific siRNA, or the double-helical oligo RNA structure containing the CTGF-specific siRNA, a synergistic effect like that of a combination therapy may be obtained.

Examples of the respiratory diseases capable of being prevented or treated by the composition of the present invention include idiopathic pulmonary fibrosis, asthma, chronic obstructive pulmonary disease (COPD), acute or chronic bronchitis, allergic rhinitis, cough and phlegm, acute lower respiratory tract infection, bronchitis, and bronchiolitis, acute upper respiratory tract infection, pharyngitis, tonsillitis, laryngitis, etc., but the present invention is not limited thereto.

Further, the nanoparticle included in the composition for preventing or treating the respiratory disease, including the nanoparticle formed of the double-helical oligo RNA structure according to the present invention may purely consist of only any one structure selected from the double-helical oligo RNA structure containing the CTGF, Cyr61, or Plekhol-specific siRNA, or may be configured in a form in which two or more kinds of the double-helical oligo RNA structures including the CTGF-specific siRNA are mixed with each other.

The composition of the present invention may be prepared by additionally including at least one pharmaceutically acceptable carrier in addition to the effective components. The pharmaceutically acceptable carrier is required to be compatible with the effective components of the present invention, and may be used by mixing one or more components selected from saline, sterile water, Ringer's solution, buffered saline, dextrose solution, maltodextrin solution, glycerol and ethanol, and other conventional additives such as antioxidant, buffer, fungistat, and the like, may be added thereto as needed. In addition, the composition may be prepared as a formulation for injection, such as an aqueous solution, suspension, emulsion, and the like, by additionally adding diluent, dispersant, surfactant, binder and lubricant thereto. In particular, it is preferred to provide the composition prepared as a lyophilized formulation. To prepare the lyophilized formulation, any method which is generally known in the technical field of the present invention may be used, wherein a stabilizer for lyophlization may be added thereto. In addition, appropriate methods in the art or a method disclosed in Remington's pharmaceutical Science, Mack Publishing Company, or Easton PA may be preferably used for formulation depending on each disease or component.

The dosage and administration method of the effective components, etc, included in the pharmaceutical composition of the present invention may be determined based on symptoms of the general patient and severity of the disease by general experts in the art. In addition, the composition may be formulated with various types such as powder, tablet, capsule, solution, injection, ointment, syrup, and the like, and may be provided as a unit-dose or a multi-dose container, for example, a sealed ampoule, bottle, and the like.

The pharmaceutical composition of the present invention may be orally or parenterally administered. Examples of an administration route of the pharmaceutical composition according to the present invention may include oral, intravenous, intramuscular, intra-arterial, intramedullary, intradural, intracardiac, transdermal, subcutaneous, intraperitoneal, intestinal, sublingual or topical administration, but the present invention is not limited thereto. Particularly, the administration route may also include administration into lung via drip infusion in respiratory organs for treatment of respiratory diseases. The administration amount of the composition may have various ranges depending on weight, age, gender, health condition, diet, administration time, method, excretion rate, the severity of disease, and the like, of a patient, and may be easily determined by a general expert in the art. In addition, the composition of the present invention may be formulated into an appropriate dosage form by using known technologies for clinical administration.

Further, the present invention provides a method for preventing or treating respiratory diseases, particularly, idiopathic pulmonary fibrosis and COPD, including: administrating the double-helical oligo RNA structure according to the present invention and the nanoparticle including the double-helical oligo RNA structure to a patient requiring such treatment.

### EXAMPLES

Hereinafter, the present invention will be described in detail with reference to the following Examples. These examples are only for exemplifying the present invention, and it will be obvious to those skilled in the art that the scope of the present invention is not construed to be limited to these examples.

### Example 1. Design of target sequence of CTGF, Cyr61 or Plekhol and production of siRNA

604 types of target sequences (sense strands) capable of being bonded to mRNA sequence of CTGF(*Homo sapiens*) gene (NM_001901), mRNA sequence of Cyr61(*Homo sapiens*) gene (NM_001554), mRNA sequence of Plekhol(*Homo sapiens*) gene (NM_016274), mRNA sequence of CTGF(*Mus musculus*) gene (NM_010217), mRNA sequence of Cyr61(*Mus musculus*) gene (NM_010516), or mRNA sequence of Plekho1(Mus musculus) gene (NM_023320) were designed, and siRNAs of antisense strands having complementary sequences to the target sequences were produced.

First, a gene design program (Turbo si-Designer) developed by Bioneer Co., was used to design target sequence that the siRNA is capable of being bonded from mRNA sequences of the corresponding genes. The siRNA for respiratory disease-related genes according to the present invention has a double stranded structure including a sense strand consisting of 19 nucleotides and an antisense strand complementary thereto. Further, siCONT (having sequence of SEQ ID NO: 601 as a sense strand) which is siRNA having a sequence in which expression of any gene is not inhibited, was produced. The siRNA was produced by linking phosphodiester bonds forming an RNA backbone structure by using β-cyanoethyl phosphoramidite (Nucleic Acids Research, 12:4539-4557, 1984). Specifically, a reaction product including RNA having a desired length was obtained by repeating a series of processes of deblocking, coupling, oxidation and capping, on a solid support to which nucleotide is attached, using RNA synthesizer (384 Synthesizer, BIONEER, Korea). RNA of the reaction product was separated and purified by HPLC LC918(Japan Analytical Industry, Japan) equipped with Daisogel C₁₈ (Daiso, Japan) column, and it was confirmed whether or not the purified RNA meets the target sequence by MALDI-TOF mass spectrometer (Shimadzu, Japan). Then, the desired double-stranded siRNA (SEQ ID NOs: 1 to 604) was produced by binding the RNA sense strand to the RNA antisense strand.

### Example 2. Production of double-helical oligo RNA structure (PEG-SAMiRNA).

The double-helical oligo RNA structure (PEG-SAMiRNA) produced in the present invention has a structure represented by the following Structural Formula (16):

Structural Formula 16 C₂₄-5'-S-3'-PEG AS

In Structural Formula (16), S is a sense strand of siRNA; AS is an antisense strand of siRNA; PEG is a hydrophilic material, that is, polyethylene glycol; C₂₄ is a hydrophobic material and tetradocosane including a disulfide bond; and 5' and 3' mean directions of the double-helical oligo RNA end.

The sense strand of siRNA of Structural Formula (16) was produced by synthesizing a double-helical oligo RNA-hydrophilic material structure of a sense strand in which polyethylene glycol is bonded to 3' end by the above-described method in which phosphodiester bonds forming an RNA backbone structure are linked by using β-cyanoethyl phosphoramidite, based on 3' polyethylene glycol (PEG, Mn=2,000)-CPG produced by Example 1 of Korean Patent Laid-Open Publication No. 10-2012-0119212, as a supporter, and binding tetradocosane including a disulfide bond to 5' end, thereby producing a sense strand of a desired RNA-polymer structure. For an antisense strand in which annealing is performed with the sense strand, the antisense strand having a complementary sequence to the sense strand was produced by the above-described reaction.

When the synthesis was completed, the RNA single strand and the RNA-polymer structure synthesized by treating 28%(v/v) ammonia in water bath at 60°C were separated from CPG and protecting moieties were removed therefrom by a deprotection reaction, respectively. The RNA single strand and the RNA-polymer structure from which the protecting moieties were removed were treated with N-methylpyrrolidone, triethylamine and triethylaminetrihydrofluoride at a volume ratio of 10:3:4 in an oven at 70°C, to remove 2' TBDMS(tert-butyldimethylsilyl).

RNA of the reaction product was separated and purified by HPLC LC918 (Japan Analytical Industry, Japan) equipped with Daisogel C18(Daiso, Japan) column, and it was confirmed whether or not the purified RNA meets the target sequence by MALDI-TOF mass spectrometer (Shimadzu, Japan). Then, to produce each double-helical oligo RNA structure, the sense strand and the antisense strand each having the same amount were mixed with each other, put in 1X annealing buffer (30 mM HEPES, 100 mM potassium acetate, 2 mM magnesium acetate, pH 7.0 ∼7.5), and reacted in a constant-temperature water bath at 90°C for 3 minutes, and reacted again at 37°C, thereby producing each double-helical oligo RNA structure including siRNA having sequence of SEQ ID NO: 42, 59, 602, 124, 153, 187, 197, 212, 218, 221, 223, 323, 424, 525 or 601 as the sense strand (hereinafter, referred to as SAMiRNALP-hCTGF, SAMiRNALP-hCyr, SAMiRNALP-hPlek, SAMiRNALP-mCTGF, SAMiRNALP-mCyr, and SAMiRNALP-mPlek SAMiRNALP-CONT, respectively). It was confirmed that the produced double-helical oligo RNA structure was annealed by electrophoresis.

### Example 3. Production of improved double-helical oligo RNA structure (Mono-HEG-SAMiRNA).

The improved double-helical oligo RNA structure produced in the present invention is obtained by using [PO₃⁻-hexaethylene glycol]₄ (hereinafter, referred to as 'Mono-HEG-SAMiRNA', see Structural Formula (17)) which is the hydrophilic material block instead of using PEG which is the hydrophilic material, and has the following Structure Formula (17):

Structure Formula 17 **C₂₄-5'-S-3'-[(Hexa Ethylene Glycol)-PO₃⁻]₄ AS**

In Structural Formula (17), S is a sense strand of siRNA; AS is an antisense strand of siRNA; [Hexa Ethylene Glycol]₄ is a hydrophilic material monomer; C₂₄ is a hydrophobic material and tetradocosane including a disulfide bond; and 5 'and 3' mean directions of the double-helical oligo RNA sense strand end.

The structure of Mono-HEG SAMiRNA according to Structural Formula (17) may be represented by the following Structural Formula (18):

### Structural Formula 18

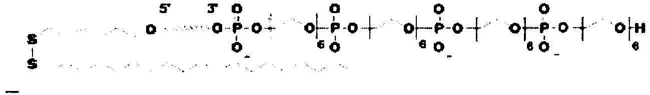

RNA of the reaction product was separated and purified by HPLC LC918 (Japan Analytical Industry, Japan) equipped with Daisogel C₁₈(Daiso, Japan) column, and it was confirmed whether or not the purified RNA meets the target sequence by MALDI-TOF mass spectrometer (Shimadzu, Japan). Then, to produce each double-helical oligo RNA structure, the sense strand and the antisense strand each having the same amount were mixed with each other, put in 1X annealing buffer (30 mM HEPES, 100 mM potassium acetate, 2 mM magnesium acetate, pH 7.0 ∼7.5), and reacted in a constant-temperature water bath at 90°C for 3 minutes, and reacted again at 37°C, thereby producing each double-helical oligo RNA structure including siRNA having sequence of SEQ ID NO: 42, 59, 602, 124, 153, 187, 197, 212, 218, 221, 223, 323, 424, 525 or 601 as the sense strand (hereinafter, referred to as Mono-HEG-SAMiRNALP-hCTGF, Mono-HEG-SAMiRNALP-hCyr, Mono-HEG-SAMiRNALP-hPlek, Mono-HEG-SAMiRNALP-mCTGF, Mono-HEG-SAMiRNALP-mCyr, Mono-HEG-SAMiRNALP-mPlek, and Mono-HEG-SAMiRNALP-CONT, respectively). It was confirmed that the produced double-helical oligo RNA structure was annealed by electrophoresis.

### Example 4. Production of nanoparticles formed of improved double-helical oligo RNA structure (Mono-HEG-SAMiRNA) and measurement of size thereof

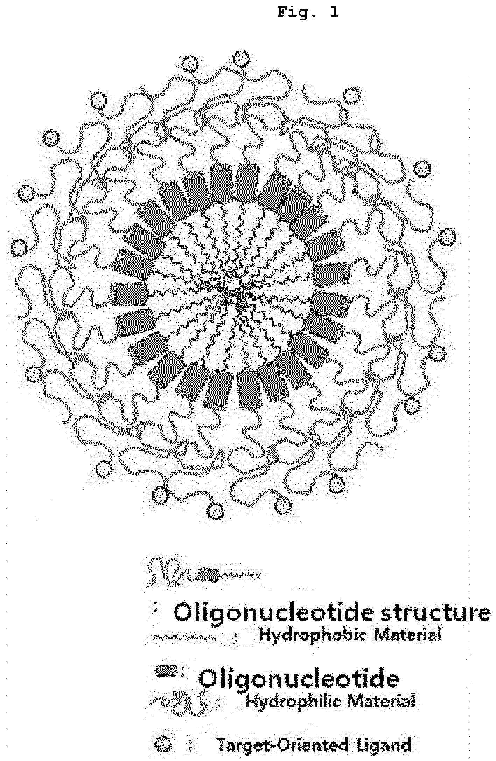

The double-helical oligo RNA structure (Mono-HEG-SAMiRNA) produced by Example 3 forms a nanoparticle, that is, micelle by a hydrophobic interaction between the hydrophobic materials bonded to the end of the double-helical oligo RNA (FIG. 1).

It was confirmed that the nanoparticle (SAMiRNA) formed of the corresponding Mono-HEG-SAMiRNA was formed by analyzing PDI (polydispersity index) of the nanoparticles formed of Mono-HEG-SAMiRNA-hCTGF, Mono-HEG-SAMiRNA-hCyr, Mono-HEG-SAMiRNA-hPlek, Mono-HEG-SAMiRNA-mCTGF, Mono-HEG-SAMiRNA-mCyr, Mono-HEG-SAMiRNA-mPlek and Mono-HEG-SAMiRNA-CONT.

### Example 4-1. Production of nanoparticle

The Mono-HEG-SAMiRNA-hCTGF was dissolved in 1.5 mℓ DPBS(Dulbecco's Phosphate Buffered Saline) at a concentration of 50 *µ*g/mℓ, the obtained mixture was freeze-dried at -75°C and 5mTorr condition for 48 hours to produce nanoparticle powder, and the nanoparticle powder was dissolved in DPBS which is a solvent to produce homogenized nanoparticles. The nanoparticles formed of Mono-HEG-SAMiRNA-hCyr, Mono-HEG-SAMiRNA-hPlek, Mono-HEG-SAMiRNA-mCTGF, Mono-HEG-SAMiRNA-mCyr, Mono-HEG-SAMiRNA-mPlek, and Mono-HEG-SAMiRNA-CONT were produced by the same method.

### Example 4-2. Measurement of size and polydispersity index (PDI) of nanoparticle

A size of the nanoparticle was measured by zeta-potential measurement. A size of the homogenized nanoparticles produced by Example 4-1 was measured by zeta-potential measurement (Nano-ZS, MALVERN, England), under conditions in which a refractive index to the material is 1.459, an absorption index is 0.001, a temperature of a solvent: DPBS is 25°C and the corresponding viscosity and refractive index are 1.0200 and 1.335, respectively. Once measurement was conducted by a size measurement including repeating 15 times and then repeating six times.

As the PDI value is decreased, the corresponding particles become uniformly distributed, and thus, it could be appreciated that the nanoparticles of the present invention have a significantly uniform size.

### Example 5. Confirmation of inhibition of target gene expression using target gene-specific siRNA for human in human fibroblast cell line (MRC-5).

Human fibroblast (MRC-5) which is a fibroblast cell line was transformed by siRNAs having sequences of SEQ ID NOs: 1 to 10, 101 to 110, 201 to 210 and 601 produced by Example 1 as the sense strand, and the expression aspect of the target gene was analyzed in the transformed fibroblast cell line (MRC-5).

### Example 5-1. Culture of human fibroblast cell line

A human fibroblast cell line (MRC-5) obtained from Korean Cell line bank (KCLB) was cultured in RPMI-1640 culture medium (GIBCO/Invitrogen, USA, 10% (v/v) fetal bovine serum, penicillin 100 units/ml and 100 *µ*g/mℓ of streptomycin) under condition of 37°C and 5%(v/v) CO₂.

### Example 5-2. Transfection of target siRNA into human fibroblast cell line

1.8×10⁵ fibroblast cell line (MRC-5) cultured by Example 5-1 was cultured in RPMI 1640 medium for 18 hours in 6-well plate under condition of 37°C and 5 %(v/v) CO₂, and the medium was removed, and 500*µ*ℓ of Opti-MEM medium (GIBCO, USA) for each well was dispensed.

Meanwhile, 3.5*µ*ℓ of Lipofectamine™ RNAi Max (Invitrogen, USA) was mixed with 246.5*µ*ℓ of Opti-MEM medium to prepare a mixed solution, and the mixed solution was reacted at room temperature for 5 minutes. siRNA solutions each having a final concentration of 5 or 20 nM were prepared by adding 5 or 20*µ*ℓ of siRNAs (1 pmole/*µ*ℓ) having sequences of SEQ ID NOs: 1 to 10, 101 to 110, 201 to 210 and 601 by Example 1, as the sense strand, to 230*µ*ℓ of Opti-MEM medium. The Lipofectamine™ RNAi Max mixture and the siRNA solution were mixed and reacted at room temperature for 15 minutes, thereby preparing a solution for transfection.

Then, 500*µ*ℓ of each transfection solution was dispensed in each well of the tumor cell line containing Opti-MEM dispensed therein, and cultured for 6 hours, and the Opti-MEM medium was removed. Here, 1 mℓ of RPMI 1640 culture medium was dispensed therein and cultured under condition of 37°C and 5%(v/v) CO2 for 24 hours.

### Example 5-3. Quantitative analysis of target gene mRNA

cDNA was produced by extracting total RNA from the transfected cell line by Example 5-2, and an mRNA expression amount of the target gene was subjected to relative-quantification by real-time PCR.

### Example 5-3-1. RNA separation from transfected cell and cDNA production

Total RNA was extracted from the transfected cell line of Example 5-2 by RNA extraction kit (AccuPrep Cell total RNA extraction kit, BIONEER, Korea), and the extracted RNA was used to produce cDNA by RNA reverse transcriptase (AccuPower CycleScript RT Premix/dT20, Bioneer, Korea) according to the following method. Specifically, 1 *µ*g of the extracted RNA per each tube was added to AccuPower CycleScript RT Premix/dT20(Bioneer, Korea) contained in 0.25mℓ Eppendorf tube, and distilled water treated with DEPC(diethyl pyrocarbonate) was added thereto so as to have a total volume of 20*µ*ℓ. A process of hybridizing RNA and primers at 30°C for 1 minute by a gene amplifier (MyGenie™ 96 Gradient Thermal Block, BIONEER, Korea) and a process of producing cDNA at 52°C for 4 minutes were repeated 6 times, and then, an amplification reaction was completed by inactivating the enzyme at 90°C for 5 minutes.

### Example 5-3-2. Relative-quantitative analysis of target gene mRNA

A relative amount of the respiratory disease-related gene mRNA was quantified by the following method through real-time PCR having the cDNA produced by Example 5-3-1 as a template. cDNA produced by Example 5-3-1 was diluted 5 times with distilled water in each well of 96-well plate. Then, 3*µ*ℓ of the diluted cDNA, 25*µ*ℓ of 2 × GreenStar™ PCR master mix (BIONEER, Korea), 19*µ*ℓ of distilled water, and 3*µ*ℓ of qPCR primers (Table 2; F and R each having 10 pmole/*µ*ℓ; BIONEER, Korea) were added thereto to prepare each mixed solution. Meanwhile, RPL13A (ribosomal protein L13a) which is a housekeeping gene (hereinafter, referred to as HK gene) was determined as a standard gene to normalize the expression amount of the target gene mRNA. The following reaction was performed on 96-well plate containing the mixture by Exicycler™ 96 Real-Time Quantitative Thermal Block (BIONEER, Korea). After the reaction at 95°C for 15 minutes to activate the enzyme and remove a secondary structure of cDNA, four processes including a process of denaturing at 94°C for 30 seconds, a process of annealing at 58°C for 30 seconds, a process of extension at 72°C for 30 seconds, and a process of SYBR green scan were repeated 42 times, and final extension was performed at 72°C for 3 minutes. Then, the temperature was maintained at 55°C for 1 minute, and a melting curve was analyzed from 55°C up to 95°C. After PCR was completed, for Ct (threshold cycle) value of each of the obtained target gene, Ct values of the target gene corrected through GAPDH gene were calculated, and then the difference (ΔCt) was obtained by using a test group treated with siRNA (SEQ ID NO: 601, siCONT) having a control sequence preventing inhibition of gene expression, as a control group.

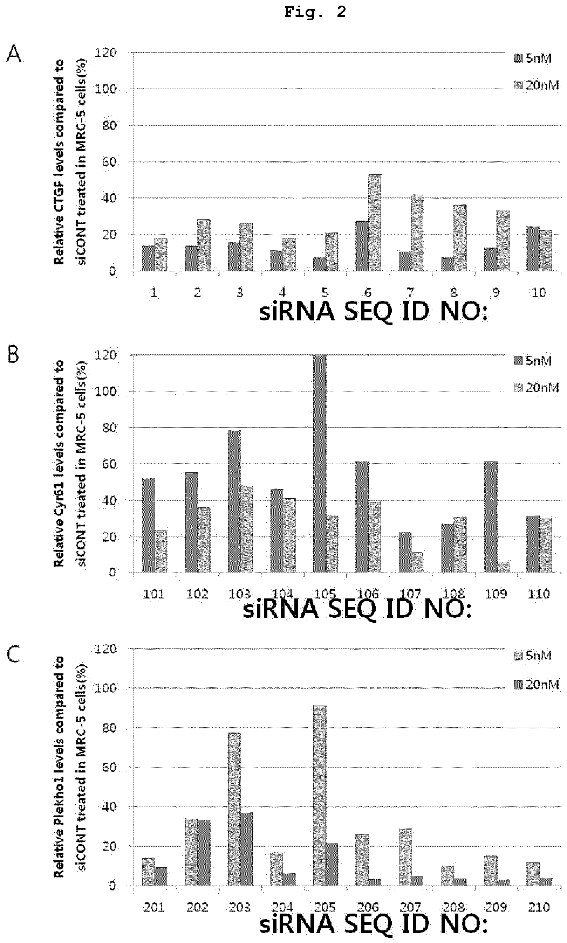

The expression amount of the target gene of the cell treated with the CTGF (Homo sapiens)-specific siRNA (having sequence of SEQ ID NOs: 1 to 10 as the sense strand) was relatively quantified by using the ΔCt value and calculation formula 2(-ΔCt)× 100 (FIG. 2A). Further, the expression amount of the target gene of the cell treated with the Cyr61(Homo sapiens)-specific siRNA (having sequence of SEQ ID NOs: 101 to 110 as the sense strand) was relatively quantified (FIG. 2B), and the expression amount of the target gene of the cell treated with the Plekho1(Homo sapiens)-specific siRNA (having sequence of SEQ ID NOs: 201 to 210 as the sense strand) was relatively quantified (FIG. 2C).

As a result, it could be confirmed that several kinds of siRNA of the present invention showed high inhibition level of target gene expression. In addition, in order to select siRNA with high efficiency, siRNAs having sequences of SEQ ID NOs: 1, 3, 4, 8, 9, 10, 102, 104, 105, 106, 107, 108, 109, 204, 206, 207, 208, 209 and 210 in which the mRNA expression amount for each gene at 5 nM concentration is significantly decreased, as the sense strand, were selected.

**Table 2 qPCR primer sequences**

| **(m, *Mus musculus* ; h, *Homo sapiens,* F, Forward prime; R-**reverse **primer)** | |
|---|---|
| Name | Sequences or product name |
| hCTGF-F | P199255(qPCR primer #, BIONEER) |
| hCTGF-R | |
| hAREG-F | ACACCTACTCTGGGAAGCGT |
| hAREG-R | GCCAGGTATTTGTGGTTCGT |
| hCYR61-F | AAAGGAAGCCTTGCTCATTC |
| hCYR61-R | TCAACTCCACAAGCTCCAAA |
| hPlekho1-F | P299777(qPCR primer #, BIONEER) |
| hPlekho1-R | |
| hRPL13A-F | AGCTCATGAGGCTACGGAAA |
| hRPL13A-R | CGTACATTCCAGGGCAACA |
| mCTGF-F | GGGCCTCTTCTGCGATTTC |
| mCTGF-R | ATCCAGGCAAGTGCATTGGTA |
| mAREG-F | GGTCTTAGGCTCAGGCCATTA |
| mAREG-R | CGCTTATGGTGGAAACCTCTC |
| mCYR61-F | CTGCGCTAAACAACTCAACGA |
| mCYR61-R | GCAGATCCCTTTCAGAGCGG |
| mPlekmol-F | AATTCTGCGGGAAAGGGATTT |
| mPlekmol-R | AACACCTCCTGACTGTTTTTCTC |
| mRPL13A-F | CCTGCTGCTCTCAAGGTTGTT |
| mRPL13A-R | CGATAGTGCATCTTGGCCTTT |

### Example 6. Selection of target gene-specific siRNA for human with high efficiency in human fibroblast cell line (MRC-5)

Human fibroblast cell line (MRC-5) was transformed by using siRNAs having sequences of SEQ ID NOs: 1, 3, 4, 8, 9, 10, 102, 104, 105, 106, 107, 108, 109, 204, 206, 207, 208, 209, 210 and 601 selected by Example 5-3-2, as the sense strand, and the expression aspect of the target gene was analyzed in the transformed fibroblast cell line (MRC-5) to select siRNA with high efficiency.

### Example 6-1. Culture of human fibroblast cell line

Human fibroblast cell line (MRC-5) obtained from Korean Cell line bank (KCLB, Korea) was cultured under the same condition as Example 5-1.

### Example 6-2. Transfection of target siRNA into human fibroblast cell line

1.8×10⁵ fibroblast cell line (MRC-5) cultured by Example 6-1 was cultured in RPMI 1640 medium for 18 hours in 6-well plate under condition of 37°C and 5 %(v/v) CO₂, and the medium was removed, and 500*µ*ℓ of Opti-MEM medium (GIBCO, USA) for each well was dispensed.

Meanwhile, 3.5*µ*ℓ of Lipofectamine™ RNAi Max (Invitrogen, USA) was mixed with 246.5*µ*ℓ of Opti-MEM medium to prepare a mixed solution, and the mixed solution was reacted at room temperature for 5 minutes. siRNA solutions each having a final concentration of 0.2 or 1 nM were prepared by adding 0.2 or 1*µ*ℓ of siRNAs (1 pmole/*µ*ℓ) having sequences of SEQ ID NOs: 1, 3, 4, 8, 9, 10, 102, 104, 105, 106, 107, 108, 109, 204, 206, 207, 208, 209, 210 and 601 by Example 1, as the sense strand, to 230*µ*ℓ of Opti-MEM medium. The Lipofectamine™ RNAi Max mixture and the siRNA solution were mixed and reacted at room temperature for 15 minutes, thereby preparing a solution for transfection.

Then, 500*µ*ℓ of each transfection solution was dispensed in each well of the tumor cell line containing Opti-MEM dispensed therein, and cultured for 6 hours, and the Opti-MEM medium was removed. Here, 1 mℓ of RPMI 1640 culture medium was dispensed therein and cultured under condition of 37°C and 5%(v/v) CO₂ for 24 hours.

### Example 6-3. Quantitative analysis of target gene mRNA

cDNA was produced by extracting total RNA from the transfected cell line by Example 6-2 through the same method as Example 4-3, and an mRNA expression amount of the target gene was subjected to relative-quantification by real-time PCR. The inhibition amount of the target gene expression according to low concentration siRNA treatment was observed to clearly confirm each siRNA efficacy, and it was confirmed that siRNAs having sequence of SEQ ID NOs: 8, 107 and 206 as the sense strand showed relatively high level of inhibition for target gene expression even at a significantly low concentration (FIG. 3).

### Example 7. Confirmation of inhibition of target gene expression using target gene-specific siRNA for human in human lung cancer cell line (A549).

Human lung cancer cell line (A549) which is a lung tumor cell line was transformed by siRNAs having sequences of SEQ ID NOs: 35, 42, 59, 602, 603, 604, 124, 153, 166, 187, 197, 212, 218, 221, 223 and 601 produced by Example 1 as the sense strand, and the expression aspect of the target gene was analyzed in the transformed lung cancer cell line (A549).

### Example 7-1. Culture of human lung cancer cell line

A human lung cancer cell line (A549) obtained from American Type Culture Collection (ATCC) was cultured in DMEM culture medium (GIBCO/Invitrogen, USA, 10% (v/v) fetal bovine serum, penicillin 100 units/ml and 100 *µ*g/mℓ of streptomycin) under condition of 37 °C and 5%(v/v) CO₂.

### Example 7-2. Transfection of target siRNA into human lung cancer cell line

1.2×10⁵ lung cancer cell line (A549) cultured by Example 7-1 was cultured in DMEM medium for 18 hours in 6-well plate under condition of 37°C and 5 % (v/v) CO₂, and the medium was removed, and 500*µ*ℓ of Opti-MEM medium (GIBCO, USA) for each well was dispensed.

Meanwhile, 3.5*µ*ℓ of Lipofectamine™ RNAi Max (Invitrogen, USA) was mixed with 246.5*µ*ℓ of Opti-MEM medium to prepare a mixed solution, and the mixed solution was reacted at room temperature for 5 minutes. siRNA solutions each having a final concentration of 5nM, 1nM, 0.5nM, 0.2nM or 0.04nM were prepared by adding 1*µ*ℓ of siRNAs (1 pmole/*µ*ℓ) having sequences of SEQ ID NOs: 35, 42, 59, 602, 603, 604, 124, 153, 166, 187, 197, 212, 218, 221 and 223 by Example 1, as the sense strand, to 230*µ*ℓ of Opti-MEM medium. The Lipofectamine™ RNAi Max mixture and the siRNA solution were mixed and reacted at room temperature for 15 minutes, thereby preparing a solution for transfection.

Then, 500*µ*ℓ of each transfection solution was dispensed in each well of the tumor cell line containing Opti-MEM dispensed therein, and cultured for 6 hours, and the Opti-MEM medium was removed. Here, 1 mℓ of RPMI 1640 culture medium was dispensed therein and cultured under condition of 37°C and 5%(v/v) CO₂ for 24 hours.

### Example 7-3. Quantitative analysis of target gene mRNA

cDNA was produced by extracting total RNA from the transfected cell line by Example 7-2, and an mRNA expression amount of the target gene was subjected to relative-quantification by real-time PCR.

### Example 7-3-1. RNA separation from transfected cell and cDNA production

Total RNA was extracted from the transfected cell line of Example 5-2 by RNA extraction kit (AccuPrep Cell total RNA extraction kit, BIONEER, Korea), and the extracted RNA was used to produce cDNA by RNA reverse transcriptase (AccuPower CycleScript RT Premix/dT20, Bioneer, Korea) according to the following method. Specifically, 1 *µ*g of the extracted RNA per each tube was added to AccuPower CycleScript RT Premix/dT20(Bioneer, Korea) contained in 0.25mℓ Eppendorf tube, and distilled water treated with DEPC (diethyl pyrocarbonate) was added thereto so as to have a total volume of 20*µ*ℓ. A process of hybridizing RNA and primers at 30°C for 1 minute by a gene amplifier (MyGenie™ 96 Gradient Thermal Block, BIONEER, Korea) and a process of producing cDNA at 52°C for 4 minutes were repeated 6 times, and then, an amplification reaction was completed by inactivating the enzyme at 90°C for 5 minutes.

### Example 7-3-2. Relative quantitative analysis of target gene mRNA

A relative amount of the respiratory disease-related gene mRNA was quantified by the following method through real-time PCR having the cDNA produced by Example 7-3-1 as a template. cDNA produced by Example 6-3-1 was diluted 5 times with distilled water in each well of 96-well plate. Then, 3*µ*ℓ of the diluted cDNA, 25*µ*ℓ of 2 × GreenStar™ PCR master mix (BIONEER, Korea), 19*µ*ℓ of distilled water, and 3*µ*ℓ of qPCR primers (Table 2; F and R each having 10 pmole/*µ*ℓ; BIONEER, Korea) were added thereto to prepare each mixed solution. Meanwhile, RPL13A (ribosomal protein L13a) which is a housekeeping gene (hereinafter, referred to as HK gene) was determined as a standard gene to normalize the expression amount of the target gene mRNA. The following reaction was performed on 96-well plate containing the mixture by Exicycler™ 96 Real-Time Quantitative Thermal Block (BIONEER, Korea). After the reaction at 95°C for 15 minutes to activate the enzyme and remove a secondary structure of cDNA, four processes including a process of denaturing at 94°C for 30 seconds, a process of annealing at 58°C for 30 seconds, a process of extension at 72°C for 30 seconds, and a process of SYBR green scan were repeated 42 times, and final extension was performed at 72°C for 3 minutes. Then, the temperature was maintained at 55°C for 1 minute, and a melting curve was analyzed from 55°C up to 95°C. After PCR was completed, for Ct (threshold cycle) value of each of the obtained target gene, Ct values of the target gene corrected through GAPDH gene were calculated, and then the difference (ΔCt) was obtained by using a test group treated with siRNA (SEQ ID NO: 601, siCONT) having a control sequence preventing inhibition of gene expression, as a control group.

The expression amount of the target gene of the cell treated with the CTGF (Homo sapiens)-specific siRNA (having sequence of SEQ ID NOs: 132, 42, 59, 602, 603, 604 as the sense strand) was relatively quantified by using the ΔCt value and calculation formula 2(^{-ΔCt})× 100 (FIG. 4A). Further, the expression amount of the target gene of the cell treated with the Cyr61(Homo sapiens)-specific siRNA (having sequence of SEQ ID NOs: 124, 153, 166, 187, and 197 as the sense strand) was relatively quantified (FIG. 4B), and the expression amount of the target gene of the cell treated with the Plekho1(Homo sapiens)-specific siRNA (having sequence of SEQ ID NOs: 212, 218, 221, and 223 as the sense strand) was relatively quantified (FIG. 4C).

As a result, it could be confirmed that several kinds of siRNA of the present invention showed high inhibition level of target gene expression. In addition, in order to select siRNA with high efficiency, siRNAs having sequences of SEQ ID NOs: 42, 59, 602, 124, 153, 187, 197, 212, 218, 221 and 223 in which the mRNA expression amount for each gene at 5 nM concentration is significantly decreased, as the sense strand, were selected.

### Example 8. Inhibition of target gene expression in human lung cancer cell line (A549) by nanoparticle (SAMiRNA) formed of double-helical oligo polymer structure

Human lung cancer cell line (A549) was transformed by using the nanoparticle formed of SAMiRNA LP including siRNA having sequences of SEQ ID NOs: 42, 59, 602, 124, 153, 187, 197, 212, 218, 221 and 223 selected by Example 7-3-2, as the sense strand, and the expression aspect of the target gene was analyzed in the transformed lung cancer cell line (A549).

### Example 8-1. Culture of human lung cancer cell line

Human lung cancer cell line (A549) obtained from American Type Culture Collection (ATCC) was cultured under the same condition as Example 7-1.

### Example 8-2. Transfection of target SAMiRNA into human lung cancer cell line

1.2×10⁵ lung cancer cell line (A549) cultured by Example 8-1 was cultured in RPMI 1640 medium for 18 hours in 12-well plate under condition of 37°C and 5 %(v/v) CO₂, and the medium was removed, and the same amount of Opti-MEM medium (GIBCO, USA) for each well was dispensed. 100*µ*ℓ of Opti-MEM medium and SAMiRNALP and monoSAMiRNALP produced by Example 4-2 were added to DPBS at a concentration of 50 *µ*g/mℓ, the obtained mixture was freeze-dried at -75°C and 5mTorr condition for 48 hours by the same method as Example 4-1 to produce homogenized nanoparticles. Then, each well of the tumor cell line in which the Opti-MEM is dispensed was treated with a transfection solution at a concentration of 200 nM, and cultured at 37°C and 5 %(v/v) CO₂ for the total of 48 hours.

### Example 8-3. Relative-quantitative analysis of target gene mRNA

cDNA was produced by extracting total RNA from the transfected cell line by Example 8-2 through the same method as Example 6-3, and an mRNA expression amount of the target gene was subjected to relative quantification by real-time PCR. The inhibition amount of the target gene expression according to low concentration siRNA treatment was observed to clearly confirm each siRNA efficacy, and it was confirmed that siRNAs having sequence of SEQ ID NOs: 42, 59 and 602 as the sense strand showed relatively high level of inhibition for target gene expression even at a significantly low concentration (FIG. 5).

### Example 9. Confirmation of inhibition of target gene expression using target gene-specific siRNA for mouse in mouse fibroblast cell line (NIH3T3).

Mouse fibroblast (NIH3T3) which is a fibroblast cell line was transformed by siRNAs having sequences of SEQ ID NOs: 301 to 330, 401 to 430, 501 to 530 and 601 produced by Example 1 as the sense strand, and an expression aspect of the target gene was analyzed in the transformed fibroblast cell line (NIH3T3).

### Example 9-1. Culture of mouse fibroblast cell line

A mouse fibroblast cell line (NIH3T3) obtained from American Type Culture Collection (ATCC) was cultured in RPMI-1640 culture medium (GIBCO/Invitrogen, USA, 10% (v/v) fetal bovine serum, penicillin 100 units/ml and 100 *µ*g/mℓ of streptomycin) under condition of 37 °C and 5%(v/v) CO₂.

### Example 9-2. Transfection of target siRNA into mouse fibroblast cell line

1×10⁵ fibroblast cell line (NIH3T3) cultured by Example 9-1 was cultured in RPMI 1640 medium for 18 hours in 12-well plate under condition of 37°C and 5 %(v/v) CO₂, and the medium was removed, and 500*µ*ℓ of Opti-MEM medium (GIBCO, USA) for each well was dispensed.

Meanwhile, 1.5*µ*ℓ of Lipofectamine™ RNAi Max (Invitrogen, USA) was mixed with 248.5*µ*ℓ of Opti-MEM medium to prepare a mixed solution, and the mixed solution was reacted at room temperature for 5 minutes. 5 or 20*µ*ℓ of siRNAs (1 pmole/*µ*ℓ) having sequences of SEQ ID NOs: 301 to 330, 401 to 430, 501 to 530 and 601 produced by Example 1, as the sense strand, were added to 230*µ*ℓ of Opti-MEM medium, thereby preparing siRNA solutions each having a final concentration of 5 or 20 nM. The Lipofectamine™ RNAi Max mixture and the siRNA solution were mixed and reacted at room temperature for 20 minutes, thereby preparing a solution for transfection.

Then, 500*µ*ℓ of each transfection solution was dispensed in each well of the tumor cell line containing Opti-MEM dispensed therein, and cultured for 6 hours, and the Opti-MEM medium was removed. Here, 1 mℓ of RPMI 1640 culture medium was dispensed therein and cultured under condition of 37°C and 5%(v/v) CO₂ for 24 hours.

### Example 9-3. Quantitative analysis of target gene mRNA

cDNA was produced by extracting total RNA from the transfected cell line by Example 9-2 through the same method as Example 5-3, and an mRNA expression amount of the target gene was subjected to relative quantification by real-time PCR.

The expression amount of the target gene of the cell treated with the CTGF (Mus musculus)-specific siRNA (having sequence of SEQ ID NOs: 301 to 330 as the sense strand) was relatively quantified (FIG. 6A). Further, the expression amount of the target gene of the cell treated with the Cyr61(Mus musculus)-specific siRNA (having sequence of SEQ ID NOs: 401 to 430 as the sense strand) was relatively quantified (FIG. 6B), and the expression amount of the target gene of the cell treated with the Plekho1(Mus musculus)-specific siRNA (having sequence of SEQ ID NOs: 501 to 530 as the sense strand) was relatively quantified (FIG. 6C).

As a result, it could be confirmed that several kinds of siRNA of the present invention showed high inhibition level of target gene expression. In addition, in order to select siRNA with high efficiency, siRNA having sequence of SEQ ID NO: 301, 302, 303, 305, 306, 307, 309, 317, 323 or 329 in which the mRNA expression amount for CTGF(Mus musculus) at 20 nM concentration is significantly decreased, as the sense strand, was selected, siRNA having sequence of SEQ ID NO: 409, 410, 415, 417, 418, 420, 422, 424, 427 or 429 in which the mRNA expression amount for Cyr61(Mus musculus) at 20 nM concentration is significantly decreased, as the sense strand, was selected, and siRNA having sequence of SEQ ID NO: 504, 505, 506, 507, 514, 515, 522, 523, 524 or 525 in which the mRNA expression amount for Plekhol(Mus musculus) at 20 nM concentration is significantly decreased, as the sense strand, was selected.

Further, in order to select siRNA with more preferable efficiency, siRNA having sequence of SEQ ID NO: 301, 303, 307 or 323 in which the mRNA expression amount for CTGF(Mus musculus) at 5 nM concentration is significantly decreased, as the sense strand, was selected, siRNA having sequence of SEQ ID NO: 410, 422, or 424 in which the mRNA expression amount for Cyr61(Mus musculus) at 5 nM concentration is significantly decreased, as the sense strand, was selected, and siRNA having sequence of SEQ ID NO: 507, 515, or 525 in which the mRNA expression amount for Plekho1(Mus musculus) at 5 nM concentration is significantly decreased, as the sense strand, was selected (FIG. 7).

### Example 10. Selection of target gene-specific siRNA for mouse with high efficiency in mouse fibroblast cell line (NIH3T3)

The expression aspect of the target gene was analyzed in the mouse fibroblast cell line (NIH3T3) using siRNAs having sequences of SEQ ID NOs: 301, 303, 307, 323, 410, 422, 424, 507, 515, 525 and 601 selected by Example 9-3, as the sense strand, to select siRNA with high efficiency.

### Example 10-1. Culture of mouse fibroblast cell line

Mouse fibroblast cell line (NIH3T3) obtained from American Type Culture Collection (ATCC) was cultured under the same condition as Example 9-1.

### Example 10-2. Transfection of target siRNA into mouse fibroblast cell line

1×10⁵ fibroblast cell line (NIH3T3) cultured by Example 10-1 was cultured in RPMI 1640 medium for 18 hours in 12-well plate under condition of 37°C and 5 %(v/v) CO₂, and the medium was removed, and 500*µ*ℓ of Opti-MEM medium (GIBCO, USA) for each well was dispensed.

Meanwhile, 1.5*µ*ℓ of Lipofectamine™ RNAi Max (Invitrogen, USA) was mixed with 248.5*µ*ℓ of Opti-MEM medium to prepare a mixed solution, and the mixed solution was reacted at room temperature for 5 minutes. 0.2, 1 or 5*µ*ℓ of siRNAs (1 pmole/*µ*ℓ) having sequences of SEQ ID NOs: 301, 303, 307, 323, 410, 422, 424, 507, 515, 525 and 601 produced by Example 1, as the sense strand, were added to 230*µ*ℓ of Opti-MEM medium, thereby preparing siRNA solutions each having a final concentration of 0.2, 1 or 5 nM. The Lipofectamine™ RNAi Max mixture and the siRNA solution were mixed and reacted at room temperature for 20 minutes, thereby preparing a solution for transfection.

Then, 500*µ*ℓ of each transfection solution was dispensed in each well of the tumor cell line containing Opti-MEM dispensed therein, and cultured for 6 hours, and the Opti-MEM medium was removed. Here, 1 mℓ of RPMI 1640 culture medium was dispensed therein and cultured under condition of 37°C and 5%(v/v) CO₂ for 24 hours.

### Example 10-3. Quantitative analysis of target gene mRNA

cDNA was produced by extracting total RNA from the transfected cell line by Example 10-2 through the same method as Example 5-3, and an mRNA expression amount of the target gene was subjected to relative quantification by real-time PCR. The expression amount of the target gene of the cell treated with the CTGF (Mus musculus)-specific siRNA (having sequence of SEQ ID NOs: 301, 303, 307, and 323 as the sense strand) was relatively quantified (FIG. 8A). Further, the expression amount of the target gene of the cell treated with the Cyr61 (Mus musculus)-specific siRNA (having sequence of SEQ ID NOs: 410, 422 and 424, as the sense strand) was relatively quantified (FIG. 8B), and the expression amount of the target gene of the cell treated with the Plekho1 (Mus musculus)-specific siRNA (having sequence of SEQ ID NOs: 507, 515, and 525 as the sense strand) was relatively quantified (FIG. 8C).

As a result, it was confirmed that each target gene-specific siRNA inhibits the expression of the target gene in a concentration-dependent manner, and it was confirmed that siRNAs having sequences of SEQ ID NOs: 307, 424 and 525 as the sense strand showed relatively high level of inhibition for target gene expression even at a significantly low concentration, to select siRNA with high efficiency.

### Example 11. Confirmation of inhibition of target gene expression using target gene-specific siRNA for human in mouse fibroblast cell line (NIH3T3).

Since biopharmaceuticals have species-specific action sites such as protein structure or gene sequence, identity of treatment drug is significantly important for securing efficiency in developing biopharmaceutical novel drug. Gene sequence homology between the target gene-specific siRNA for human and the target gene-specific siRNA for mouse designed in Example 1 was analyzed to select siRNA sequences that may confirm the inhibition effect of the target gene expression in the mouse fibroblast cell.

The selected siRNA sequences are siRNAs having sequences of SEQ ID NOs: 4, 5, 6, 8, 9, 102, 104, 105, 107, 108, 109, 202, 204, 206, 207, 208 and 209, as the sense strand, which are the target gene-specific siRNAs for human produced by Example 1, siRNAs having sequences of SEQ ID NOs: 307, 424 and 525, as the sense strand, which are the target gene-specific siRNAs for mouse, and siRNA having sequence of SEQ ID NO: 601, as the sense strand, which is a control group. The expression aspect of the target gene was analyzed in the mouse fibroblast cell line (NIH3T3) using these selected siRNAs, and efficacy thereof was confirmed in the mouse cell of siRNA designed based on the human gene.

### Example 11-1. Culture of mouse fibroblast cell line

Mouse fibroblast cell line (NIH3T3) obtained from American Type Culture Collection (ATCC) was cultured under the same condition as Example 9-1.

### Example 11-2. Transfection of target siRNA into mouse fibroblast cell line

1.8×10⁵ fibroblast cell line (NIH3T3) cultured by Example 11-1 was cultured in RPMI 1640 medium for 18 hours in 6-well plate under condition of 37°C and 5 %(v/v) CO₂, and the medium was removed, and 500*µ*ℓ of Opti-MEM medium (GIBCO, USA) for each well was dispensed.

Meanwhile, 3.5*µ*ℓ of Lipofectamine™ RNAi Max (Invitrogen, USA) was mixed with 246.5*µ*ℓ of Opti-MEM medium to prepare a mixed solution, and the mixed solution was reacted at room temperature for 5 minutes. siRNA solutions each having a final concentration of 5 or 20 nM were prepared by adding 5 or 20*µ*ℓ of siRNAs (1 pmole/*µ*ℓ) having sequences of SEQ ID NOs: 4, 5, 6, 8, 9, 102, 104, 105, 107, 108, 109, 202, 204, 206, 207, 208, 209, 307, 424, 525 and 601 produced by Example 1, as the sense strand, to 230*µ*ℓ of Opti-MEM medium. The Lipofectamine™ RNAi Max mixture and the siRNA solution were mixed and reacted at room temperature for 15 minutes, thereby preparing a solution for transfection.

Then, 500*µ*ℓ of each transfection solution was dispensed in each well of the tumor cell line containing Opti-MEM dispensed therein, and cultured for 6 hours, and the Opti-MEM medium was removed. Here, 1 mℓ of RPMI 1640 culture medium was dispensed therein and cultured under condition of 37°C and 5%(v/v) CO₂ for 24 hours.

### Example 11-3. Quantitative analysis of target gene mRNA

cDNA was produced by extracting total RNA from the transfected cell line by Example 11-2 through the same method as Example 5-3, and an mRNA expression amount of the target gene was subjected to relative quantification by real-time PCR. The expression amount of the target gene of the cell treated with the CTGF (Mus musculus)-specific siRNA (SEQ ID NO: 307) or treated with the CTGF (Homo sapiens)-specific siRNA (SEQ ID NO: 4, 5, 6, 8 or 9) was relatively quantified (FIG. 9A). The expression amount of the target gene of the cell treated with Cyr61(Mus musculus)-specific siRNA (having a sequence of SEQ ID NO: 424 as the sense strand) or treated with Cyr61(Homo sapiens)-specific siRNA (having a sequence of SEQ ID NO: 102, 104, 105, 107, 108 or 109 as the sense strand) was relatively quantified (FIG. 9B), and the expression amount of the target gene of the cell treated with Plekho1 (Mus musculus)-specific siRNA (having a sequence of SEQ ID NO: 525 as the sense strand) or treated with Plekho1(Homo sapiens)-specific siRNA (having a sequence of SEQ ID NO: 202, 204, 206, 207, 208 or 209 as the sense strand) was relatively quantified (FIG. 9C).

As a result, it was confirmed that each target gene-specific siRNA for human inhibits the expression of the target gene according to sequence homology, and it was confirmed that siRNAs having sequences of SEQ ID NOs: 6, 8, 102, 104, 105, 204, 207 and 208 as the sense strand showed relatively high level of inhibition for target gene expression at 20nM, and among them, IC50(inhibition concentration 50%) was less than 20nM in siRNAs having sequences of SEQ ID NOs: 6, 102 and 207 even in mouse cell lines. Therefore, it was confirmed that relatively high level of inhibition for target gene expression was maintained even at a low concentration, that is, these siRNAs had high efficiency (FIG. 10).

### ADVANTAGEOUS EFFECTS

The CTGF-specific siRNA according to the present invention, the double-helical oligo RNA structure containing the siRNA, and the pharmaceutical composition containing the double-helical oligo RNA structure for treatment, are capable of inhibiting expression of CTGF, Cyr61 or Plekho1 at a high efficiency without side effects to provide treatment effects for respiratory diseases, particularly, idiopathic pulmonary fibrosis and chronic obstructive pulmonary disease (COPD), which may be significantly usefully used for treating respiratory diseases in which there is no appropriate therapeutic agent at present, particularly, idiopathic pulmonary fibrosis and chronic obstructive pulmonary disease (COPD).
## Claims

1. A structure comprising double-helical oligo RNA, represented by the following Structural Formula (1):
Structural Formula 1 A-X-R-Y-B
wherein A is a hydrophilic material, B is a hydrophobic material, X and Y are each independently a simple covalent bond or a linker-mediated covalent bond, and R is CTGF-specific siRNA,
wherein the hydrophilic material has the structure represented by the following Structural Formula (5) or Structural Formula (6):
Structural Formula 5 (A'ₘ-J)ₙ
Structural Formula 6 (J-A'ₘ)ₙ
wherein A' is a hydrophilic material monomer,
J is a linker for connecting between m hydrophilic material monomers, or a linker for connecting between m hydrophilic material monomers with siRNA, m is an integer of 1 to 15, n is an integer of 1 to 10,
the hydrophilic material monomer A' is a compound selected from the group consisting of compounds (1) to (3), and
the linker J is selected from the group consisting of PO₃⁻, SO₃ and CO₂.
| Compound (1) | Compound (2) | Compound (3) |
|---|---|---|
| 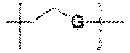 | 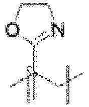 | 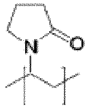 |
| G is O, S or NH | | |

2. The structure according to claim 1, wherein the structure comprising double-helical oligo RNA represented by the following Structural Formula (2):
Structural Formula 2 A-X-S-Y-B AS
wherein S is sense strand of the siRNA of claim 1, and AS is an antisense strand of claim 1, A, B, X and Y are the same as being defined in claim 1.

3. The structure according to claim 2, wherein the structure comprising double-helical oligo RNA represented by the following Structural Formula (3) or Structural Formula (4):
Structural Formula 3 A-X-5' S 3'-Y-B AS
Structural Formula 4 A-X-3' S 5'-Y-B AS
wherein A, B, X, Y S and AS are the same as being defined in claim 2, and 5' and 3' mean 5' end and 3' end of the sense strand of siRNA.

4. The structure according to any one of claims 1 to 3, wherein the hydrophilic material has a molecular weight of 200 to 10,000, preferably the hydrophilic material is any one selected from the group consisting of polyethylene glycol, polyvinyl pyrrolidone and polyoxazoline.

5. The structure according to claim 1, wherein the CTGF-specific siRNA is siRNA comprising a sense strand selected from the group consisting of SEQ ID NOs: 1 to 100 and 301 to 400 and an anti-sense strand complementary to the sense strand.

6. The structure according to any one of claims 1 to 5, wherein the hydrophobic material has a molecular weight of 250 to 1,000, preferably the hydrophobic material is one selected from the group consisting of a steroid derivative, a glyceride derivative, glycerol ether, polypropylene glycol, C₁₂ to C₅₀ unsaturated or saturated hydrocarbon, diacylphosphatidylcholine, fatty acid, phospholipid and lipopolyamine, preferably the steroid derivative is selected from the group consisting of cholesterol, cholestanol, cholic acid, cholesteryl formate, cholestanyl formate, and cholesteryl amine, preferably the glyceride derivative is selected from the group consisting of mono-, di- and tri-glyceride.

7. The structure according to any one of claims 1 to 6, wherein the covalent bond represented by X or Y is non-degradable bond or a degradable bond, preferably the non-degradable bond is amide bond or a phosphorylation bond, preferably the degradable bond is a disulfide bond, an acid degradable bond, an ester bond, an anhydride bond, a biodegradable bond or an enzymatically degradable bond.

8. The structure according to any one of claims 1 to 7, further comprising a ligand bonded to the hydrophilic material, which is specifically bonded to a receptor that promotes target cell internalization through receptor-mediated endocytosis (RME), preferably the ligand is selected from the group consisting of a target receptor-specific antibody, aptamer, peptide, N-acetyl galactosamine (NAG), glucose and mannose.

9. The structure according to any one of claims 1 to 8, further comprising amine group or polyhistidine introduced into the distal end bonded with the siRNA in the hydrophilic material, preferably the amine group or polyhistidine is bonded to hydrophilic material or hydrophilic block with more than a linker, preferably the amine group is one selected from the group consisting of primary to tertiary amine groups, preferably the polyhistidine comprises 3 to 10 histidines.

10. Nanoparticle(s) comprising the structure according to any one of claims 1 to 9, preferably the structure comprising the double-helical oligo RNA containing siRNAs with different sequences, is mixed in the nanoparticle(s).

11. A pharmaceutical composition comprising the structure according to any one of claims 1 to 9, or the nanoparticle(s) according to claim 10 as an active ingredient, preferably the composition is for prevention or treatment of respiratory diseases, preferably the respiratory diseases is selected from the group consisting of asthma, idiopathic pulmonary fibrosis, chronic obstructive pulmonary disease (COPD), acute or chronic bronchitis, allergic rhinitis, cough and phlegm, acute lower respiratory tract infection, bronchitis, bronchiolitis, acute upper respiratory tract infection, pharyngitis, tonsillitis, and laryngitis.

12. A lyophilized formulation comprising the pharmaceutical composition according to claim 11.

## Patentansprüche

1. Struktur, umfassend eine doppelthelikale Oligo-RNA, die durch die folgende Strukturformel (1) wiedergegeben wird:
Strukturformel 1 **A-X-R-Y-B,**
wobei A eine hydrophile Substanz ist, B eine hydrophobe Substanz ist, X und Y jeweils unabhängig eine einfache kovalente Bindung oder eine linkervermittelte kovalente Bindung sind und R eine CTGF-spezifische siRNA ist;
wobei die hydrophile Substanz die Struktur aufweist, die durch die folgende Strukturformel (5) oder Strukturformel (6) wiedergegeben wird:
Strukturformel 5 **(A'ₘ-J)ₙ,**
Strukturformel 6 (J-A'ₘ)ₙ,
wobei A' ein Monomer einer hydrophilen Substanz ist;
J ein Linker zur Verbindung zwischen m Monomeren der hydrophilen Substanz oder ein Linker zur Verbindung zwischen m Monomeren der hydrophilen Substanz mit siRNA ist, m eine ganze Zahl von 1 bis 15 ist, n eine ganze Zahl von 1 bis 10 ist,
das Monomer A' der hydrophilen Substanz eine Verbindung ist, die aus der Gruppe ausgewählt ist, die aus den Verbindungen (1) bis (3) besteht; und
der Linker J aus der Gruppe ausgewählt ist, die aus PO₃⁻, SO₃ und CO₂ besteht:
| Verbindung (1) | Verbindung (2) | Verbindung (3) |
|---|---|---|
| 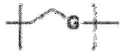 | 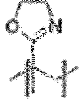 | 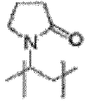 |
| wobei G = O, S oder NH ist | | |

2. Struktur gemäß Anspruch 1, wobei die Struktur, die eine doppelthelikale Oligo-RNA umfasst, durch die folgende Strukturformel (2) wiedergegeben wird:
Strukturformel 2 **A-X-S-Y-B AS,**
wobei S ein Sense-Strang der siRNA gemäß Anspruch 1 ist und AS ein Antisense-Strang gemäß Anspruch 1 ist, A, B, X und Y genauso wie in Anspruch 1 definiert sind.

3. Struktur gemäß Anspruch 2, wobei die Struktur, die eine doppelthelikale Oligo-RNA umfasst, durch die folgende Strukturformel (3) oder Strukturformel (4) wiedergegeben wird:
Strukturformel 3 **A-X-5'** S 3'-Y-B **AS,**
Strukturformel 4 A-X-3' **S** 5'-Y-B **AS,**
wobei A, B, X, Y, S und AS genauso wie in Anspruch 2 definiert sind und 5' und 3' das 5'-Ende bzw. das 3'-Ende des Sense-Stranges der siRNA bedeuten.

4. Struktur gemäß einem der Ansprüche 1 bis 3, wobei die hydrophile Substanz ein Molekulargewicht von 200 bis 10 000 aufweist, wobei vorzugsweise die hydrophile Substanz aus der Gruppe ausgewählt ist, die aus Polyethylenglycol, Polyvinylpyrrolidon und Polyoxazolin besteht.

5. Struktur gemäß Anspruch 1, wobei die CTGF-spezifische siRNA eine siRNA ist, die einen Sense-Strang, der aus der Gruppe ausgewählt ist, die aus SEQ ID Nr. 1 bis 100 und 301 bis 400 besteht, und einen zu dem Sense-Strang komplementären Antisense-Strang umfasst.

6. Struktur gemäß einem der Ansprüche 1 bis 5, wobei die hydrophobe Substanz ein Molekulargewicht von 250 bis 1000 aufweist, wobei vorzugsweise die hydrophobe Substanz aus der Gruppe ausgewählt ist, die aus einem Steroidderivat, einem Glyceridderivat, Glycerinether, Polypropylenglycol, ungesättigtem oder gesättigtem C₁₂- bis Cso-Kohlenwasserstoff, Diacylphosphatidylcholin, Fettsäure, Phospholipid und Lipopolyamin besteht, wobei vorzugsweise das Steroidderivat aus der Gruppe ausgewählt ist, die aus Cholesterin, Cholestanol, Cholsäure, Cholesterylformiat, Cholestanylformiat und Cholesterylamin besteht, wobei vorzugsweise das Glyceridderivat aus der Gruppe ausgewählt ist, die aus Mono-, Di- und Triglycerid besteht.

7. Struktur gemäß einem der Ansprüche 1 bis 6, wobei die durch X oder Y repräsentierte kovalente Bindung eine nicht abbaubare Bindung oder eine abbaubare Bindung ist, wobei vorzugsweise die nicht abbaubare Bindung eine Amidbindung oder eine Phosphorylierungsbindung ist, wobei vorzugsweise die abbaubare Bindung eine Disulfidbindung, eine säureabbaubare Bindung, eine Esterbindung, eine Anhydridbindung, eine bioabbaubare Bindung oder eine enzymatisch abbaubare Bindung ist.

8. Struktur gemäß einem der Ansprüche 1 bis 7, weiterhin umfassend einen Liganden, der an die hydrophile Substanz gebunden ist, welcher spezifisch an einen Rezeptor gebunden ist, der die Internalisierung in die Zielzelle durch rezeptorvermittelte Endocytose (RME) fördert, wobei vorzugsweise der Ligand aus der Gruppe ausgewählt ist, die aus einem zielrezeptorspezifischen Antikörper, Aptamer, Peptid, N-Acetylgalactosamin (NAG), Glucose und Mannose besteht.

9. Struktur gemäß einem der Ansprüche 1 bis 8, weiterhin umfassend eine Amingruppe oder ein Polyhistidin, eingeführt in das distale Ende und gebunden an die siRNA in der hydrophilen Substanz, wobei vorzugsweise die Amingruppe oder das Polyhistidin mit mehr als einem Linker an hydrophile Substanz oder hydrophilen Block gebunden ist, wobei vorzugsweise die Amingruppe eine ist, die aus der Gruppe ausgewählt ist, die aus primären bis tertiären Amingruppen besteht, wobei vorzugsweise das Polyhistidin 3 bis 10 Histidinreste umfasst.

10. Nanopartikel, umfassend die Struktur gemäß einem der Ansprüche 1 bis 9, wobei vorzugsweise die Struktur, die die doppelthelikale Oligo-RNA, welche siRNAs mit verschiedenen Sequenzen enthält, umfasst, in die Nanopartikel eingemischt ist.

11. Pharmazeutische Zusammensetzung, die die Struktur gemäß einem der Ansprüche 1 bis 9 oder die Nanopartikel gemäß Anspruch 10 als Wirkstoff umfasst, wobei vorzugsweise die Zusammensetzung zur Prävention oder Behandlung von Atemwegserkrankungen dient, wobei vorzugsweise die Atemwegserkrankungen aus der Gruppe ausgewählt sind, die aus Asthma, idiopathischer Lungenfibrose, chronisch obstruktiver Lungenerkrankung (COPD), akuter oder chronischer Bronchitis, allergischer Rhinitis, Husten und Auswurf, akuter Infektion der unteren Atemwege, Bronchitis, Bronchiolitis, akuter Infektion der oberen Atemwege, Pharyngitis, Tonsillitis und Laryngitis besteht.

12. Lyophilisierte Zubereitung, die die pharmazeutische Zusammensetzung gemäß Anspruch 11 umfasst.

## Revendications

1. Structure comprenant un oligo-RNA en double hélice, représentée par la formule développée (1) suivante :
formule développée 1 **A-X-R-Y-B,**
où A est une matière hydrophile, B est une matière hydrophobe, X et Y sont chacun indépendamment une liaison covalente simple ou une liaison covalente impliquant un groupe de liaison, et R est un siRNA CTGF-spécifique,
dans laquelle ladite matière hydrophile présente la structure représentée par la formule développée (5) ou la formule développée (6) suivantes :
formule développée 5 **(A'ₘ-J)ₙ,**
formule développée 6 **(J-A'ₘ)ₙ,**
où A' est un monomère d'une matière hydrophile,
J est un groupe de liaison pour établir une liaison entre m monomères de la matière hydrophile, ou un groupe de liaison pour établir une liaison entre m monomères de la matière hydrophile avec du siRNA, m est un nombre entier de 1 à 15, n est un nombre entier de 1 à 10,
le monomère de la matière hydrophile A' est un composé choisi dans le groupe consistant en les composés (1) à (3), et
le groupe de liaison J est choisi dans le groupe consistant en PO₃⁻, SO₃ et CO₂ :
| composé (1) | composé (2) | composé (3) |
|---|---|---|
| 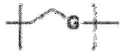 | 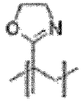 | 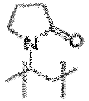 |
| où G est O, S ou NH | | |

2. Structure selon la revendication 1, dans laquelle la structure comprenant un oligo-RNA en double hélice est représentée par la formule développée (2) suivante :
formule développée 2 **A-X-S-Y-B AS,**
où S est le brin sens du siRNA selon la revendication 1 et AS est un brin antisens du siRNA selon la revendication 1, A, B, X et Y sont tels que définis dans la revendication 1.

3. Structure selon la revendication 2, dans laquelle la structure comprenant un oligo-RNA en double hélice est représentée par la formule développée (3) ou par la formule développée (4) suivantes :
formule développée 3 **A-X-5' S** 3'-Y-B **AS,**
formule développée 4 **A-X-3' S** 5'-Y-B **AS,**
où A, B, X, Y, S et AS sont tels que définis dans la revendication 2, et 5' et 3' signifient l'extrémité 5' et l'extrémité 3' respectivement du brin sens du siRNA.

4. Structure selon l'une quelconque des revendications 1 à 3, dans laquelle la matière hydrophile présente un poids moléculaire de 200 à 10000, de préférence la matière hydrophile étant l'une quelconque choisie dans le groupe consistant en polyéthylène glycol, polyvinylpyrrolidone et polyoxazoline.

5. Structure selon la revendication 1, dans laquelle ledit siRNA CTGF-spécifique est un siRNA comprenant un brin sens choisi dans le groupe consistant en SEQ ID n°s 1 à 100 et 301 à 400, et un brin antisens complémentaire audit brin sens.

6. Structure selon l'une quelconque des revendications 1 à 5, dans laquelle la matière hydrophobe présente un poids moléculaire de 250 à 1000, de préférence la matière hydrophobe étant l'une choisie dans le groupe consistant en un dérivé stéroïde, un dérivé de glycéride, un éther glycérique, un polypropylène glycol, un hydrocarbure insaturé ou saturé en C₁₂ à C₅₀, une diacylphosphatidylcholine, un acide gras, un phospholipide et une lipopolyamine, de préférence le dérivé stéroïde étant choisi dans le groupe consistant en cholestérol, cholestanol, acide cholique, formiate de cholestéryle, formiate de cholestanyle, et cholestérylamine, de préférence le dérivé de glycéride est choisi dans le groupe consistant en mono-, di- et triglycéride.

7. Structure selon l'une quelconque des revendications 1 à 6, dans laquelle la liaison covalente représentée par X ou Y est une liaison non dégradable ou une liaison dégradable, de préférence la liaison non dégradable est une liaison amide ou une liaison de phosphorylation, de préférence la liaison dégradable est une liaison disulfide, une liaison dégradable par acide, une liaison ester, une liaison d'anhydride, une liaison biodégradable, ou une liaison dégradable enzymatiquement.

8. Structure selon l'une quelconque des revendications 1 à 7, comprenant en outre un ligand relié à la matière hydrophile, qui est spécifiquement relié à un récepteur qui favorise l'internalisation dans une cellule cible par endocytose induite par récepteurs (RME), de préférence le ligand étant choisi dans le groupe consistant en un anticorps spécifique du récepteur cible, un aptamère, un peptide, N-acétylgalactosamine (NAG), glucose et mannose.

9. Structure selon l'une quelconque des revendications 1 à 8, comprenant en outre un groupe amine ou une polyhistidine, introduit dans l'extrémité distale, relié avec le siRNA dans la matière hydrophile, de préférence le groupe amine ou la polyhistidine étant relié à une matière hydrophile ou un bloc hydrophile avec plus d'un groupe de liaison, de préférence le groupe amine étant un choisi dans le groupe consistant en groupes amine primaires à tertiaires, de préférence la polyhistidine comprenant 3 à 10 résidus histidine.

10. Nanoparticule(s) comprenant la structure selon l'une quelconque des revendications 1 à 9, de préférence la structure comprenant l'oligo-RNA en double hélice contenant des siRNAs à différentes séquences étant mélangée dans la ou les nanoparticule(s).

11. Composition pharmaceutique comprenant la structure selon l'une quelconque des revendications 1 à 9 ou la ou les nanoparticule(s) selon la revendication 10 comme principe actif, de préférence la composition servant à la prévention ou au traitement de maladies respiratoires, de préférence les maladies respiratoires étant choisies dans le groupe consistant en asthme, fibrose pulmonaire idiopathique, bronchopneumopathie chronique obstructive (BPCO), bronchite aiguë ou chronique, rhinite allergique, toux et flegme, infection aiguë des voies respiratoires inférieures, bronchite, bronchiolite, infection aiguë des voies respiratoires supérieures, pharyngite, amygdalite, et laryngite.

12. Formulation lyophilisée comprenant la composition pharmaceutique selon la revendication 11.
